(19) Europäisches Patentamt / European Patent Office / Office européen des brevets

(11) EP 3 182 124 B1

(12) **EUROPEAN PATENT SPECIFICATION**

(45) Date of publication and mention of the grant of the patent:
**22.08.2018 Bulletin 2018/34**

(51) Int Cl.:
***G01N 33/50*** *(2006.01)* ***G01N 33/574*** *(2006.01)*

(21) Application number: **17153845.7**

(22) Date of filing: **21.12.2011**

(54) **DETECTION OF ACUTE MYELOID LEUKAEMIA**

NACHWEIS VON AKUTER MYELOISCHER LEUKÄMIE

DÉTECTION DE LA LEUCÉMIE MYÉLOÏDE AIGUË

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2010 GB 201021623**

(43) Date of publication of application:
**21.06.2017 Bulletin 2017/25**

(62) Document number(s) of the earlier application(s) in accordance with Art. 76 EPC:
**11804770.3 / 2 656 069**

(73) Proprietor: **Oxford University Innovation Limited**
**Oxford OX2 0SZ (GB)**

(72) Inventors:
- **VYAS, Paresh**
  **Oxford, Oxfordshire OX3 9DS (GB)**
- **GOARDON, Nicolas**
  **Oxford, Oxfordshire OX3 9DS (GB)**
- **FREEMAN, Sylvie**
  **Birmingham, B15 2TT (GB)**

(74) Representative: **Hobson, David James et al**
**Mathys & Squire LLP**
**The Shard**
**32 London Bridge Street**
**London SE1 9SG (GB)**

(56) References cited:
**WO-A1-2010/102244 US-A1- 2007 111 238**

- **JORDAN C T ET AL: "The interleukin-3 receptor alpha chain is a unique marker for human acute myelogenous leukemia stem cells", LEUKEMIA, MACMILLAN PRESS LTD, US, vol. 14, no. 10, 1 October 2000 (2000-10-01), pages 1777-1784, XP002390122, ISSN: 0887-6924, DOI: 10.1038/SJ.LEU.2401903**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 182 124 B1

Printed by Jouve, 75001 PARIS (FR)

SEQUENCE LISTING

<110> OXFORD UNIVERSITY INNOVATION LIMITED
Vyas, Paresh
Goardon, Nicolas
Freeman, Sylvie

<120> Detection of Acute Myeloid Leukaemia

<130> P34622EP-D1-PCT

<140> Not yet assigned
<141> 2011-12-21

<150> GB 1021623.2
<151> 2010-12-21

<160> 20

<170> PatentIn version 3.5

<210> 1
<211> 328
<212> PRT
<213> Homo sapiens

<400> 1

```
Met Leu Val Arg Arg Gly Ala Arg Ala Gly Pro Arg Met Pro Arg Gly
1               5                   10                  15

Trp Thr Ala Leu Cys Leu Leu Ser Leu Leu Pro Ser Gly Phe Met Ser
            20                  25                  30

Leu Asp Asn Asn Gly Thr Ala Thr Pro Glu Leu Pro Thr Gln Gly Thr
        35                  40                  45

Phe Ser Asn Val Ser Thr Asn Val Ser Tyr Gln Glu Thr Thr Thr Pro
    50                  55                  60

Ser Thr Leu Gly Ser Thr Ser Leu His Pro Val Ser Gln His Gly Asn
65                  70                  75                  80

Glu Ala Thr Thr Asn Ile Thr Glu Thr Thr Val Lys Phe Thr Ser Thr
                85                  90                  95

Ser Val Ile Thr Ser Val Tyr Gly Asn Thr Asn Ser Ser Val Gln Ser
            100                 105                 110

Gln Thr Ser Val Ile Ser Thr Val Phe Thr Thr Pro Ala Asn Val Ser
        115                 120                 125

Thr Pro Glu Thr Thr Leu Lys Pro Ser Leu Ser Pro Gly Asn Val Ser
    130                 135                 140
```

```
Asp Leu Ser Thr Thr Ser Thr Ser Leu Ala Thr Ser Pro Thr Lys Pro
145                 150                 155                 160

Tyr Thr Ser Ser Ser Pro Ile Leu Ser Asp Ile Lys Ala Glu Ile Lys
                165                 170                 175

Cys Ser Gly Ile Arg Glu Val Lys Leu Thr Gln Gly Ile Cys Leu Glu
            180                 185                 190

Gln Asn Lys Thr Ser Ser Cys Ala Glu Phe Lys Lys Asp Arg Gly Glu
        195                 200                 205

Gly Leu Ala Arg Val Leu Cys Gly Glu Glu Gln Ala Asp Ala Asp Ala
    210                 215                 220

Gly Ala Gln Val Cys Ser Leu Leu Leu Ala Gln Ser Glu Val Arg Pro
225                 230                 235                 240

Gln Cys Leu Leu Leu Val Leu Ala Asn Arg Thr Glu Ile Ser Ser Lys
                245                 250                 255

Leu Gln Leu Met Lys Lys His Gln Ser Asp Leu Lys Lys Leu Gly Ile
            260                 265                 270

Leu Asp Phe Thr Glu Gln Asp Val Ala Ser His Gln Ser Tyr Ser Gln
        275                 280                 285

Lys Thr Leu Ile Ala Leu Val Thr Ser Gly Ala Leu Leu Ala Val Leu
    290                 295                 300

Gly Ile Thr Gly Tyr Phe Leu Met Asn Arg Arg Ser Trp Ser Pro Thr
305                 310                 315                 320

Gly Glu Arg Leu Glu Leu Glu Pro
                325
```

<210> 2
<211> 1143
<212> PRT
<213> Homo sapiens

<400> 2

```
Met Tyr Leu Trp Leu Lys Leu Leu Ala Phe Gly Phe Ala Phe Leu Asp
1               5                   10                  15

Thr Glu Val Phe Val Thr Gly Gln Ser Pro Thr Pro Ser Pro Thr Asp
            20                  25                  30
```

```
Ala Tyr Leu Asn Ala Ser Glu Thr Thr Thr Leu Ser Pro Ser Gly Ser
        35                  40                  45

Ala Val Ile Ser Thr Thr Thr Ile Ala Thr Thr Pro Ser Lys Pro Thr
    50                  55                  60

Cys Asp Glu Lys Tyr Ala Asn Ile Thr Val Asp Tyr Leu Tyr Asn Lys
65                  70                  75                  80

Glu Thr Lys Leu Phe Thr Ala Lys Leu Asn Val Asn Glu Asn Val Glu
                85                  90                  95

Cys Gly Asn Asn Thr Cys Thr Asn Asn Glu Val His Asn Leu Thr Glu
            100                 105                 110

Cys Lys Asn Ala Ser Val Ser Ile Ser His Asn Ser Cys Thr Ala Pro
        115                 120                 125

Asp Lys Thr Leu Ile Leu Asp Val Pro Pro Gly Val Glu Lys Phe Gln
    130                 135                 140

Leu His Asp Cys Thr Gln Val Glu Lys Ala Asp Thr Thr Ile Cys Leu
145                 150                 155                 160

Lys Trp Lys Asn Ile Glu Thr Phe Thr Cys Asp Thr Gln Asn Ile Thr
                165                 170                 175

Tyr Arg Phe Gln Cys Gly Asn Met Ile Phe Asp Asn Lys Glu Ile Lys
            180                 185                 190

Leu Glu Asn Leu Glu Pro Glu His Glu Tyr Lys Cys Asp Ser Glu Ile
        195                 200                 205

Leu Tyr Asn Asn His Lys Phe Thr Asn Ala Ser Lys Ile Ile Lys Thr
    210                 215                 220

Asp Phe Gly Ser Pro Gly Glu Pro Gln Ile Ile Phe Cys Arg Ser Glu
225                 230                 235                 240

Ala Ala His Gln Gly Val Ile Thr Trp Asn Pro Pro Gln Arg Ser Phe
                245                 250                 255

His Asn Phe Thr Leu Cys Tyr Ile Lys Glu Thr Glu Lys Asp Cys Leu
            260                 265                 270

Asn Leu Asp Lys Asn Leu Ile Lys Tyr Asp Leu Gln Asn Leu Lys Pro
        275                 280                 285
```

Tyr Thr Lys Tyr Val Leu Ser Leu His Ala Tyr Ile Ile Ala Lys Val
290 295 300

Gln Arg Asn Gly Ser Ala Ala Met Cys His Phe Thr Thr Lys Ser Ala
305 310 315 320

Pro Pro Ser Gln Val Trp Asn Met Thr Val Ser Met Thr Ser Asp Asn
325 330 335

Ser Met His Val Lys Cys Arg Pro Pro Arg Asp Arg Asn Gly Pro His
340 345 350

Glu Arg Tyr His Leu Glu Val Glu Ala Gly Asn Thr Leu Val Arg Asn
355 360 365

Glu Ser His Lys Asn Cys Asp Phe Arg Val Lys Asp Leu Gln Tyr Ser
370 375 380

Thr Asp Tyr Thr Phe Lys Ala Tyr Phe His Asn Gly Asp Tyr Pro Gly
385 390 395 400

Glu Pro Phe Ile Leu His His Ser Thr Ser Tyr Asn Ser Lys Ala Leu
405 410 415

Ile Ala Phe Leu Ala Phe Leu Ile Ile Val Thr Ser Ile Ala Leu Leu
420 425 430

Val Val Leu Tyr Lys Ile Tyr Asp Leu His Lys Lys Arg Ser Cys Asn
435 440 445

Leu Asp Glu Gln Gln Glu Leu Val Glu Arg Asp Asp Glu Lys Gln Leu
450 455 460

Met Asn Val Glu Pro Ile His Ala Asp Ile Leu Leu Glu Thr Tyr Lys
465 470 475 480

Arg Lys Ile Ala Asp Glu Gly Arg Leu Phe Leu Ala Glu Phe Gln Ser
485 490 495

Ile Pro Arg Val Phe Ser Lys Phe Pro Ile Lys Glu Ala Arg Lys Pro
500 505 510

Phe Asn Gln Asn Lys Asn Arg Tyr Val Asp Ile Leu Pro Tyr Asp Tyr
515 520 525

Asn Arg Val Glu Leu Ser Glu Ile Asn Gly Asp Ala Gly Ser Asn Tyr

```
    530                 535                 540

Ile Asn Ala Ser Tyr Ile Asp Gly Phe Lys Glu Pro Arg Lys Tyr Ile
545                 550                 555                 560

Ala Ala Gln Gly Pro Arg Asp Glu Thr Val Asp Asp Phe Trp Arg Met
                565                 570                 575

Ile Trp Glu Gln Lys Ala Thr Val Ile Val Met Val Thr Arg Cys Glu
            580                 585                 590

Glu Gly Asn Arg Asn Lys Cys Ala Glu Tyr Trp Pro Ser Met Glu Glu
        595                 600                 605

Gly Thr Arg Ala Phe Gly Asp Val Val Val Lys Ile Asn Gln His Lys
    610                 615                 620

Arg Cys Pro Asp Tyr Ile Ile Gln Lys Leu Asn Ile Val Asn Lys Lys
625                 630                 635                 640

Glu Lys Ala Thr Gly Arg Glu Val Thr His Ile Gln Phe Thr Ser Trp
                645                 650                 655

Pro Asp His Gly Val Pro Glu Asp Pro His Leu Leu Leu Lys Leu Arg
            660                 665                 670

Arg Arg Val Asn Ala Phe Ser Asn Phe Phe Ser Gly Pro Ile Val Val
        675                 680                 685

His Cys Ser Ala Gly Val Gly Arg Thr Gly Thr Tyr Ile Gly Ile Asp
    690                 695                 700

Ala Met Leu Glu Gly Leu Glu Ala Glu Asn Lys Val Asp Val Tyr Gly
705                 710                 715                 720

Tyr Val Val Lys Leu Arg Arg Gln Arg Cys Leu Met Val Gln Val Glu
                725                 730                 735

Ala Gln Tyr Ile Leu Ile His Gln Ala Leu Val Glu Tyr Asn Gln Phe
            740                 745                 750

Gly Glu Thr Glu Val Asn Leu Ser Glu Leu His Pro Tyr Leu His Asn
        755                 760                 765

Met Lys Lys Arg Asp Pro Pro Ser Glu Pro Ser Pro Leu Glu Ala Glu
    770                 775                 780
```

```
Phe Gln Arg Leu Pro Ser Tyr Arg Ser Trp Arg Thr Gln His Ile Gly
785                 790                 795                 800

Asn Gln Glu Glu Asn Lys Ser Lys Asn Arg Asn Ser Asn Val Ile Pro
                805                 810                 815

Tyr Asp Tyr Asn Arg Val Pro Leu Lys His Glu Leu Glu Met Ser Lys
            820                 825                 830

Glu Ser Glu His Asp Ser Asp Glu Ser Ser Asp Asp Asp Ser Asp Ser
        835                 840                 845

Glu Glu Pro Ser Lys Tyr Ile Asn Ala Ser Phe Ile Met Ser Tyr Trp
    850                 855                 860

Lys Pro Glu Val Met Ile Ala Ala Gln Gly Pro Leu Lys Glu Thr Ile
865                 870                 875                 880

Gly Asp Phe Trp Gln Met Ile Phe Gln Arg Lys Val Lys Val Ile Val
                885                 890                 895

Met Leu Thr Glu Leu Lys His Gly Asp Gln Glu Ile Cys Ala Gln Tyr
            900                 905                 910

Trp Gly Glu Gly Lys Gln Thr Tyr Gly Asp Ile Glu Val Asp Leu Lys
        915                 920                 925

Asp Thr Asp Lys Ser Ser Thr Tyr Thr Leu Arg Val Phe Glu Leu Arg
    930                 935                 940

His Ser Lys Arg Lys Asp Ser Arg Thr Val Tyr Gln Tyr Gln Tyr Thr
945                 950                 955                 960

Asn Trp Ser Val Glu Gln Leu Pro Ala Glu Pro Lys Glu Leu Ile Ser
                965                 970                 975

Met Ile Gln Val Val Lys Gln Lys Leu Pro Gln Lys Asn Ser Ser Glu
            980                 985                 990

Gly Asn Lys His His Lys Ser Thr  Pro Leu Leu Ile His  Cys Arg Asp
        995                 1000                1005

Gly Ser  Gln Gln Thr Gly Ile  Phe Cys Ala Leu Leu  Asn Leu Leu
    1010                 1015                 1020

Glu Ser  Ala Glu Thr Glu Glu  Val Val Asp Ile Phe  Gln Val Val
    1025                 1030                 1035
```

Lys Ala Leu Arg Lys Ala Arg Pro Gly Met Val Ser Thr Phe Glu
1040 1045 1050

Gln Tyr Gln Phe Leu Tyr Asp Val Ile Ala Ser Thr Tyr Pro Ala
1055 1060 1065

Gln Asn Gly Gln Val Lys Lys Asn Asn His Gln Glu Asp Lys Ile
1070 1075 1080

Glu Phe Asp Asn Glu Val Asp Lys Val Lys Gln Asp Ala Asn Cys
1085 1090 1095

Val Asn Pro Leu Gly Ala Pro Glu Lys Leu Pro Glu Ala Lys Glu
1100 1105 1110

Gln Ala Glu Gly Ser Glu Pro Thr Ser Gly Thr Glu Gly Pro Glu
1115 1120 1125

His Ser Val Asn Gly Pro Ala Ser Pro Ala Leu Asn Gln Gly Ser
1130 1135 1140

<210> 3
<211> 161
<212> PRT
<213> Homo sapiens

<400> 3

Met Asn Leu Ala Ile Ser Ile Ala Leu Leu Leu Thr Val Leu Gln Val
1 5 10 15

Ser Arg Gly Gln Lys Val Thr Ser Leu Thr Ala Cys Leu Val Asp Gln
20 25 30

Ser Leu Arg Leu Asp Cys Arg His Glu Asn Thr Ser Ser Ser Pro Ile
35 40 45

Gln Tyr Glu Phe Ser Leu Thr Arg Glu Thr Lys Lys His Val Leu Phe
50 55 60

Gly Thr Val Gly Val Pro Glu His Thr Tyr Arg Ser Arg Thr Asn Phe
65 70 75 80

Thr Ser Lys Tyr Asn Met Lys Val Leu Tyr Leu Ser Ala Phe Thr Ser
85 90 95

Lys Asp Glu Gly Thr Tyr Thr Cys Ala Leu His His Ser Gly His Ser
100 105 110

Pro Pro Ile Ser Ser Gln Asn Val Thr Val Leu Arg Asp Lys Leu Val
115 120 125

Lys Cys Glu Gly Ile Ser Leu Leu Ala Gln Asn Thr Ser Trp Leu Leu
130 135 140

Leu Leu Leu Leu Ser Leu Ser Leu Leu Gln Ala Thr Asp Phe Met Ser
145 150 155 160

Leu

<210> 4
<211> 378
<212> PRT
<213> Homo sapiens

<400> 4

Met Val Leu Leu Trp Leu Thr Leu Leu Leu Ile Ala Leu Pro Cys Leu
1 5 10 15

Leu Gln Thr Lys Glu Asp Pro Asn Pro Pro Ile Thr Asn Leu Arg Met
20 25 30

Lys Ala Lys Ala Gln Gln Leu Thr Trp Asp Leu Asn Arg Asn Val Thr
35 40 45

Asp Ile Glu Cys Val Lys Asp Ala Asp Tyr Ser Met Pro Ala Val Asn
50 55 60

Asn Ser Tyr Cys Gln Phe Gly Ala Ile Ser Leu Cys Glu Val Thr Asn
65 70 75 80

Tyr Thr Val Arg Val Ala Asn Pro Pro Phe Ser Thr Trp Ile Leu Phe
85 90 95

Pro Glu Asn Ser Gly Lys Pro Trp Ala Gly Ala Glu Asn Leu Thr Cys
100 105 110

Trp Ile His Asp Val Asp Phe Leu Ser Cys Ser Trp Ala Val Gly Pro
115 120 125

Gly Ala Pro Ala Asp Val Gln Tyr Asp Leu Tyr Leu Asn Val Ala Asn
130 135 140

Arg Arg Gln Gln Tyr Glu Cys Leu His Tyr Lys Thr Asp Ala Gln Gly
145 150 155 160

Thr Arg Ile Gly Cys Arg Phe Asp Asp Ile Ser Arg Leu Ser Ser Gly
165 170 175

Ser Gln Ser Ser His Ile Leu Val Arg Gly Arg Ser Ala Ala Phe Gly
180 185 190

Ile Pro Cys Thr Asp Lys Phe Val Val Phe Ser Gln Ile Glu Ile Leu
195 200 205

Thr Pro Pro Asn Met Thr Ala Lys Cys Asn Lys Thr His Ser Phe Met
210 215 220

His Trp Lys Met Arg Ser His Phe Asn Arg Lys Phe Arg Tyr Glu Leu
225 230 235 240

Gln Ile Gln Lys Arg Met Gln Pro Val Ile Thr Glu Gln Val Arg Asp
245 250 255

Arg Thr Ser Phe Gln Leu Leu Asn Pro Gly Thr Tyr Thr Val Gln Ile
260 265 270

Arg Ala Arg Glu Arg Val Tyr Glu Phe Leu Ser Ala Trp Ser Thr Pro
275 280 285

Gln Arg Phe Glu Cys Asp Gln Glu Glu Gly Ala Asn Thr Arg Ala Trp
290 295 300

Arg Thr Ser Leu Leu Ile Ala Leu Gly Thr Leu Leu Ala Leu Val Cys
305 310 315 320

Val Phe Val Ile Cys Arg Arg Tyr Leu Val Met Gln Arg Leu Phe Pro
325 330 335

Arg Ile Pro His Met Lys Asp Pro Ile Gly Asp Ser Phe Gln Asn Asp
340 345 350

Lys Leu Val Val Trp Glu Ala Gly Lys Ala Gly Leu Glu Glu Cys Leu
355 360 365

Val Thr Glu Val Gln Val Val Gln Lys Thr
370 375

<210> 5
<211> 300
<212> PRT
<213> Homo sapiens

<400> 5

```
Met Ala Asn Cys Glu Phe Ser Pro Val Ser Gly Asp Lys Pro Cys Cys
1               5                   10                  15

Arg Leu Ser Arg Arg Ala Gln Leu Cys Leu Gly Val Ser Ile Leu Val
            20                  25                  30

Leu Ile Leu Val Val Val Leu Ala Val Val Val Pro Arg Trp Arg Gln
        35                  40                  45

Gln Trp Ser Gly Pro Gly Thr Thr Lys Arg Phe Pro Glu Thr Val Leu
    50                  55                  60

Ala Arg Cys Val Lys Tyr Thr Glu Ile His Pro Glu Met Arg His Val
65                  70                  75                  80

Asp Cys Gln Ser Val Trp Asp Ala Phe Lys Gly Ala Phe Ile Ser Lys
                85                  90                  95

His Pro Cys Asn Ile Thr Glu Glu Asp Tyr Gln Pro Leu Met Lys Leu
            100                 105                 110

Gly Thr Gln Thr Val Pro Cys Asn Lys Ile Leu Leu Trp Ser Arg Ile
        115                 120                 125

Lys Asp Leu Ala His Gln Phe Thr Gln Val Gln Arg Asp Met Phe Thr
    130                 135                 140

Leu Glu Asp Thr Leu Leu Gly Tyr Leu Ala Asp Asp Leu Thr Trp Cys
145                 150                 155                 160

Gly Glu Phe Asn Thr Ser Lys Ile Asn Tyr Gln Ser Cys Pro Asp Trp
                165                 170                 175

Arg Lys Asp Cys Ser Asn Asn Pro Val Ser Val Phe Trp Lys Thr Val
            180                 185                 190

Ser Arg Arg Phe Ala Glu Ala Ala Cys Asp Val Val His Val Met Leu
        195                 200                 205

Asn Gly Ser Arg Ser Lys Ile Phe Asp Lys Asn Ser Thr Phe Gly Ser
    210                 215                 220

Val Glu Val His Asn Leu Gln Pro Glu Lys Val Gln Thr Leu Glu Ala
225                 230                 235                 240

Trp Val Ile His Gly Gly Arg Glu Asp Ser Arg Asp Leu Cys Gln Asp
                245                 250                 255
```

Pro Thr Ile Lys Glu Leu Glu Ser Ile Ile Ser Lys Arg Asn Ile Gln
260 265 270

Phe Ser Cys Lys Asn Ile Tyr Arg Pro Asp Lys Phe Leu Gln Cys Val
275 280 285

Lys Asn Pro Glu Asp Ser Ser Cys Thr Ser Glu Ile
290 295 300

<210> 6
<211> 556
<212> PRT
<213> Homo sapiens

<400> 6

Met Pro Pro Pro Arg Leu Leu Phe Phe Leu Leu Phe Leu Thr Pro Met
1 5 10 15

Glu Val Arg Pro Glu Glu Pro Leu Val Val Lys Val Glu Glu Gly Asp
20 25 30

Asn Ala Val Leu Gln Cys Leu Lys Gly Thr Ser Asp Gly Pro Thr Gln
35 40 45

Gln Leu Thr Trp Ser Arg Glu Ser Pro Leu Lys Pro Phe Leu Lys Leu
50 55 60

Ser Leu Gly Leu Pro Gly Leu Gly Ile His Met Arg Pro Leu Ala Ile
65 70 75 80

Trp Leu Phe Ile Phe Asn Val Ser Gln Gln Met Gly Gly Phe Tyr Leu
85 90 95

Cys Gln Pro Gly Pro Pro Ser Glu Lys Ala Trp Gln Pro Gly Trp Thr
100 105 110

Val Asn Val Glu Gly Ser Gly Glu Leu Phe Arg Trp Asn Val Ser Asp
115 120 125

Leu Gly Gly Leu Gly Cys Gly Leu Lys Asn Arg Ser Ser Glu Gly Pro
130 135 140

Ser Ser Pro Ser Gly Lys Leu Met Ser Pro Lys Leu Tyr Val Trp Ala
145 150 155 160

Lys Asp Arg Pro Glu Ile Trp Glu Gly Glu Pro Pro Cys Leu Pro Pro
165 170 175

```
Arg Asp Ser Leu Asn Gln Ser Leu Ser Gln Asp Leu Thr Met Ala Pro
            180                 185                 190

Gly Ser Thr Leu Trp Leu Ser Cys Gly Val Pro Pro Asp Ser Val Ser
        195                 200                 205

Arg Gly Pro Leu Ser Trp Thr His Val His Pro Lys Gly Pro Lys Ser
    210                 215                 220

Leu Leu Ser Leu Glu Leu Lys Asp Asp Arg Pro Ala Arg Asp Met Trp
225                 230                 235                 240

Val Met Glu Thr Gly Leu Leu Leu Pro Arg Ala Thr Ala Gln Asp Ala
                245                 250                 255

Gly Lys Tyr Tyr Cys His Arg Gly Asn Leu Thr Met Ser Phe His Leu
            260                 265                 270

Glu Ile Thr Ala Arg Pro Val Leu Trp His Trp Leu Leu Arg Thr Gly
        275                 280                 285

Gly Trp Lys Val Ser Ala Val Thr Leu Ala Tyr Leu Ile Phe Cys Leu
    290                 295                 300

Cys Ser Leu Val Gly Ile Leu His Leu Gln Arg Ala Leu Val Leu Arg
305                 310                 315                 320

Arg Lys Arg Lys Arg Met Thr Asp Pro Thr Arg Arg Phe Phe Lys Val
                325                 330                 335

Thr Pro Pro Pro Gly Ser Gly Pro Gln Asn Gln Tyr Gly Asn Val Leu
            340                 345                 350

Ser Leu Pro Thr Pro Thr Ser Gly Leu Gly Arg Ala Gln Arg Trp Ala
        355                 360                 365

Ala Gly Leu Gly Gly Thr Ala Pro Ser Tyr Gly Asn Pro Ser Ser Asp
    370                 375                 380

Val Gln Ala Asp Gly Ala Leu Gly Ser Arg Ser Pro Pro Gly Val Gly
385                 390                 395                 400

Pro Glu Glu Glu Glu Gly Glu Gly Tyr Glu Glu Pro Asp Ser Glu Glu
                405                 410                 415

Asp Ser Glu Phe Tyr Glu Asn Asp Ser Asn Leu Gly Gln Asp Gln Leu
```

420 425 430

Ser Gln Asp Gly Ser Gly Tyr Glu Asn Pro Glu Asp Glu Pro Leu Gly
435 440 445

Pro Glu Asp Glu Asp Ser Phe Ser Asn Ala Glu Ser Tyr Glu Asn Glu
450 455 460

Asp Glu Glu Leu Thr Gln Pro Val Ala Arg Thr Met Asp Phe Leu Ser
465 470 475 480

Pro His Gly Ser Ala Trp Asp Pro Ser Arg Glu Ala Thr Ser Leu Gly
485 490 495

Ser Gln Ser Tyr Glu Asp Met Arg Gly Ile Leu Tyr Ala Ala Pro Gln
500 505 510

Leu Arg Ser Ile Arg Gly Gln Pro Gly Pro Asn His Glu Glu Asp Ala
515 520 525

Asp Ser Tyr Glu Asn Met Asp Asn Pro Asp Gly Pro Asp Pro Ala Trp
530 535 540

Gly Gly Gly Gly Arg Met Gly Thr Trp Ser Thr Arg
545 550 555

<210> 7
<211> 322
<212> PRT
<213> Homo sapiens

<400> 7

```
Trp Pro Leu Val Ala Ala Leu Leu Leu Gly Ser Ala Cys Cys Gly Ser
1               5                   10                  15

Ala Gln Leu Leu Phe Asn Lys Thr Lys Ser Val Glu Phe Thr Phe Cys
            20                  25                  30

Asn Asp Thr Val Val Ile Pro Cys Phe Val Thr Asn Met Glu Ala Gln
        35                  40                  45

Asn Thr Thr Glu Val Tyr Val Lys Trp Lys Phe Lys Gly Arg Asp Ile
    50                  55                  60

Tyr Thr Phe Asp Gly Ala Leu Asn Lys Ser Thr Val Pro Thr Asp Phe
65                  70                  75                  80

Ser Ser Ala Lys Ile Glu Val Ser Gln Leu Leu Lys Gly Asp Ala Ser
```

85 90 95

Leu Lys Met Asp Lys Ser Asp Ala Val Ser His Thr Gly Asn Tyr Thr
100 105 110

Cys Glu Val Thr Glu Leu Thr Arg Glu Gly Glu Thr Ile Ile Glu Leu
115 120 125

Lys Tyr Arg Val Val Ser Trp Phe Ser Pro Asn Glu Asn Ile Leu Ile
130 135 140

Val Ile Phe Pro Ile Phe Ala Ile Leu Leu Phe Trp Gly Gln Phe Gly
145 150 155 160

Ile Lys Thr Leu Lys Tyr Arg Ser Gly Gly Met Asp Glu Lys Thr Ile
165 170 175

Ala Leu Leu Val Ala Gly Leu Val Ile Thr Val Ile Val Ile Val Gly
180 185 190

Ala Ile Leu Phe Val Pro Gly Glu Tyr Ser Leu Lys Asn Ala Thr Gly
195 200 205

Leu Gly Leu Ile Val Thr Ser Thr Gly Ile Leu Ile Leu Leu His Tyr
210 215 220

Tyr Val Phe Ser Thr Ala Ile Gly Leu Thr Ser Phe Val Ile Ala Ile
225 230 235 240

Leu Val Ile Gln Val Ile Ala Tyr Ile Leu Ala Val Val Gly Leu Ser
245 250 255

Leu Cys Ile Ala Ala Cys Ile Pro Met His Gly Pro Leu Leu Ile Ser
260 265 270

Gly Leu Ser Ile Leu Ala Leu Ala Gln Leu Leu Gly Leu Val Tyr Met
275 280 285

Lys Phe Val Ala Ser Asn Gln Lys Thr Ile Gln Pro Pro Arg Lys Ala
290 295 300

Val Glu Glu Pro Leu Asn Ala Phe Lys Glu Ser Lys Gly Met Met Asn
305 310 315 320

Asp Glu

<210> 8
<211> 355
<212> PRT
<213> Homo sapiens

<400> 8

```
Met Asp Tyr Thr Leu Asp Leu Ser Val Thr Thr Val Thr Asp Tyr Tyr
1               5                   10                  15

Tyr Pro Asp Ile Phe Ser Ser Pro Cys Asp Ala Glu Leu Ile Gln Thr
            20                  25                  30

Asn Gly Lys Leu Leu Leu Ala Val Phe Tyr Cys Leu Leu Phe Val Phe
        35                  40                  45

Ser Leu Leu Gly Asn Ser Leu Val Ile Leu Val Leu Val Val Cys Lys
    50                  55                  60

Lys Leu Arg Ser Ile Thr Asp Val Tyr Leu Leu Asn Leu Ala Leu Ser
65                  70                  75                  80

Asp Leu Leu Phe Val Phe Ser Phe Pro Phe Gln Thr Tyr Tyr Leu Leu
                85                  90                  95

Asp Gln Trp Val Phe Gly Thr Val Met Cys Lys Val Val Ser Gly Phe
            100                 105                 110

Tyr Tyr Ile Gly Phe Tyr Ser Ser Met Phe Phe Ile Thr Leu Met Ser
        115                 120                 125

Val Asp Arg Tyr Leu Ala Val Val His Ala Val Tyr Ala Leu Lys Val
    130                 135                 140

Arg Thr Ile Arg Met Gly Thr Thr Leu Cys Leu Ala Val Trp Leu Thr
145                 150                 155                 160

Ala Ile Met Ala Thr Ile Pro Leu Leu Val Phe Tyr Gln Val Ala Ser
                165                 170                 175

Glu Asp Gly Val Leu Gln Cys Tyr Ser Phe Tyr Asn Gln Gln Thr Leu
            180                 185                 190

Lys Trp Lys Ile Phe Thr Asn Phe Lys Met Asn Ile Leu Gly Leu Leu
        195                 200                 205

Ile Pro Phe Thr Ile Phe Met Phe Cys Tyr Ile Lys Ile Leu His Gln
    210                 215                 220
```

Leu Lys Arg Cys Gln Asn His Asn Lys Thr Lys Ala Ile Arg Leu Val
225 230 235 240

Leu Ile Val Val Ile Ala Ser Leu Leu Phe Trp Val Pro Phe Asn Val
245 250 255

Val Leu Phe Leu Thr Ser Leu His Ser Met His Ile Leu Asp Gly Cys
260 265 270

Ser Ile Ser Gln Gln Leu Thr Tyr Ala Thr His Val Thr Glu Ile Ile
275 280 285

Ser Phe Thr His Cys Cys Val Asn Pro Val Ile Tyr Ala Phe Val Gly
290 295 300

Glu Lys Phe Lys Lys His Leu Ser Glu Ile Phe Gln Lys Ser Cys Ser
305 310 315 320

Gln Ile Phe Asn Tyr Leu Gly Arg Gln Met Pro Arg Glu Ser Cys Glu
325 330 335

Lys Ser Ser Ser Cys Gln Gln His Ser Ser Arg Ser Ser Ser Val Asp
340 345 350

Tyr Ile Leu
355

<210> 9
<211> 725
<212> PRT
<213> Homo sapiens

<400> 9

Met Ser Phe Pro Lys Ala Pro Leu Lys Arg Phe Asn Asp Pro Ser Gly
1 5 10 15

Cys Ala Pro Ser Pro Gly Ala Tyr Asp Val Lys Thr Leu Glu Val Leu
20 25 30

Lys Gly Pro Val Ser Phe Gln Lys Ser Gln Arg Phe Lys Gln Gln Lys
35 40 45

Glu Ser Lys Gln Asn Leu Asn Val Asp Lys Asp Thr Thr Leu Pro Ala
50 55 60

Ser Ala Arg Lys Val Lys Ser Ser Glu Ser Lys Lys Glu Ser Gln Lys
65 70 75 80

```
Asn Asp Lys Asp Leu Lys Ile Leu Glu Lys Glu Ile Arg Val Leu Leu
                85                  90                  95

Gln Glu Arg Gly Ala Gln Asp Arg Arg Ile Gln Asp Leu Glu Thr Glu
            100                 105                 110

Leu Glu Lys Met Glu Ala Arg Leu Asn Ala Ala Leu Arg Glu Lys Thr
        115                 120                 125

Ser Leu Ser Ala Asn Asn Ala Thr Leu Glu Lys Gln Leu Ile Glu Leu
    130                 135                 140

Thr Arg Thr Asn Glu Leu Leu Lys Ser Lys Phe Ser Glu Asn Gly Asn
145                 150                 155                 160

Gln Lys Asn Leu Arg Ile Leu Ser Leu Glu Leu Met Lys Leu Arg Asn
                165                 170                 175

Lys Arg Glu Thr Lys Met Arg Gly Met Met Ala Lys Gln Glu Gly Met
            180                 185                 190

Glu Met Lys Leu Gln Val Thr Gln Arg Ser Leu Glu Glu Ser Gln Gly
        195                 200                 205

Lys Ile Ala Gln Leu Glu Gly Lys Leu Val Ser Ile Glu Lys Glu Lys
    210                 215                 220

Ile Asp Glu Lys Ser Glu Thr Glu Lys Leu Leu Glu Tyr Ile Glu Glu
225                 230                 235                 240

Ile Ser Cys Ala Ser Asp Gln Val Glu Lys Tyr Lys Leu Asp Ile Ala
                245                 250                 255

Gln Leu Glu Glu Asn Leu Lys Glu Lys Asn Asp Glu Ile Leu Ser Leu
            260                 265                 270

Lys Gln Ser Leu Glu Glu Asn Ile Val Ile Leu Ser Lys Gln Val Glu
        275                 280                 285

Asp Leu Asn Val Lys Cys Gln Leu Leu Glu Lys Glu Lys Glu Asp His
    290                 295                 300

Val Asn Arg Asn Arg Glu His Asn Glu Asn Leu Asn Ala Glu Met Gln
305                 310                 315                 320

Asn Leu Lys Gln Lys Phe Ile Leu Glu Gln Gln Glu Arg Glu Lys Leu
                325                 330                 335
```

```
Gln Gln Lys Glu Leu Gln Ile Asp Ser Leu Leu Gln Gln Glu Lys Glu
            340                 345                 350

Leu Ser Ser Ser Leu His Gln Lys Leu Cys Ser Phe Gln Glu Glu Met
        355                 360                 365

Val Lys Glu Lys Asn Leu Phe Glu Glu Glu Leu Lys Gln Thr Leu Asp
    370                 375                 380

Glu Leu Asp Lys Leu Gln Gln Lys Glu Glu Gln Ala Glu Arg Leu Val
385                 390                 395                 400

Lys Gln Leu Glu Glu Glu Ala Lys Ser Arg Ala Glu Glu Leu Lys Leu
                405                 410                 415

Leu Glu Glu Lys Leu Lys Gly Lys Glu Ala Glu Leu Glu Lys Ser Ser
            420                 425                 430

Ala Ala His Thr Gln Ala Thr Leu Leu Leu Gln Glu Lys Tyr Asp Ser
        435                 440                 445

Met Val Gln Ser Leu Glu Asp Val Thr Ala Gln Phe Glu Ser Tyr Lys
    450                 455                 460

Ala Leu Thr Ala Ser Glu Ile Glu Asp Leu Lys Leu Glu Asn Ser Ser
465                 470                 475                 480

Leu Gln Glu Lys Ala Ala Lys Ala Gly Lys Asn Ala Glu Asp Val Gln
                485                 490                 495

His Gln Ile Leu Ala Thr Glu Ser Ser Asn Gln Glu Tyr Val Arg Met
            500                 505                 510

Leu Leu Asp Leu Gln Thr Lys Ser Ala Leu Lys Glu Thr Glu Ile Lys
        515                 520                 525

Glu Ile Thr Val Ser Phe Leu Gln Lys Ile Thr Asp Leu Gln Asn Gln
    530                 535                 540

Leu Lys Gln Gln Glu Glu Asp Phe Arg Lys Gln Leu Glu Asp Glu Glu
545                 550                 555                 560

Gly Arg Lys Ala Glu Lys Glu Asn Thr Thr Ala Glu Leu Thr Glu Glu
                565                 570                 575

Ile Asn Lys Trp Arg Leu Leu Tyr Glu Glu Leu Tyr Asn Lys Thr Lys
            580                 585                 590
```

Pro Phe Gln Leu Gln Leu Asp Ala Phe Glu Val Glu Lys Gln Ala Leu
595 600 605

Leu Asn Glu His Gly Ala Ala Gln Glu Gln Leu Asn Lys Ile Arg Asp
610 615 620

Ser Tyr Ala Lys Leu Leu Gly His Gln Asn Leu Lys Gln Lys Ile Lys
625 630 635 640

His Val Val Lys Leu Lys Asp Glu Asn Ser Gln Leu Lys Ser Glu Val
645 650 655

Ser Lys Leu Arg Cys Gln Leu Ala Lys Lys Lys Gln Ser Glu Thr Lys
660 665 670

Leu Gln Glu Glu Leu Asn Lys Val Leu Gly Ile Lys His Phe Asp Pro
675 680 685

Ser Lys Ala Phe His His Glu Ser Lys Glu Asn Phe Ala Leu Lys Thr
690 695 700

Pro Leu Lys Glu Gly Asn Thr Asn Cys Tyr Arg Ala Pro Met Glu Cys
705 710 715 720

Gln Glu Ser Trp Lys
725

<210> 10
<211> 329
<212> PRT
<213> Homo sapiens

<400> 10

Met Asp Pro Gln Cys Thr Met Gly Leu Ser Asn Ile Leu Phe Val Met
1 5 10 15

Ala Phe Leu Leu Ser Gly Ala Ala Pro Leu Lys Ile Gln Ala Tyr Phe
20 25 30

Asn Glu Thr Ala Asp Leu Pro Cys Gln Phe Ala Asn Ser Gln Asn Gln
35 40 45

Ser Leu Ser Glu Leu Val Val Phe Trp Gln Asp Gln Glu Asn Leu Val
50 55 60

Leu Asn Glu Val Tyr Leu Gly Lys Glu Lys Phe Asp Ser Val His Ser
65 70 75 80

```
Lys Tyr Met Gly Arg Thr Ser Phe Asp Ser Asp Ser Trp Thr Leu Arg
                85                  90                  95

Leu His Asn Leu Gln Ile Lys Asp Lys Gly Leu Tyr Gln Cys Ile Ile
            100                 105                 110

His His Lys Lys Pro Thr Gly Met Ile Arg Ile His Gln Met Asn Ser
        115                 120                 125

Glu Leu Ser Val Leu Ala Asn Phe Ser Gln Pro Glu Ile Val Pro Ile
    130                 135                 140

Ser Asn Ile Thr Glu Asn Val Tyr Ile Asn Leu Thr Cys Ser Ser Ile
145                 150                 155                 160

His Gly Tyr Pro Glu Pro Lys Lys Met Ser Val Leu Leu Arg Thr Lys
                165                 170                 175

Asn Ser Thr Ile Glu Tyr Asp Gly Val Met Gln Lys Ser Gln Asp Asn
            180                 185                 190

Val Thr Glu Leu Tyr Asp Val Ser Ile Ser Leu Ser Val Ser Phe Pro
        195                 200                 205

Asp Val Thr Ser Asn Met Thr Ile Phe Cys Ile Leu Glu Thr Asp Lys
    210                 215                 220

Thr Arg Leu Leu Ser Ser Pro Phe Ser Ile Glu Leu Glu Asp Pro Gln
225                 230                 235                 240

Pro Pro Pro Asp His Ile Pro Trp Ile Thr Ala Val Leu Pro Thr Val
                245                 250                 255

Ile Ile Cys Val Met Val Phe Cys Leu Ile Leu Trp Lys Trp Lys Lys
            260                 265                 270

Lys Lys Arg Pro Arg Asn Ser Tyr Lys Cys Gly Thr Asn Thr Met Glu
        275                 280                 285

Arg Glu Glu Ser Glu Gln Thr Lys Lys Arg Glu Lys Ile His Ile Pro
    290                 295                 300

Glu Arg Ser Asp Glu Ala Gln Arg Val Phe Lys Ser Ser Lys Thr Ser
305                 310                 315                 320

Ser Cys Asp Lys Ser Asp Thr Cys Phe
```

325

<210> 11
<211> 1158
<212> DNA
<213> Homo sapiens

<400> 11

atgctggtcc gcaggggcgc gcgcgcaggg cccaggatgc cgcggggctg gaccgcgctt 60

tgcttgctga gtttgctgcc ttctgggttc atgagtcttg acaacaacgg tactgctacc 120

ccagagttac ctacccaggg aacattttca aatgtttcta caaatgtatc ctaccaagaa 180

actacaacac ctagtaccct tggaagtacc agcctgcacc ctgtgtctca acatggcaat 240

gaggccacaa caaacatcac agaaacgaca gtcaaattca catctacctc tgtgataacc 300

tcagtttatg gaaacacaaa ctcttctgtc cagtcacaga cctctgtaat cagcacagtg 360

ttcaccaccc cagccaacgt ttcaactcca gagacaacct tgaagcctag cctgtcacct 420

ggaaatgttt cagacctttc aaccactagc actagccttg caacatctcc cactaaaccc 480

tatacatcat cttctcctat cctaagtgac atcaaggcag aaatcaaatg ttcaggcatc 540

agagaagtga aattgactca gggcatctgc ctggagcaaa ataagacctc cagctgtgcg 600

gagtttaaga aggacagggg agagggcctg gcccgagtgc tgtgtgggga ggagcaggct 660

gatgctgatg ctggggccca ggtatgctcc ctgctccttg cccagtctga ggtgaggcct 720

cagtgtctac tgctggtctt ggccaacaga acagaaattt ccagcaaact ccaacttatg 780

aaaaagcacc aatctgacct gaaaaagctg gggatcctag atttcactga gcaagatgtt 840

gcaagccacc agagctattc ccaaaagacc ctgattgcac tggtcacctc gggagccctg 900

ctggctgtct tgggcatcac tggctatttc ctgatgaatc gccgcagctg gagccccaca 960

ggagaaaggc tgggcgaaga cccttattac acggaaaacg gtggaggcca gggctatagc 1020

tcaggacctg ggacctcccc tgaggctcag ggaaaggcca gtgtgaaccg aggggctcag 1080

gaaaacggga ccggccaggc cacctccaga aacggccatt cagcaagaca acacgtggtg 1140

gctgataccg aattgtga 1158

<210> 12
<211> 3915
<212> DNA
<213> Homo sapiens

<400> 12

```
atgtatttgt ggcttaaact cttggcattt ggctttgcct ttctggacac agaagtattt       60
gtgacagggc aaagcccaac accttccccc actggattga ctacagcaaa gatgcccagt      120
gttccacttt caagtgaccc cttacctact cacaccactg cattctcacc cgcaagcacc      180
tttgaaagag aaaatgactt ctcagagacc acaacttctc ttagtccaga caatacttcc      240
```

acccaagtat ccccggactc tttggataat gctagtgctt ttaataccac aggtgtttca 300
tcagtacaga cgcctcacct tcccacgcac gcagactcgc agacgccctc tgctggaact 360
gacacgcaga cattcagcgg ctccgccgcc aatgcaaaac tcaaccctac cccaggcagc 420
aatgctatct cagatgtccc aggagagagg agtacagcca gcacctttcc tacagaccca 480
gtttccccat tgacaaccac cctcagcctt gcacaccaca gctctgctgc cttacctgca 540
cgcacctcca acaccaccat cacagcgaac acctcagatg cctaccttaa tgcctctgaa 600
acaaccactc tgagcccttc tggaagcgct gtcatttcaa ccacaacaat agctactact 660
ccatctaagc caacatgtga tgaaaaatat gcaaacatca ctgtggatta cttatataac 720
aaggaaacta aattatttac agcaaagcta aatgttaatg agaatgtgga atgtggaaac 780
aatacttgca caaacaatga ggtgcataac cttacagaat gtaaaaatgc gtctgtttcc 840
atatctcata attcatgtac tgctcctgat aagacattaa tattagatgt gccaccaggg 900
gttgaaaagt ttcagttaca tgattgtaca caagttgaaa aagcagatac tactatttgt 960
ttaaaatgga aaaatattga aacctttact tgtgatacac agaatattac ctacagattt 1020
cagtgtggta atatgatatt tgataataaa gaaattaaat tagaaaacct tgaacccgaa 1080
catgagtata agtgtgactc agaaatactc tataataacc acaagtttac taacgcaagt 1140
aaaattatta aaacagattt tgggagtcca ggagagcctc agattatttt ttgtagaagt 1200
gaagctgcac atcaaggagt aattacctgg aatccccctc aaagatcatt tcataatttt 1260
accctctgtt atataaaaga gacagaaaaa gattgcctca atctggataa aaacctgatc 1320
aaatatgatt tgcaaaattt aaaaccttat acgaaatatg ttttatcatt acatgcctac 1380
atcattgcaa aagtgcaacg taatggaagt gctgcaatgt gtcatttcac aactaaaagt 1440
gctcctccaa gccaggtctg gaacatgact gtctccatga catcagataa tagtatgcat 1500
gtcaagtgta ggcctcccag ggaccgtaat ggcccccatg aacgttacca tttggaagtt 1560
gaagctggaa atactctggt tagaaatgag tcgcataaga attgcgattt ccgtgtaaaa 1620
gatcttcaat attcaacaga ctacactttt aaggcctatt ttcacaatgg agactatcct 1680
ggagaaccct ttattttaca tcattcaaca tcttataatt ctaaggcact gatagcattt 1740
ctggcatttc tgattattgt gacatcaata gccctgcttg ttgttctcta caaaatctat 1800
gatctacata agaaaagatc ctgcaattta gatgaacagc aggagcttgt tgaaagggat 1860
gatgaaaaac aactgatgaa tgtggagcca atccatgcag atattttgtt ggaaacttat 1920
aagaggaaga ttgctgatga aggaagactt tttctggctg aatttcagag catcccgcgg 1980
gtgttcagca agtttcctat aaaggaagct cgaaagccct ttaaccagaa taaaaaccgt 2040
tatgttgaca ttcttcctta tgattataac cgtgttgaac tctctgagat aaacggagat 2100

gcagggtcaa actacataaa tgccagctat attgatggtt tcaaagaacc caggaaatac 2160
attgctgcac aaggtcccag ggatgaaact gttgatgatt tctggaggat gatttgggaa 2220
cagaaagcca cagttattgt catggtcact cgatgtgaag aaggaaacag gaacaagtgt 2280
gcagaatact ggccgtcaat ggaagagggc actcgggctt ttggagatgt tgttgtaaag 2340
atcaaccagc acaaaagatg tccagattac atcattcaga aattgaacat tgtaaataaa 2400
aaagaaaaag caactggaag agaggtgact cacattcagt tcaccagctg gccagaccac 2460
ggggtgcctg aggatcctca cttgctcctc aaactgagaa ggagagtgaa tgccttcagc 2520
aatttcttca gtggtcccat tgtggtgcac tgcagtgctg gtgttgggcg cacaggaacc 2580
tatatcggaa ttgatgccat gctagaaggc ctggaagccg agaacaaagt ggatgtttat 2640
ggttatgttg tcaagctaag gcgacagaga tgcctgatgg ttcaagtaga ggcccagtac 2700
atcttgatcc atcaggcttt ggtggaatac aatcagtttg gagaaacaga agtgaatttg 2760
tctgaattac atccatatct acataacatg aagaaaaggg atccacccag tgagccgtct 2820
ccactagagg ctgaattcca gagacttcct tcatatagga gctggaggac acagcacatt 2880
ggaaatcaag aagaaaataa aagtaaaaac aggaattcta atgtcatccc atatgactat 2940
aacagagtgc cacttaaaca tgagctggaa atgagtaaag agagtgagca tgattcagat 3000
gaatcctctg atgatgacag tgattcagag gaaccaagca aatacatcaa tgcatctttt 3060
ataatgagct actggaaacc tgaagtgatg attgctgctc agggaccact gaaggagacc 3120
attggtgact tttggcagat gatcttccaa agaaaagtca aagttattgt tatgctgaca 3180
gaactgaaac atggagacca ggaaatctgt gctcagtact ggggagaagg aaagcaaaca 3240
tatggagata ttgaagttga cctgaaagac acagacaaat cttcaactta tacccttcgt 3300
gtctttgaac tgagacattc caagaggaaa gactctcgaa ctgtgtacca gtaccaatat 3360
acaaactgga gtgtggagca gcttcctgca gaacccaagg aattaatctc tatgattcag 3420
gtcgtcaaac aaaaacttcc ccagaagaat tcctctgaag ggaacaagca tcacaagagt 3480
acacctctac tcattcactg cagggatgga tctcagcaaa cgggaatatt ttgtgctttg 3540
ttaaatctct tagaaagtgc ggaaacagaa gaggtagtgg atatttttca agtggtaaaa 3600
gctctacgca aagctaggcc aggcatggtt tccacattcg agcaatatca attcctatat 3660
gacgtcattg ccagcaccta ccctgctcag aatggacaag taaagaaaaa caaccatcaa 3720
gaagataaaa ttgaatttga taatgaagtg gacaaagtaa agcaggatgc taattgtgtt 3780
aatccacttg gtgccccaga aaagctccct gaagcaaagg aacaggctga aggttctgaa 3840
cccacgagtg gcactgaggg gccagaacat tctgtcaatg gtcctgcaag tccagcttta 3900
aatcaaggtt catag 3915

<210> 13
<211> 486
<212> DNA
<213> Homo sapiens

<400> 13

```
atgaacctgg ccatcagcat cgctctcctg ctaacagtct tgcaggtctc ccgagggcag        60
aaggtgacca gcctaacggc ctgcctagtg gaccagagcc ttcgtctgga ctgccgccat       120
gagaatacca gcagttcacc catccagtac gagttcagcc tgacccgtga gacaaagaag       180
cacgtgctct ttggcactgt gggggtgcct gagcacacat accgctcccg aaccaacttc       240
accagcaaat acaacatgaa ggtcctctac ttatccgcct tcactagcaa ggacgagggc       300
acctacacgt gtgcactcca ccactctggc cattccccac ccatctcctc ccagaacgtc       360
acagtgctca gagacaaact ggtcaagtgt gagggcatca gcctgctggc tcagaacacc       420
tcgtggctgc tgctgctcct gctctccctc tccctcctcc aggccacgga tttcatgtcc       480
ctgtga                                                                  486
```

<210> 14
<211> 1137
<212> DNA
<213> Homo sapiens

<400> 14

```
atggtcctcc tttggctcac gctgctcctg atcgccctgc cctgtctcct gcaaacgaag      60
gaagatccaa acccaccaat cacgaaccta aggatgaaag caaaggctca gcagttgacc     120
tgggacctta acagaaatgt gaccgatatc gagtgtgtta aagacgccga ctattctatg     180
ccggcagtga acaatagcta ttgccagttt ggagcaattt ccttatgtga agtgaccaac     240
tacaccgtcc gagtggccaa cccaccattc tccacgtgga tcctcttccc tgagaacagt     300
gggaagcctt gggcaggtgc ggagaatctg acctgctgga ttcatgacgt ggatttcttg     360
agctgcagct gggcggtagg cccgggggcc cccgcggacg tccagtacga cctgtacttg     420
aacgttgcca acaggcgtca acagtacgag tgtcttcact acaaaacgga tgctcaggga     480
acacgtatcg ggtgtcgttt cgatgacatc tctcgactct ccagcggttc tcaaagttcc     540
cacatcctgg tgcggggcag gagcgcagcc ttcggtatcc cctgcacaga taagtttgtc     600
gtcttttcac agattgagat attaactcca cccaacatga ctgcaaagtg taataagaca     660
cattccttta tgcactggaa aatgagaagt catttcaatc gcaaatttcg ctatgagctt     720
cagatacaaa agagaatgca gcctgtaatc acagaacagg tcagagacag aacctccttc     780
cagctactca atcctggaac gtacacagta caaataagag cccgggaaag agtgtatgaa     840
ttcttgagcg cctggagcac cccccagcgc ttcgagtgcg accaggagga gggcgcaaac     900
acacgtgcct ggcggacgtc gctgctgatc gcgctgggga cgctgctggc cctggtctgt     960
gtcttcgtga tctgcagaag gtatctggtg atgcagagac tctttccccg catccctcac    1020
atgaaagacc ccatcggtga cagcttccaa aacgacaagc tggtggtctg ggaggcgggc    1080
aaagccggcc tggaggagtg tctggtgact gaagtacagg tcgtgcagaa aacttga       1137
```

<210> 15
<211> 903
<212> DNA
<213> Homo sapiens

<400> 15

atggccaact gcgagttcag cccggtgtcc ggggacaaac cctgctgccg gctctctagg 60
agagcccaac tctgtcttgg cgtcagtatc ctggtcctga tcctcgtcgt ggtgctcgcg 120
gtggtcgtcc cgaggtggcg ccagcagtgg agcggtccgg gcaccaccaa gcgctttccc 180
gagaccgtcc tggcgcgatg cgtcaagtac actgaaattc atcctgagat gagacatgta 240
gactgccaaa gtgtatggga tgctttcaag ggtgcattta tttcaaaaca tccttgcaac 300
attactgaag aagactatca gccactaatg aagttgggaa ctcagaccgt accttgcaac 360
aagattcttc tttggagcag aataaaagat ctggcccatc agttcacaca ggtccagcgg 420
gacatgttca ccctggagga cacgctgcta ggctaccttg ctgatgacct cacatggtgt 480
ggtgaattca acacttccaa aataaactat caatcttgcc cagactggag aaaggactgc 540
agcaacaacc ctgtttcagt attctggaaa acggtttccc gcaggtttgc agaagctgcc 600
tgtgatgtgg tccatgtgat gctcaatgga tcccgcagta aaatctttga caaaaacagc 660
acttttggga gtgtggaagt ccataatttg caaccagaga aggttcagac actagaggcc 720
tgggtgatac atggtggaag agaagattcc agagacttat gccaggatcc caccataaaa 780
gagctggaat cgattataag caaaaggaat attcaatttt cctgcaagaa tatctacaga 840
cctgacaagt ttcttcagtg tgtgaaaaat cctgaggatt catcttgcac atctgagatc 900
tga 903

<210> 16
<211> 1671
<212> DNA
<213> Homo sapiens

<400> 16

atgccacctc ctcgcctcct cttcttcctc ctcttcctca cccccatgga agtcaggccc 60
gaggaacctc tagtggtgaa ggtggaagag ggagataacg ctgtgctgca gtgcctcaag 120
gggacctcag atggccccac tcagcagctg acctggtctc gggagtcccc gcttaaaccc 180
ttcttaaaac tcagcctggg gctgccaggc ctgggaatcc acatgaggcc cctggccatc 240
tggcttttca tcttcaacgt ctctcaacag atggggggct tctacctgtg ccagccgggg 300
ccccccctctg agaaggcctg gcagcctggc tggacagtca atgtggaggg cagcggggag 360

```
ctgttccggt ggaatgtttc ggacctaggt ggcctgggct gtggcctgaa gaacaggtcc      420
tcagagggcc ccagctcccc ttccgggaag ctcatgagcc ccaagctgta tgtgtgggcc      480
aaagaccgcc ctgagatctg ggagggagag cctccgtgtc tcccaccgag ggacagcctg      540
aaccagagcc tcagccagga cctcaccatg gcccctggct ccacactctg gctgtcctgt      600
ggggtacccc ctgactctgt gtccaggggc cccctctcct ggacccatgt gcaccccaag      660
gggcctaagt cattgctgag cctagagctg aaggacgatc gcccggccag agatatgtgg      720
gtaatggaga cgggtctgtt gttgccccgg gccacagctc aagacgctgg aaagtattat      780
tgtcaccgtg gcaacctgac catgtcattc cacctggaga tcactgctcg gccagtacta      840
tggcactggc tgctgaggac tggtggctgg aaggtctcag ctgtgacttt ggcttatctg      900
atcttctgcc tgtgttccct tgtgggcatt cttcatcttc aaagagccct ggtcctgagg      960
aggaaaagaa agcgaatgac tgaccccacc aggagattct tcaaagtgac gcctccccca     1020
ggaagcgggc cccagaacca gtacgggaac gtgctgtctc tccccacacc cacctcaggc     1080
ctcggacgcg cccagcgttg ggccgcaggc ctgggggcca ctgccccgtc ttatggaaac     1140
ccgagcagcg acgtccaggc ggatggagcc ttggggtccc ggagcccgcc gggagtgggc     1200
ccagaagaag aggaagggga gggctatgag gaacctgaca gtgaggagga ctccgagttc     1260
tatgagaacg actccaacct tgggcaggac cagctctccc aggatggcag cggctacgag     1320
aaccctgagg atgagcccct gggtcctgag gatgaagact ccttctccaa cgctgagtct     1380
tatgagaacg aggatgaaga gctgacccag ccggtcgcca ggacaatgga cttcctgagc     1440
cctcatgggt cagcctggga ccccagccgg gaagcaacct ccctggggtc ccagtcctat     1500
gaggatatga gaggaatcct gtatgcagcc ccccagctcc gctccattcg gggccagcct     1560
ggacccaatc atgaggaaga tgcagactct tatgagaaca tggataatcc cgatgggcca     1620
gacccagcct ggggaggagg gggccgcatg ggcacctgga gcaccaggtg a              1671
```

<210> 17
<211> 972
<212> DNA
<213> Homo sapiens

<400> 17

```
atgtggcccc tggtagcggc gctgttgctg ggctcggcgt gctgcggatc agctcagcta      60
ctatttaata aaacaaaatc tgtagaattc acgttttgta atgacactgt cgtcattcca     120
tgctttgtta ctaatatgga ggcacaaaac actactgaag tatacgtaaa gtggaaattt     180
aaaggaagag atatttacac ctttgatgga gctctaaaca agtccactgt ccccactgac     240
tttagtagtg caaaaattga agtctcacaa ttactaaaag gagatgcctc tttgaagatg     300
gataagagtg atgctgtctc acacacagga aactacactt gtgaagtaac agaattaacc     360
agagaaggtg aaacgatcat cgagctaaaa tatcgtgttg tttcatggtt ttctccaaat     420
gaaaatattc ttattgttat tttcccaatt tttgctatac tcctgttctg ggacagtttt     480
ggtattaaaa cacttaaata tagatccggt ggtatggatg agaaaacaat tgctttactt     540
gttgctggac tagtgatcac tgtcattgtc attgttggag ccattctttt cgtcccaggt     600
gaatattcat taaagaatgc tactggcctt ggtttaattg tgacttctac agggatatta     660
atattacttc actactatgt gtttagtaca gcgattggat taacctcctt cgtcattgcc     720
atattggtta ttcaggtgat agcctatatc ctcgctgtgg ttggactgag tctctgtatt     780
gcggcgtgta taccaatgca tggccctctt ctgatttcag gtttgagtat cttagctcta     840
gcacaattac ttggactagt ttatatgaaa tttgtggctt ccaatcagaa gactatacaa     900
cctcctagga aagctgtaga ggaacccctt aatgcattca aagaatcaaa aggaatgatg     960
aatgatgaat aa                                                         972
```

<210> 18
<211> 1487
<212> DNA
<213> Homo sapiens

<400> 18

tttgtagtgg gaggatacct ccagagaggc tgctgctcat tgagctgcac tcacatgagg 60
atacagactt tgtgaagaag gaattggcaa cactgaaacc tccagaacaa aggctgtcac 120
taaggtcccg ctgccttgat ggattataca cttgacctca gtgtgacaac agtgaccgac 180
tactactacc ctgatatctt ctcaagcccc tgtgatgcgg aacttattca gacaaatggc 240
aagttgctcc ttgctgtctt ttattgcctc ctgtttgtat tcagtcttct gggaaacagc 300
ctggtcatcc tggtccttgt ggtctgcaag aagctgagga gcatcacaga tgtatacctc 360
ttgaacctgg ccctgtctga cctgcttttt gtcttctcct tcccctttca gacctactat 420
ctgctggacc agtgggtgtt tgggactgta atgtgcaaag tggtgtctgg cttttattac 480
attggcttct acagcagcat gtttttcatc accctcatga gtgtggacag gtacctggct 540
gttgtccatg ccgtgtatgc cctaaaggtg aggacgatca ggatgggcac aacgctgtgc 600
ctggcagtat ggctaaccgc cattatggct accatcccat tgctagtgtt ttaccaagtg 660
gcctctgaag atggtgttct acagtgttat tcattttaca atcaacagac tttgaagtgg 720
aagatcttca ccaacttcaa aatgaacatt ttaggcttgt tgatcccatt caccatcttt 780
atgttctgct acattaaaat cctgcaccag ctgaagaggt gtcaaaacca caacaagacc 840
aaggccatca ggttggtgct cattgtggtc attgcatctt tacttttctg ggtcccattc 900
aacgtggttc ttttcctcac ttccttgcac agtatgcaca tcttggatgg atgtagcata 960
agccaacagc tgacttatgc cacccatgtc acagaaatca tttcctttac tcactgctgt 1020

gtgaaccctg ttatctatgc ttttgttggg gagaagttca agaaacacct ctcagaaata 1080
tttcagaaaa gttgcagcca aatcttcaac tacctaggaa gacaaatgcc tagggagagc 1140
tgtgaaaagt catcatcctg ccagcagcac tcctcccgtt cctccagcgt agactacatt 1200
ttgtgaggat caatgaagac taaatataaa aaacattttc ttgaatggca tgctagtagc 1260
agtgagcaaa ggtgtgggtg tgaaaggttt ccaaaaaaag ttcagcatga aggatgccat 1320
atatgttgtt gccaacactt ggaacacaat gactaaagac atagttgtgc atgcctggca 1380
caacatcaag cctgtgattg tgtttattga tgatgttgaa caagtggtaa ctttaaagga 1440
ttctgtatgc caagtgaaaa aaaaagatgt ctgacctcct tacatat 1487

<210> 19
<211> 3144
<212> DNA
<213> Homo sapiens

<400> 19

attctttctt cgtgttcctg tgcgggattg gtgtgcccag gggtttggct ttccaattgg 60
ctaacgccgg ggtgggtggg gaatgtgggg agatttgaat ttgaaaccgg tagggagtga 120
taatccgcat tcagttgtcg aggagtgcca gtcaccttca gtttctggag ctggccgtca 180
acatgtcctt tcctaaggcg cccttgaaac gattcaatga cccttctggt tgtgcaccat 240
ctccaggtgc ttatgatgtt aaaactttag aagtattgaa aggaccagta tcctttcaga 300
aatcacaaag atttaaacaa caaaaagaat ctaaacaaaa tcttaatgtt gacaaagata 360
ctaccttgcc tgcttcagct agaaaagtta agtcttcgga atcaaagaag gaatctcaaa 420
agaatgataa agatttgaag atattagaga aagagattcg tgttcttcta caggaacgtg 480
gtgcccagga caggcggatc caggatctgg aaactgagtt ggaaaagatg gaagcaaggc 540
taaatgctgc actaagggaa aaaacatctc tctctgcaaa taatgctaca ctggaaaaac 600
aacttattga attgaccagg actaatgaac tactaaaatc taagttttct gaaaatggta 660
accagaagaa tttgagaatt ctaagcttgg agttgatgaa acttagaaac aaaagagaaa 720
caaagatgag ggtatgatg gctaagcaag aaggcatgga gatgaagctg caggtcaccc 780
aaaggagtct cgaagagtct caagggaaaa tagcccaact ggagggaaaa cttgtttcaa 840
tagagaaaga aaagattgat gaaaaatctg aaacagaaaa actcttggaa tacatcgaag 900
aaattagttg tgcttcagat caagtggaaa aatacaagct agatattgcc cagttagaag 960
aaaatttgaa agagaagaat gatgaaattt taagccttaa gcagtctctt gaggagaata 1020
ttgttatatt atctaaacaa gtagaagatc taaatgtgaa atgtcagctg cttgaaaaag 1080
aaaaagaaga ccatgtcaac aggaatagag aacacaacga aaatctaaat gcagagatgc 1140
aaaacttaaa acagaagttt attcttgaac aacaggaacg tgaaaagctt caacaaaaag 1200

aattacaaat tgattcactt ctgcaacaag agaaagaatt atcttcgagt cttcatcaga 1260
agctctgttc ttttcaagag gaaatggtta aagagaagaa tctgtttgag gaagaattaa 1320
agcaaacact ggatgagctt gataaattac agcaaaagga ggaacaagct gaaaggctgg 1380
tcaagcaatt ggaagaggaa gcaaaatcta gagctgaaga attaaaactc ctagaagaaa 1440
agctgaaagg gaaggaggct gaactggaga aaagtagtgc tgctcatacc caggccaccc 1500
tgcttttgca ggaaaagtat gacagtatgg tgcaaagcct tgaagatgtt actgctcaat 1560
ttgaaagcta taaagcgtta acagccagtg agatagaaga tcttaagctg gagaactcat 1620
cattacagga aaaagcggcc aaggctggga aaaatgcaga ggatgttcag catcagattt 1680
tggcaactga gagctcaaat caagaatatg taaggatgct tctagatctg cagaccaagt 1740
cagcactaaa ggaaacagaa attaaagaaa tcacagtttc ttttcttcaa aaaataactg 1800
atttgcagaa ccaactcaag caacaggagg aagactttag aaaacagctg gaagatgaag 1860
aaggaagaaa agctgaaaaa gaaaatacaa cagcagaatt aactgaagaa attaacaagt 1920
ggcgtctcct ctatgaagaa ctatataata aaacaaaacc ttttcagcta caactagatg 1980
cttttgaagt agaaaaacag gcattgttga atgaacatgg tgcagctcag gaacagctaa 2040
ataaaataag agattcatat gctaaattat tgggtcatca gaatttgaaa caaaaaatca 2100
agcatgttgt gaagttgaaa gatgaaaata gccaactcaa atcggaagta tcaaaactcc 2160
gctgtcagct tgctaaaaaa aaacaaagtg agacaaaact tcaagaggaa ttgaataaag 2220
ttctaggtat caaacacttt gatccttcaa aggcttttca tcatgaaagt aaagaaaatt 2280
ttgccctgaa gaccccatta aaagaaggca atacaaactg ttaccgagct cctatggagt 2340
gtcaagaatc atggaagtaa acatctgaga aacctgttga agattatttc attcgtcttg 2400
ttgttattga tgttgctgtt attatatttg acatgggtat tttataatgt tgtatttaat 2460
tttaactgcc aatccttaaa tatgtgaaag gaacattttt taccaaagtg tcttttgaca 2520
ttttattttt tcttgcaaat acctcctccc taatgctcac ctttatcacc tcattctgaa 2580
ccctttcgct ggctttccag cttagaatgc atctcatcaa cttaaaagtc agtatcatat 2640
tattatcctc ctgttctgaa accttagttt caagagtcta aaccccagat tcttcagctt 2700
gatcctggag gtcttttcta gtctgagctt ctttagctag gctaaaacac cttggcttgt 2760
tattgcctct actttgattc tgataatgct cacttggtcc tacctattat ccttctactt 2820
gtccagttca aataagaaat aaggacaagc ctaacttcat agaaacctct ctatttttaa 2880
tcagttgttt aataatttac aggttcttag gctccatcct gtttgtatga aattataatc 2940
tgtggattgg cctttaagcc tgcattctta acaaactctt cagttaattc ttagatacac 3000
taaaaatctg agaaactcta catgtaacta tttcttcaga gtttgtcata tactgcttgt 3060

catctgcatg tctactcagc atttgattaa catttgtgta atatgaaata aaattacaca 3120

gtaagtcatt taaccaatta aaaa 3144

<210> 20
<211> 2784
<212> DNA
<213> Homo sapiens

<400> 20

```
ggaaggcttg cacagggtga aagctttgct tctctgctgc tgtaacaggg actagcacag      60
acacacggat gagtggggtc atttccagat attaggtcac agcagaagca gccaaaatgg     120
atccccagtg cactatggga ctgagtaaca ttctctttgt gatggccttc ctgctctctg     180
gtgctgctcc tctgaagatt caagcttatt tcaatgagac tgcagacctg ccatgccaat     240
ttgcaaactc tcaaaaccaa agcctgagtg agctagtagt attttggcag gaccaggaaa     300
acttggttct gaatgaggta tacttaggca aagagaaatt tgacagtgtt cattccaagt     360
atatgggccg cacaagtttt gattcggaca gttggaccct gagacttcac aatcttcaga     420
tcaaggacaa gggcttgtat caatgtatca tccatcacaa aaagcccaca ggaatgattc     480
gcatccacca gatgaattct gaactgtcag tgcttgctaa cttcagtcaa cctgaaatag     540
taccaatttc taatataaca gaaaatgtgt acataaattt gacctgctca tctatacacg     600
gttacccaga acctaagaag atgagtgttt tgctaagaac caagaattca actatcgagt     660
atgatggtgt tatgcagaaa tctcaagata atgtcacaga actgtacgac gtttccatca     720
gcttgtctgt ttcattccct gatgttacga gcaatatgac catcttctgt attctggaaa     780
ctgacaagac gcggctttta tcttcacctt tctctataga gcttgaggac cctcagcctc     840
ccccagacca cattccttgg attacagctg tacttccaac agttattata tgtgtgatgg     900
ttttctgtct aattctatgg aaatggaaga agaagaagcg gcctcgcaac tcttataaat     960
gtggaaccaa cacaatggag agggaagaga gtgaacagac caagaaaaga gaaaaaatcc    1020
atatacctga aagatctgat gaagcccagc gtgtttttaa aagttcgaag acatcttcat    1080
gcgacaaaag tgatacatgt ttttaattaa agagtaaagc ccatacaagt attcattttt    1140
tctacccttt cctttgtaag ttcctgggca acctttttga tttcttccag aaggcaaaaa    1200
gacattacca tgagtaataa gggggctcca ggactccctc taagtggaat agcctccctg    1260
taactccagc tctgctccgt atgccaagag gagactttaa ttctcttact gcttcttttc    1320
acttcagagc acacttatgg gccaagccca gcttaatggc tcatgacctg gaaataaaat    1380
ttaggaccaa tacctcctcc agatcagatt cttctcttaa tttcatagat tgtgtttttt    1440
ttttaaatag acctctcaat ttctggaaaa ctgcctttta tctgcccaga attctaagct    1500
ggtgccccac tgaattttgt gtgtacctgt gactaaacaa ctacctcctc agtctgggtg    1560
```

```
ggacttatgt atttatgacc ttatagtgtt aatatcttga aacatagaga tctatgtact     1620
gtaatagtgt gattactatg ctctagagaa aagtctaccc ctgctaagga gttctcatcc     1680
ctctgtcagg gtcagtaagg aaaacggtgg cctagggtac aggcaacaat gagcagacca     1740
acctaaattt ggggaaatta ggagaggcag agatagaacc tggagccact tctatctggg     1800
ctgttgctaa tattgaggag gcttgcccca cccaacaagc catagtggag agaactgaat     1860
aaacaggaaa atgccagagc ttgtgaaccc tgtttctctt gaagaactga ctagtgagat     1920
ggcctgggga agctgtgaaa gaaccaaaag agatcacaat actcaaaaga gagagagaga     1980
gaaaaaagag agatcttgat ccacagaaat acatgaaatg tctggtctgt ccaccccatc     2040
aacaagtctt gaaacaagca acagatggat agtctgtcca aatggacata agacagacag     2100
cagtttccct ggtggtcagg gaggggtttt ggtgataccc aagttattgg gatgtcatct     2160
tcctggaagc agagctgggg agggagagcc atcaccttga taatgggatg aatggaagga     2220
ggcttaggac tttccactcc tggctgagag aggaagagct gcaacggaat taggaagacc     2280
aagacacaga tcacccgggg cttacttagc ctacagatgt cctacgggaa cgtgggctgg     2340
cccagcatag ggctagcaaa tttgagttgg atgattgttt ttgctcaagg caaccagagg     2400
aaacttgcat acagagacag atatactggg agaaatgact ttgaaaacct ggctctaagg     2460
tgggatcact aagggatggg gcagtctctg cccaaacata aagagaactc tggggagcct     2520
gagccacaaa aatgttcctt tattttatgt aaaccctcaa gggttataga ctgccatgct     2580
agacaagctt gtccatgtaa tattcccatg tttttaccct gcccctgcct tgattagact     2640
cctagcacct ggctagtttc taacatgttt tgtgcagcac agtttttaat aaatgcttgt     2700
tacattcaaa aaaaaaaaaa aaaaaaaaaa aaaaaaaaaa aaaaaaaaaa aaaaaaaaaa     2760
aaaaaaaaaa aaaaaaaaaa aaaa                                            2784
```

## Description

The present disclosure relates to diagnostic markers of acute myeloid leukaemia, and to the use of a diagnostic screen based on said markers in diagnostic and prognostic methods.

Human Acute Myeloid Leukaemia (AML) is an aggressive cancer of white blood cells and is the most common adult acute leukaemia. In more detail, AML is a cancer of the myeloid line of blood cells. It is characterized by the rapid growth of an abnormal white blood cell population. Approximately 80% of AML patients are over the age of 60 and the overall survival of this patient group lies at only approximately 5%.

AML can be classified into several subgroups. By way of example, classification according to the World Health Organization (WHO) criteria is based on examination of bone marrow aspirate or a blood sample via light microscopy. Alternatively, bone marrow or blood may be tested for chromosomal translocations by routine cytogenetic methods or fluorescent *in situ* hybridisation (FISH), and for specific genetic mutations (such as mutations in the *FLT3, NPM1* and *CEBPA* genes) may be detected by polymerase chain reaction (PCR). Immunophenotyping is another method that may be used to identify the AML subtype, which involves detection of cell surface and cytoplasmic markers using flow cytometry.

Flow cytometry is a technique for counting and examining microscopic particles such as cells by suspending them in a stream of fluid and capturing the light that emerges from each cell as it passes through a laser beam. Cell surface molecules often referred to as "cluster of differentiation" (CD) molecules may be exploited in flow cytometry to characterise cell populations. For example, in fluorescence-activated cell sorting, a diagnostic antibody (labelled with a fluorophore) is employed, which binds to a surface molecule (e.g. a CD molecule) present on and characteristic of the cell population in question. Thereafter, the flourophore (attached to the antibody) is activated by a laser beam and the fluorescence signal detected by the flow cytometer. In this manner, fluorescently-labelled antibodies can be used to detect and sort cells displaying a specific CD molecule (or set of CD molecules).

Current AML therapies typically involve induction chemotherapy followed by post-induction therapy. The goal of induction chemotherapy is to reduce the amount of leukaemic cells to less than 5% of all the nucleated cells in a bone marrow sample. Regrettably, this level of reduction of leukaemic cells is not enough to prevent disease recurrence (i.e. relapse) and almost all patients relapse without post-induction therapy. Post-induction therapy typically involves further cycles of chemotherapy, and in some cases, a hematopoietic stem cell transplant that aims to eliminate minimal residual disease (MRD). MRD is the population of leukaemic cells that is recaltricant to therapy. It is thought that this population of cells contains a sub-population of cells termed a leukaemic stem cell (LSC) population that is largely quiescent and serves to sustain disease.

Current methods used to detect MRD include real time quantitative PCR (RQ-PCR) or by multi-parameter flow cytometry (MFC). However, RQ-PCR based MRD assessment is not possible in approximately half of patients with AML. In addition, and despite recent technical developments, there is still a lack of a validated MFC methodology demonstrating clinical utility - current sensitivity levels of MFC are at least 1 log below real time that of RQ-PCR assays.

There is, therefore, a need to provide an alternative and/ or improved diagnostic screen for acute myeloid leukaemia. In addition, there is a need to provide an alternative and/ or improved method for diagnosis and/or prognosis of acute myeloid leukaemia. In particular, there is a need to provide an alternative and/ or improved method to detect and monitor MRD for acute myeloid leukaemia.

US 2007/0111238 A1 describes methods for diagnosing and evaluating treatment of blood disorders.

WO 2010/102244 A1 describes treatment of leukemias and chronic myeloproliferative diseases with antibodies to EphA3.

Jordan et al (2000), Leukemia, 14, 10, 1777-1784 describes the interluekin-3 receptor alpha as a marker for human acute myelogenous leukemia stem cells.

The present invention solves one or more of the above mentioned problems.

The invention is defined by the claims.

A diagnostic screen for detecting acute myeloid leukaemia, wherein said screen detects the presence (+) or absence (-), as indicated below, of the following cell surface polypeptide markers:

i) $CD34^+$;
ii) $CD45RA^+$; and
iii) $CD90^-$ and/ or $CD123^+$ is described herein.

A cell surface polypeptide marker may be displayed (at least in part) on the extracellular surface of a cell. Markers of the present invention may include CD34, CD45RA, CD90, CD123, CD38, CD19, CD47, CCR8, RHAMM and/or CD86. CD34 is a heavily glycosylated, 105-120 kDa transmembrane glycoprotein expressed on hematopoietic progenitor cells, vascular endothelial cells and some fibroblasts. The CD34 cytoplasmic domain is a target for phosphorylation by activated protein kinase C, suggesting a role for CD34 in signal transduction. CD34 may also play a role in adhesion of certain antigens to endothelium. CD45R, also designated CD45 and PTPRC, has been identified as a transmembrane glycoprotein, broadly expressed among hematopoietic cells. Multiple isoforms of CD45R are distributed throughout the immune system according to cell type including CD45RA. CD45R functions as a phosphotyrosine phosphatase, a vital component for efficient tyrosine phosphorylation induction by the TCR/CD3 complex. CD90 is a 25-37 kDa heavily N-glycosylated, glycophosphatidylinositol (GPI) anchored conserved cell surface protein originally discovered as a thymocyte antigen. The CD123 antigen (also known as interleukin-3 receptor) is a molecule found on cells which helps transmit the signal of interleukin-3, a soluble cytokine important in the immune system. CD38, also known as cyclic ADP ribose hydrolase is a glycoprotein found on the surface of many immune cells (white blood cells. CD38 is thought to function in cell adhesion, signal transduction and calcium signaling. CD19 is a 95 kDa type-I transmembrane glycoprotein that belongs to the immunglobulin superfamily. It is expressed on B cells throughout most stages of B cell differentiation and associates with CD21, CD81, and CD225 (Leu-13) forming a signal transduction complex. CD19 functions as a regulator in B cell development, activation, and differentiation. CD47 is an integral membrane protein that plays a role in the regulation of cation fluxes across cell membranes. It is also a receptor for the C-terminal cell binding domain of thrombospondin (SIRP). CD47 is expressed on hemopoietic cells, epithelial cells, endothelial cells, fibroblasts, brain and mesenchymal cells. Chemokine receptor 8, also known as CCR8 is a member of the beta chemokine receptor family CCR8. HMMR hyaluronan-mediated motility receptor (RHAMM) is a cell surface receptor. The CD86 gene encodes a type I membrane protein that is expressed on antigen-presenting cells.

The present inventors have unexpectedly found that a combination of the above-mentioned cell surface markers represents a robust diagnostic screen for acute myeloid leukaemia. Diagnostic capacity in this context may also embrace prognostic capacity and diagnosis/ monitoring of MRD.

A screen as defined above has many useful applications including diagnostic and prognostic applications such as in clinical guidance and for determining therapy, for patient management and for assessing treatment efficacy.

A diagnostic screen as defined above, wherein the marker iii) is $CD90^-$ is described herein.

A diagnostic screen as defined above, wherein the marker iii) is $CD123^+$ is described herein.

A diagnostic screen as defined above, wherein the marker iii) is $CD90^-$ and $CD123^+$ is described herein.

A diagnostic screen as defined above, further comprising the cell surface polypeptide marker CD38+ is described herein. A diagnostic screen as defined above, further comprising the cell surface polypeptide marker $CD38^-$ (i.e. $CD38^-$ instead of $CD38^+$) is described herein.

A diagnostic screen as defined above, further comprising one or more (or two or more, or three or more, or four or more) of the cell surface polypeptide markers selected from $CD19^-$, $CD47^{+/-}$, $CCRS^{+/-}$, $CD86^-$ and/or $RHAMM^{+/-}$ is described herein. A diagnostic screen as defined above, comprising the cell surface polypeptide marker CD47+ is described herein. A diagnostic screen as defined above, comprising the cell surface polypeptide marker $CD47^-$ (i.e. $CD47^-$ instead of $CD47^+$) is described herein. A diagnostic screen as defined above, comprising the cell surface polypeptide marker CCR8+ is described herein. A diagnostic screen as defined above, comprising the cell surface polypeptide marker $CCR8^-$ (i.e. $CCR8^-$ instead of $CCR8^+$) is described herein. A diagnostic screen as defined above, comprising the cell surface polypeptide marker RHAMM+ is described herein. A diagnostic screen as defined above, comprising the cell surface polypeptide marker $RHAMM^-$ (i.e. $RHAMM^-$ instead of $RHAMM^+$) is described herein.

A diagnostic screen that comprises one or more antibodies that bind to one or more of the identified markers is described herein. Thus, said one or more antibodies may be used to confirm the presence (+) or absence (-) of said cell surface polypeptide markers. The presence (+) of a marker refers to an elevation in the levels of marker in a sample above a background level. Likewise, the absence (-) of a marker refers to a reduction in the levels of a marker in a sample below a background level. The elevation in the levels of marker in a sample above a background level is 1 or more (such as 2, 3, 4, 5, 6, 7, 8, 10, 15, 20, 25) flourescence units. A reduction in the levels of a marker in a sample below a background level may be 1 or more (such as 2, 3, 4, 5, 6, 7, 8, 10, 15, 20, 25) flourescence units. In this regard, it would be routine for a skilled person in the art to determine the background level of marker expression in a sample. Thus, said cell surface polypeptide markers may be detected by specific binding of said one or more antibodies.

A screen that comprises one or more antibodies that bind to one or more cell surface polypeptide markers selected from CD34, CD45RA, CD90, CD123, CD38, CD19, CD47, CCR8, CD86 and/or RHAMM is described herein.

A screen that comprises a first antibody that binds to CD34, a second antibody that binds to CD45RA, and a third antibody that binds to CD90 and/ or to CD123 is described herein. A screen comprises a first antibody that binds to CD34, a second antibody that binds to CD45RA, a third antibody that binds to CD90 and/ or to CD123, and a fourth antibody that binds to CD38 is described herein.

Any one or more of said antibodies may bind to one of said markers and not (substantially) to any of the other markers. For example, each of the employed antibodies may bind to one of said markers and not (substantially) to any of the other markers. Alternatively, any one or more of said antibodies may bind to two, three, four, five, six ,seven, eight, nine or all ten of said markers.

A screen that comprises three antibodies, wherein:

a first antibody binds to CD34 and preferably not to CD45, CD90 and/or CD123;
a second antibody that binds to CD45RA and preferably not to CD34 CD90 and/or CD123; and
a third antibody that binds to CD90 and preferably not to CD34, CD45RA and/or CD123 is described herein.

A screen that comprises three antibodies, wherein:

a first antibody binds to CD34 and preferably not to CD45, CD90 and/or CD123;
a second antibody that binds to CD45RA and preferably not to CD34 CD90 and/or CD123; and
a third antibody that binds to CD123 and preferably not to CD34, CD45RA and/or CD90 is described herein.

The antibodies described herein may recognise and bind to specific epitopes of the above mentioned cell surface polypeptide markers. For example, an antibody may bind to an epitope in the N-terminal/ C-terminal/ mid-region domains/ extracellular domains of CD34/ CD45RA/ CD90/ CD123/ CD38/ CD19/ CD47/ CD86/ CCR8 and/or RHAMM. The sequence of CD34, CD45RA, CD90, CD123, CD38, CD19, CD47, CD86/ CCR8 and RHAMM are available from the NCBI website (http://www.ncbi.nlm.nih.gov/projects/genome/assembly/grc/human/index.shtml). These protein sequences are provided as SEQ ID NOs: 1-10. The corresponding nucleic acid sequences are provided as SEQ ID NOs: 11-20.

The antibodies described herein may bind to a CD34/ CD45RA/ CD90/ CD123/ CD38/ CD19/ CD47/ CCR8, CD86 and/or RHAMM molecules comprising an amino acid sequence having at least 80% (such at least 85%, 90%, 95%, 98%, 99% or 100%) sequence identity to SEQ ID NOs: 1-10, or a fragment thereof.

Conventional methods for determining nucleic acid sequence identity are discussed in more detail later in the specification.

The antibodies may be polyclonal and/ or monoclonal antibodies.

An antibody that binds to one of the above-mentioned cell surface polypeptide markers may be one capable of binding that marker with sufficient affinity such that the antibody is useful as a diagnostic/ and or prognostic agent. In one embodiment, the term 'binds' is equivalent to 'specifically binds'. An antibody that binds/ specifically binds to a cell surface polypeptide marker of interest is one that binds to one of the above mentioned markers with an affinity (Ka) of at least $10^4$ M.

Suitable antibodies may include FITC or PE-Cy7 conjugated anti-CD38, PE or FITC-conjugated anti-CD45RA, PE-Cy7-conjugated or APC conjugated anti-CD123, biotin-conjugated anti-CD90, PE-Cy5 or PERCP-conjugated anti-CD34, PE-conjugated CD47, CD19 Horizon V450 and APC-Alexa Fluor 750 or APC-eFluor 780 conjugated streptavidin which are available from a number of different commercial suppliers including BD Biosciences Europe ebioscience, Beckman Coulter and Pharmingen.

The antibody may be a labelled antibody, such as a fluorescently labelled antibody. Suitable labelled compounds include conventionally known labelled compounds, such as fluorescent substances such as cyanine dyes Cy3 (registered trademark of Amersham Life Science), fluorescein isothiacyanate (FITC), allophycocyanin (APC), rhodamine, Phycoerythrin (PE), PE-Cy5 (Phycoerythrin-Cy5), PE-Cy7 (Phycoerythrin-Cy7), APC-Alexa Fluor 750, APC-eFluor 780, Pacific Blue, Horizon V450 and quantum dot, biotin-conjugated; light scattering substances such as gold particles; photoabsorptive substances such as ferrite; radioactive substances such as <125> I; and enzymes such as peroxidase or alkali phosphatase.

Different antibodies may be labelled respectively with mutually distinguishable labels. Labelling may be conducted by binding a labelled compound directly to each antibody. Preferably, the antibodies are labelled with different fluorescent dyes with different fluorescence wavelengths to enable easy discrimination from one another. For example a first antibody may be labelled in red (for example PE-Cy5), a second antibody in orange (for example PI, APC, R-PE) and a third antibody in green (for example Alexa488, FITC). Suitable labelling strategies are routine and known to a person skilled in the art. By way of example, the Lightening Link™ antibody labeling kit may be used (Innova Biosciences, UK).

Methods suitable for detection of the cell surface polypeptide markers described herein using labelled antibodies are conventional techniques known to those skilled in the art. For example, when a fluorescent label is used, an antibody that specifically binds to a marker may be detected by observing the emitted fluorescence colour under a microscope. A fluorescent label can also be detected by irradiating a sample with an exciting light - if the label is present, fluorescence is emitted from the sample. Thus, whether a cell is positive or negative for a particular cell surface marker may be judged by using a labelled antibody specific for said marker and observing the emitted fluorescence colour under a microscope. In a preferred embodiment of the invention, fluorescence-activated cell sorting (FACS) is used for detection of the cell surface polypeptide markers/ labeled antibodies of the present invention.

A screen (as defined above) for use in a method of diagnosis of acute myeloid leukaemia is described herein.

In one aspect, the invention provides a method for diagnosing acute myeloid leukaemia according to claim 1.

A method of diagnosis comprising:

i) contacting an isolated sample containing a blood cell population with one or more labelled antibodies that bind to

a) CD34;
b) CD45RA; and
c) CD90 and/ or CD123;

ii) detecting the presence or absence of said one or more labelled antibodies bound to a blood cell; and
iii) confirming the presence of a blood cell having a cell surface phenotype comprising:

a) $CD34^+$;
b) $CD45RA^+$; and
c) $CD90^-$ and/ or $CD123^+$ is described herein.

All descriptions relating to the diagnostic screen apply equally to the method of diagnosis aspect. The latter aspect further comprises the cell surface polypeptide marker $CD38^-$.
A screen (as defined above) for use in a method of prognosis of acute myeloid leukaemia is described herein.
In a related aspect, the invention provides a method for determining prognosis of acute myeloid leukaemia according to claim 2.
A method of prognosis comprising:

i) contacting an isolated sample containing a blood cell population with one or more labelled antibodies that bind to:

a) CD34;
b) CD45RA; and
c) CD90 and/ or CD123;

ii) detecting the presence or absence of said one or more labelled antibodies bound to a blood cell; and
iii) confirming the presence of a blood cell having a cell surface phenotype comprising:

a) $CD34^+$;
b) $CD45RA^+$; and
c) $CD90^-$ and/ or $CD123^+$ is described herein.

All descriptions relating to the diagnostic screen apply equally to the method of prognosis aspect. The latter aspect further comprises the cell surface polypeptide marker $CD38^-$.
A screen (as defined above) for use in a method of identifying a therapeutic candidate for the treatment of acute myeloid leukaemia is described herein.
In a related aspect, the invention provides a method of identifying a therapeutic candidate for the treatment of acute myeloid leukaemia according to claim 3.
A method of identifying a therapeutic candidate for the treatment of acute myeloid leukaemia comprising:

i) contacting the therapeutic candidate with an isolated sample containing a population of blood cells, wherein said blood cell has a cell surface phenotype comprising:

a) $CD34^+$;
b) $CD45RA^+$; and
c) $CD90^-$ and/ or $CD123^+$;

ii) incubating said therapeutic candidate with said isolated sample;
iii) contacting said isolated sample after step ii) with one or more labelled antibodies that bind to:

a) CD34;
b) CD45RA; and
c) CD90 and/ or CD123;

iv) identifying blood cells by step iii) that have a cell surface phenotype comprising:

a) $CD34^+$;

b) $CD45RA^+$; and
c) $CD90^-$ and/ or $CD123^+$;

v) correlating the number of blood cells identified by step iv) with the number of blood cells present in an isolated sample prior to step i) that have a cell surface phenotype comprising:

a) $CD34^+$;
b) $CD45RA^+$; and
c) $CD90^-$ and/ or $CD123^+$;

vi) confirming the presence of a therapeutic candidate having anti-acute myeloid leukaemia cell activity by identifying a relative decrease in the number of blood cells in step v) after contact with the therapeutic candidate; or confirming the absence of a therapeutic candidate having anti-acute myeloid leukaemia cell activity by identifying no significant relative decrease in the number of blood cells in step v) after contact with the therapeutic candidate is described herein.

All descriptions relating to the diagnostic screen apply equally to the method of identifying a therapeutic candidate aspect. The latter aspect further comprises the cell surface polypeptide marker $CD38^-$.

A screen (as defined above) for use in a method of monitoring efficacy of a therapeutic molecule in treating acute myeloid leukaemia is described herein.

In a related aspect, the invention provides a method for monitoring efficacy of a therapeutic molecule in treating acute myeloid leukaemia according to claim 4.

A method for monitoring efficacy of a therapeutic molecule in treating acute myeloid leukaemia, said method comprising:

i) contacting an isolated sample from a patient, wherein said patient has been administered the therapeutic molecule, with a screen that comprises one or more labelled antibodies that bind to:

a) CD34;
b) CD45RA; and
c) CD90 and/ or CD123;

ii) identifying blood cells by step i) that have a cell surface phenotype comprising:

a) $CD34^+$;
b) $CD45RA^+$; and
c) $CD90^-$ and/ or $CD123^+$;

iii) correlating the number of blood cells identified by step ii) with the number of blood cells present in an isolated sample taken from a patient prior to administration of the therapeutic molecule, wherein said blood cells taken prior to administration of the therapeutic molecule have a cell surface phenotype comprising:

a) $CD34^+$;
b) $CD45RA^+$; and
c) $CD90^-$ and/ or $CD123^+$;

iv) confirming efficacy of the therapeutic molecule by identifying a relative decrease in the number of blood cells in step iii) after contact with the therapeutic molecule; or
confirming the absence of efficacy of the therapeutic molecule by identifying a no significant relative decrease in the number of blood cells in step iii) after contact with the therapeutic molecule is described herein.

All descriptions relating to the diagnostic screen apply equally to the method for monitoring efficacy of a therapeutic molecule in treating acute myeloid leukaemia aspect. The latter aspect further comprises the cell surface polypeptide marker $CD38^-$.

A kit for diagnosis and/ or prognosis of acute myeloid leukaemia, said kit comprising at least one antibody that binds to a cell surface polypeptide marker selected from:

i) CD34;

ii) CD45RA; and
iii) CD90 and/ or CD123 is described herein.

A kit comprising a first antibody that binds to CD34, a second antibody that binds to CD45, and a third antibody that binds to CD90 and/ or CD123 is described herein. Each of said antibodies may be different. Each of said antibodies may not substantially bind to any other marker described herein - for example: the first antibody does not substantially bind to any of CD45RA, CD90, or CD123; the second antibody does not substantially bind to any of CD34, CD90, or CD123; and the third antibody substantially binds only to one of CD90 or CD123, wherein the third antibody does not substantially bind to either of CD34 or CD45RA. The third antibody may be present that binds to CD90 and not substantially to any of CD34, CD45RA or CD123. A fourth antibody may be present that binds to CD123 and not substantially to any of CD34, CD45RA, or CD90.

The kit may further comprise instructions explaining how to use the antibodies thereof in a diagnostic/ prognostic method described herein.

A kit may optionally comprise suitable labels as described above (e.g. a fluorophore label) in addition to the one or more antibodies. The kit may optionally contain an instruction manual instructing the user to perform the methods described herein.

## Definitions

The term 'diagnosis' may be used to mean determining the incidence of AML by examining whether one or more of the cell surface polypeptide markers of the diagnostic screen is present. Diagnosis of AML may embrace diagnosis of minimal residual disease (MRD). Accordingly, reference herein to acute myeloid leukaemia (AML) may embrace MRD.

A sample may be obtained from a mammal, such as a human. A suitable sample is a bone marrow or blood sample. The white blood cell population of the sample is preferably extracted or enriched prior to detection of the marker-set with antibodies of the present invention. Methods suitable for extraction of enrichment of the white blood cells from a sample are conventional techniques known to those skilled in the art. By way of example, one approach is to deplete a sample of of red cells by red cell lysis. Another approach is to isolate a mononuclear by density centrifugation using a density media like Ficoll. CD34+ cells can be then be purified from mononuclear cells by incubation with magnetic beads coated with CD34 antibody and separating CD34+ cells using a magnet.

The methods referred to herein are performed *in vitro.* In one embodiment, the methods referred to herein are performed ex *vivo.*

The term "antibody" is used in the broadest sense and specifically covers monoclonal and polyclonal antibodies (and fragments thereof) so long as they exhibit the desired biological activity. In particular, an antibody is a protein including at least one or two, heavy (H) chain variable regions (abbreviated herein as VHC), and at least one or two light (L) chain variable regions (abbreviated herein as VLC). The VHC and VLC regions can be further subdivided into regions of hypervariability, termed "complementarity determining regions" ("CDR"), interspersed with regions that are more conserved, termed "framework regions" (FR). The extent of the framework region and CDRs has been precisely defined (see, Kabat, E.A., et al. Sequences of Proteins of Immunological Interest, Fifth Edition, U.S. Department of Health and Human Services, NIH Publication No. 91-3242, 1991, and Chothia, C. et al, J. Mol. Biol. 196:901-917, 1987). Preferably, each VHC and VLC is composed of three CDRs and four FRs, arranged from amino-terminus to carboxy-terminus in the following order: FRI, CDRI, FR2, DR2, FR3, CDR3, FR4. The VHC or VLC chain of the antibody can further include all or part of a heavy or light chain constant region. In one embodiment, the antibody is a tetramer of two heavy immunoglobulin chains and two light immunoglobulin chains, wherein the heavy and light immunoglobulin chains are interconnected by, e.g., disulfide bonds. The heavy chain constant region includes three domains, CHI, CH2 and CH3. The light chain constant region is comprised of one domain, CL. The variable region of the heavy and light chains contains a binding domain that interacts with an antigen. The term "antibody" includes intact immunoglobulins of types IgA, IgG, IgE, IgD, IgM (as well as subtypes thereof), wherein the light chains of the immunoglobulin may be of types kappa or lambda. The term antibody, as used herein, also refers to a portion of an antibody that binds to one of the above-mentioned markers, e.g., a molecule in which one or more immunoglobulin chains is not full length, but which binds to a marker. Examples of binding portions encompassed within the term antibody include (i) a Fab fragment, a monovalent fragment consisting of the VLC, VHC, CL and CHI domains; (ii) a F(ab')2 fragment, a bivalent fragment comprising two Fab fragments linked by a disulfide bridge at the hinge region; (iii) a Fc fragment consisting of the VHC and CHI domains; (iv) a Fv fragment consisting of the VLC and VHC domains of a single arm of an antibody, (v) a dAb fragment (Ward et al, Nature 341:544-546, 1989), which consists of a VHC domain; and (vi) an isolated complementarity determining region (CDR) having sufficient framework to bind, e.g. an antigen binding portion of a variable region. An antigen binding portion of a light chain variable region and an antigen binding portion of a heavy chain variable region, e.g., the two domains of the Fv fragment, VLC and VHC, can be joined, using recombinant methods, by a synthetic linker that enables them to be made as a single protein chain in which the VLC and VHC regions pair to form monovalent molecules (known as single chain Fv (scFv); see e.g., Bird et al. (1988) Science IAl-ATi-Alβ; and Huston et al. (1988) Proc. Natl. Acad. ScL USA 85:5879-5883). Such single chain antibodies are also encompassed within the term antibody. These may be obtained using conventional techniques known to those skilled in the art, and the portions are screened for utility in the same manner as are intact antibodies.

**Antibody preparation**

The antibodies described herein can be obtained using conventional techniques known to persons skilled in the art and their utility confirmed by conventional binding studies. By way of example, a simple binding assay is to incubate the cell expressing an antigen with the antibody. If the antibody is tagged with a fluorophore, the binding of the antibody to the antigen can be detected by FACS analysis.

Antibodies described herein can be raised in various animals including mice, rats, rabbits, goats, sheep, monkeys or horses. Blood isolated from these animals contains polyclonal antibodies - multiple antibodies that bind to the same antigen. Antigens may also be injected into chickens for generation of polyclonal antibodies in egg yolk. To obtain a monoclonal antibody that is specific for a single epitope of an antigen, antibody-secreting lymphocytes are isolated from an animal and immortalized by fusing them with a cancer cell line. The fused cells are called hybridomas, and will continually grow and secrete antibody in culture. Single hybridoma cells are isolated by dilution cloning to generate cell clones that all produce the same antibody; these antibodies are called monoclonal antibodies. Methods for producing monoclonal antibodies are conventional techniques known to those skilled in the art (see e.g. Making and Using Antibodies: A Practical Handbook. GC Howard. CRC Books. 2006. ISBN 0849335280). Polyclonal and monoclonal antibodies are often purified using Protein A/G or antigen-affinity chromatography.

**Sequence homology:**

Any of a variety of sequence alignment methods can be used to determine percent identity, including, without limitation, global methods, local methods and hybrid methods, such as, e.g., segment approach methods. Protocols to determine percent identity are routine procedures within the scope of one skilled in the art. Global methods align sequences from the beginning to the end of the molecule and determine the best alignment by adding up scores of individual residue pairs and by imposing gap penalties. Non-limiting methods include, e.g., CLUSTAL W, see, e.g., Julie D. Thompson et al., CLUSTAL W: Improving the Sensitivity of Progressive Multiple Sequence Alignment Through Sequence Weighting, Position- Specific Gap Penalties and Weight Matrix Choice, 22(22) Nucleic Acids Research 4673-4680 (1994); and iterative refinement, see, e.g., Osamu Gotoh, Significant Improvement in Accuracy of Multiple Protein. Sequence Alignments by Iterative Refinement as Assessed by Reference to Structural Alignments, 264(4) J. Mol. Biol. 823-838 (1996). Local methods align sequences by identifying one or more conserved motifs shared by all of the input sequences. Non-limiting methods include, e.g., Match-box, see, e.g., Eric Depiereux and Ernest Feytmans, Match-Box: A Fundamentally New Algorithm for the Simultaneous Alignment of Several Protein Sequences, 8(5) CABIOS 501 - 509 (1992); Gibbs sampling, see, e.g., C. E. Lawrence et al., Detecting Subtle Sequence Signals: A Gibbs Sampling Strategy for Multiple Alignment, 262(5131) Science 208-214 (1993); Align-M, see, e.g., Ivo Van Walle et al., Align-M - A New Algorithm for Multiple Alignment of Highly Divergent Sequences, 20(9) Bioinformatics: 1428-1435 (2004). Thus, percent sequence identity is determined by conventional methods. See, for example, Altschul et al., Bull. Math. Bio. 48: 603-16, 1986 and Henikoff and Henikoff, Proc. Natl. Acad. Sci. USA 89:10915-19, 1992. Briefly, two amino acid sequences are aligned to optimize the alignment scores using a gap opening penalty of 10, a gap extension penalty of 1, and the "blosum 62" scoring matrix of Henikoff and Henikoff (ibid.) as shown below (amino acids are indicated by the standard one-letter codes).

**Alignment scores for determining sequence identity**

| | A | R | N | D | C | Q | E | G | H | I | L | K | M | F | P | S | T | W | Y | V |
|---|---|---|---|---|---|---|---|---|---|---|---|---|---|---|---|---|---|---|---|---|
| A | 4 | | | | | | | | | | | | | | | | | | | |
| R | -1 | 5 | | | | | | | | | | | | | | | | | | |
| N | -2 | 0 | 6 | | | | | | | | | | | | | | | | | |
| D | -2 | -2 | 1 | 6 | | | | | | | | | | | | | | | | |
| C | 0 | -3 | -3 | -3 | 9 | | | | | | | | | | | | | | | |
| Q | -1 | 1 | 0 | 0 | -3 | 5 | | | | | | | | | | | | | | |
| E | -1 | 0 | 0 | 2 | -4 | 2 | 5 | | | | | | | | | | | | | |
| G | 0 | -2 | 0 | -1 | -3 | -2 | -2 | 6 | | | | | | | | | | | | |
| H | -2 | 0 | 1 | -1 | -3 | 0 | 0 | -2 | 8 | | | | | | | | | | | |
| I | -1 | -3 | -3 | -3 | -1 | -3 | -3 | -4 | -3 | 4 | | | | | | | | | | |
| L | -1 | -2 | -3 | -4 | -1 | -2 | -3 | -4 | -3 | 2 | 4 | | | | | | | | | |
| K | -1 | 2 | 0 | -1 | -3 | 1 | 1 | -2 | -1 | -3 | -2 | 5 | | | | | | | | |
| M | -1 | -1 | -2 | -3 | -1 | 0 | -2 | -3 | -2 | 1 | 2 | -1 | 5 | | | | | | | |
| F | -2 | -3 | -3 | -3 | -2 | -3 | -3 | -3 | -1 | 0 | 0 | -3 | 0 | 6 | | | | | | |
| P | -1 | -2 | -2 | -1 | -3 | -1 | -1 | -2 | -2 | -3 | -3 | -1 | -2 | -4 | 7 | | | | | |
| S | 1 | -1 | 1 | 0 | -1 | 0 | 0 | 0 | -1 | -2 | -2 | 0 | -1 | -2 | -1 | 4 | | | | |
| T | 0 | -1 | 0 | -1 | -1 | -1 | -1 | -2 | -2 | -1 | -1 | -1 | -1 | -2 | -1 | 1 | 5 | | | |
| W | -3 | -3 | -4 | -4 | -2 | -2 | -3 | -2 | -2 | -3 | -2 | -3 | -1 | 1 | -4 | -3 | -2 | 11 | | |
| Y | -2 | -2 | -2 | -3 | -2 | -1 | -2 | -3 | 2 | -1 | -1 | -2 | -1 | 3 | -3 | -2 | -2 | 2 | 7 | |
| V | 0 | -3 | -3 | -3 | -1 | -2 | -2 | -3 | -3 | 3 | 1 | -2 | 1 | -1 | -2 | -2 | 0 | -3 | -1 | 4 |

The percent identity is then calculated as:

$$\frac{\text{Total number of identical matches}}{\text{[length of the longer sequence plus the number of gaps introduced into the longer sequence in order to align the two sequences]}} \times 100$$

The present invention will now be described, by way of example only, with reference to the accompanying Examples and Figures, in which:

**Figure** 1 shows the gating strategy for immunophenotyping and FACS-sorting. stem/progenitor compartments. Normal CD34+ haemopoietic progenitors were analyzed for expression of lineage markers, CD34, CD38, CD45RA, CD90, and CD123. HSCs: Lin- CD34+ CD38- CD90+ CD45RA+: MPPs: Lin- CD34+ CD38-CD90- CD45RA; CD38-CD45RA+: Lin- CD34+ CD38- CD90- CD45RA+; CMPs: Lin- CD34+ CD38+ CD123low/+ CD45RA-, GMPs: Lin-CD34+ CD38+ CD123+ CD45RA+ and MEPs: Lin- CD34+ CD38+ CD123-/low CD45RA-.

**Figure 2** shows the reanalysis of FACS-sorted stem/progenitor cells. Purity of the haematopoietic progenitor populations was analyzed on a FACS ARIA II or MFLO XDP after sort. Cells were sorted to at least 99% purity.

**Figure 3** shows the immunophenotype of stem/progenitor populations in human CD34+ AML. Representative immunophenotypes of CD34+ populations in AML and normal control samples.

(A) CD90 and CD45RA expression in CD34+CD38- cells (top) from control sample (left), an AML sample where the CD45RA+ population is expanded in CD34+CD38- and CD34+CD38+-compartments (centre -45RA+-like expanded) and an AML sample where the CD45RA- population is expanded in the CD34+CD38- and CD34+CD38+ compartments (right - MPP-like expanded).
(B) Different patterns of CD38 expression in CD34+ cells in the MPP-like expanded AML group (top) and 45RA+-like expanded AML group (bottom) with a normal control shown on the top left. The % values are the average for all samples within the group.
(C) Different patterns of CD90 and CD45RA expression in CD34+CD38- cells in MPP-like expanded AML group (top) and 45RA+-like expanded AML group (bottom).

In A-C, the % values are the average for all samples within the group.

**Figure 4** shows LSC activity in CD45RA+-like expanded AML

(A) % marrow blood engraftment of hCD45RA+ cells of individual NOD-SCID mice 12 weeks after transplantation with 6 different AML patient samples. CD38-CD45RA+ cells (squares) and GMP-like populations (ovals) from each patient were injected into 4 mice.
(B) FISH analysis of engrafted populations from patient OX208 (left) and OX268 (right). In both cases, the cell on the left is from a mouse engrafted with CD38-CD45RA+ cells and the cell on the right engrafted with GMP-like AML population. In patient OX 208, chromosome 14 is detected by a dual colour IGH locus probe and in patient OX268, chromosomes 8 (red signal) and 12 (green) are detected by centromere probes. Cell nuclei are visualized by DAPI counterstain.
(C) A representative example of hCD45RA and hCD34 expression (left, (i) and (v)) and hCD19 and hCD33 expression in hCD34+(centre, (ii) and (v)) and CD34-(right, (iii) and (vi)) populations from primary engrafted mice when transplanted with CD38-CD45RA+ (top) and GMP-like (bottom) AML populations. The mean % values from all 6 patients are shown.
(D) A representative example of hCD38 and hCD34 expression on engrafted hCD34+ cells (left, (i) and (iv)), hCD90 and hCD45RA expression in hCD34+CD38- cells (centre, (ii)) when CD38-CD45RA+ cells (top) and GMP-like AML (bottom) cells are injected into primary engrafted mice. The mean % values from all 6 patients are shown.
(E) % engraftment of hCD45RA+ cells in marrow of individual in secondary transplanted mice 12 weeks after intravenous injection with pooled hCD45+ cells from primary engrafted mice initially injected with either CD38-CD45RA+ (squares) and GMP-like (oval) cells. 4 secondary recipient mice were injected per population. Pooled cells from each primary leukaemia are shown as a different symbol.

**Figure 5** shows immunophenotypic analysis of engrafted human cells in secondary xenotransplanted animals.

(A) % marrow blood engraftment of hCD45RA+ cells of individual NOD-SCID mice 12 weeks after transplantation with 4 different AML patient samples.
(B) A representative example of human (h) CD38 and CD34 expression of engrafted hCD34+ cells (left), hCD90 and hCD45RA expression in hCD34+CD38- cells (centre) and hCD110 and hCD45RA expression in hCD34+CD38+ cells when total CD45+ cells are injected into secondary recipient mice after primary engraftment of CD34+ CD38- CD45RA+ (top) or CD34+ CD38+ CD110- CD45RA+ (bottom) cells. The % values are the mean from all patients.
(C) A representative example of hCD45RA and hCD34 expression (left) and hCD19 and hCD33 expression in hCD34+(centre) and CD34- (right) populations when total CD45+ cells are injected into secondary recipient mice after primary engraftment of CD34+ CD38- CD45RA+ (top) or CD34+ CD38+ CD110-CD45RA+ (bottom) cells. The % values are the mean from all patients.
(D) % marrow blood engraftment of hCD45RA+ cells of individual NOD-SCID mice 12 weeks after transplantation with 4 different AML patient samples.

**Figure 6** shows in vitro hierarchy in CD45RA+ like expanded AML.

(A) Schematic experimental outline. FACS-sorted CD38-CD45RA+ or GMP-like populations from 5 AML samples were separately cultured for either 4, or 8 days, and then analyzed by FACS.
(B) (i) Top left, representative example of CD34 and CD38 expression in an AML sample. The purity of CD38-CD45RA+ (right) populations post-FACS sort and prior to culture is shown.

Below, representative FACS analysis of cell populations when CD38-CD45RA+ and GMP-like populations have been cultured for either 4 days (ii - left) or 8 days (iii - right). Top panel, CD34 and CD38 expression; bottom panel, CD90 and CD45RA expression in CD34+CD38- cells;. The percentages shown are the mean for gated populations for all 5 AML samples studied.

**Figure 7** shows gene expression of AML LSC populations and normal stem and progenitor populations.

(A) 3-D Principle Component Analysis (PCA) displaying gene expression profiles using 917 differentially expressed probes (corresponding to 748 mapped genes) determined by a paired t-test with a cut-off of 0.01 from 18 AML patients where both CD38-CD45RA+ (blue spheres) and GMP-like populations (red spheres) were available from the same patient.
(B) Rank product analysis of probe sets showing either increased expression in CD38-CD45RA+ compared to GMP-like populations (i) and vice versa (ii) using gene expression profiles from 18 AML patients where both CD38-CD45RA+ and GMP-like population samples were available from the same patient. On the y-axis, the false discovery rate; on the x-axis the rank product of the probes. At a false discovery rate of 0.05, the numbers of differentially expressed probes are shown as red lines.
(C) 3-D Principal Component Analysis of expression profiles of FACS-sorted 4 normal populations (HSC - black spheres, MPP - brown spheres, CD34+CD38-CD90-CD45RA+ - yellow spheres, CMP - pink spheres and GMP - green spheres) using a 2629 ANOVA gene set (2789 probes) of differentially expressed genes in the normal populations. The positions of 22 CD38-CD45RA+ AML populations (blue spheres) (left) and 21 GMP-like AML populations (red spheres) (right) are shown.
(D-G) Classifier analysis using the 2789 ANOVA selected differentially expressed set of probes that separates normal populations. This probe set was used to call the identity of the 22 CD38-CD45RA+ AML populations (D-E) and 21 GMP-like AML populations (F-G). On the x-axis more probes (from the most differentially expressed to the least differentially expressed) are used in the classifier from right to left. The top x-axis shows the numbers of probes used. The bottom x-axis depicts the corresponding threshold values. Y-axis, the number of AML samples called.

**Figure 8** shows the analysis of stem/progenitor expression profiles in AML LSC and normal stem/progenitor cells.

(A) Non-paired t-test was used to identify a gene set differentially expressed between 22 CD38-CD45RA+ and 21 GMP-like AML populations. This gene set was used to display gene expression in a 3-D PCA analysis. Gene expression in each CD38-CD45RA+ AML sample is shown blue sphere and that in each GMP-like AML sample as a red sphere.
(B) Hierarchical clustering analysis of gene expression of 22 CD38-CD45RA+ and 21 GMP-like AMP populations using the set of differentially expressed genes identified by t-test analysis.
(C and D) Hierarchical clustering analysis to show the relationship between normal HSC/term populations and CD38-CD45RA+ (C) and GMP-like AML populations using an Anova gene set of 2789 genes that maximally differentiates normal HSC/progenitor populations by gene expression.

**Figure 9** shows that normal CD38-CD45RA+ cells have lymphoid primed multipotential myeloid potential.

(A) Myeloid/erythroid colony growth of FACS sorted normal marrow HSC, MPP, CMP, GMP, MEP and CD38-CD45RA+ populations from 5 normal samples. Number of colonies/500 cells plated were scored (mean$\pm$1 S.D.). Colony lineage affiliation is shown by different colored bars (right of panel).
(B) Cells from colonies in the primary platings (A) were plated in a secondary replating assay. The number of colonies/2500 cells plated from each of the cell type is illustrated (mean$\pm$1 S.D.).
(C) Megakaryocyte colony growth from FACS sorted normal marrow HSC, MPP, CMP, GMP, MEP and CD38-CD45RA+ populations from 3 normal individuals. The number of colonies/1000 cells plated is depicted. Colony lineage affiliation is shown by different colored bars (right of panel).
(D and E) Limit dilution analysis to determine frequency of cells with myeloid potential (D) and mixed B-cell/myeloid potential (E) in CD38-CD45RA+ (CD45RA+) (green line) and GMP cells (blue line). 1-500 sorted cells from 4 normal marrow samples were tested in individual wells in MS5 stroma/cytokine co-culture (144 replicates for each condition). Cell output was analyzed by FACS.

(F) Representative FACS analysis of CD19 and CD33 expression in cells produced from bulk culture of FACS sorted HSC, MPP, CD38-CD45RA+, CMP and GMP cells from 4 normal marrows.

(G) Limit dilution analysis to determine T-cell potential frequency in CD38-CD45RA+ (green line) and GMP cells (blue line). 1-500 sorted cells from 3 marrow samples were tested in individual wells in OP-DL1 stroma/cytokine co-culture (36 replicates for each condition). Cell output was analyzed by FACS.

(H) Representative FACS analysis plots of CD1a, CD7 (top) and CD33 and CD3 expression in cells produced from bulk culture of FACS sorted CD38-CD45RA+ and GMP cells from 3 marrows.

**Figure 10** demonstrates granulocyte-monocyte and lymphoid but not erythroid-megakaryocyte gene expression in CD34+CD38-CD90-CD45RA+ cells.

(A) Schematic diagram of the cellular hierarchy of human stem/progenitor cells and the cascade of lineage-affiliated gene signatures. HSC, haematopoietic stem cells; MPP, multi-potential cells; CD38-CD45RA+, Lin-CD34+CD38-CD90-CD45RA+ cells; MEP, megakaryocytic-erythroid progenitor; GMP, granulocyte-macrophage progenitor. HSC- and CD38-CD45RA+-affiliated gene signature (green); myeloid (GM) lineage-affiliated gene signature (blue); lymphoid lineage-affiliated gene signature (yellow); ME lineage-affiliated gene signature (red).

(B) Mean mRNA expression (±1 SD) of indicated genes relative to *GAPDH* was determined in 5 replicates by Quantitative Real-Time RT-PCR in 10 FACS-sorted HSC, CD38-CD45RA+, GMP and MEP cells from 2 independent control normal samples. Data from genes affiliated with: (i) HSC and CD38-CD45RA+ cells (green bars); (ii) granulocyte-monocyte lineage cells (blue bars); (iii) lymphoid lineage cells (yellow bars); (iv) erythroid-megakaryocyte lineage cells (red bars).

**Figure 11** shows gene expression data in highly purified normal stem/progenitor cells.
Multiplex quantitative PCR data of indicated genes on FACS sorted HSC, CD38-CD45RA+, GMP and MEP cells. All data were normalized to the expression of GAPDH. Results represent the mean value from 5 replicates from 2 control samples. Data from genes affiliated with: (i) HSC and CD38-CD45RA+ cells (green bars); (ii) granulocyte-monocyte lineage cells (blue bars); (iii) lymphoid lineage cells (yellow bars); (iv) erythroid-megakaryocyte lineage cells (red bars).

**Figure 12** shows the results of demographic and immunophenotypic characteristics associated with samples used in xenotransplantation experiments, related to Figure 4

**Figure 13** shows the results of cytogenetic analysis of FACS-sorted engrafted human cells in primary xenotransplant animals, related to Figure 4.

**KEY TO SEQ ID NOs:**

**SEQ ID NO: 1 CD34 amino acid sequence**
**SEQ ID NO: 2 CD45RA amino acid sequence**
**SEQ ID NO: 3 CD90 amino acid sequence**
**SEQ ID NO: 4 CD123 amino acid sequence**
**SEQ ID NO: 5 CD38 amino acid sequence**
**SEQ ID NO: 6 CD19 amino acid sequence**
**SEQ ID NO: 7 CD47 amino acid sequence**
**SEQ ID NO: 8 CCR8 amino acid sequence**
**SEQ ID NO: 9 RHAMM amino acid sequence**
**SEQ ID NO: 10 CD86 amino acid sequence**
**SEQ ID NO: 11 CD34 nucleic acid sequence**
**SEQ ID NO: 12 CD45RA nucleic acid sequence**
**SEQ ID NO: 13 CD90 nucleic acid sequence**
**SEQ ID NO: 14 CD123 nucleic acid sequence**
**SEQ ID NO: 15 CD38 nucleic acid sequence**
**SEQ ID NO: 16 CD19 nucleic acid sequence**
**SEQ ID NO: 17 CD47 nucleic acid sequence**

**SEQ ID NO: 18 CCR8 nucleic acid sequence**
**SEQ ID NO: 19 RHAMM nucleic acid sequence**
**SEQ ID NO: 20 CD86 nucleic acid sequence**

## SEQUENCE LISTING:

**SEQ ID NO: 1**

```
mlvrrgarag prmprgwtal cllsllpsgf msldnngtat pelptqgtfs nvstnvsyqe
tttpstlgst slhpvsqhgn eattnitett vkftstsvit svygntnssv qsqtsvistv
fttpanvstp ettlkpslsp gnvsdlstts tslatsptkp ytssspilsd ikaeikcsgi
revkltqgic leqnktssca efkkdrgegl arvlcgeeqa dadagaqvcs lllaqsevrp
qclllvlanr teissklqlm kkhqsdlkkl gildfteqdv ashqsysqkt lialvtsgal
lavlgitgyf lmnrrswspt gerlelep
```

**SEQ ID NO: 2**

```
"MYLWLKLLAFGFAFLDTEVFVTGQSPTPSPTDAYLNASETTTLS
PSGSAVISTTTIATTPSKPTCDEKYANITVDYLYNKETKLFTAKLNVNENVECGNNTC
TNNEVHNLTECKNASVSISHNSCTAPDKTLILDVPPGVEKFQLHDCTQVEKADTTICL
KWKNIETFTCDTQNITYRFQCGNMIFDNKEIKLENLEPEHEYKCDSEILYNNHKFTNA
SKIIKTDFGSPGEPQIIFCRSEAAHQGVITWNPPQRSFHNFTLCYIKETEKDCLNLDK
NLIKYDLQNLKPYTKYVLSLHAYIIAKVQRNGSAAMCHFTTKSAPPSQVWNMTVSMTS
DNSMHVKCRPPRDRNGPHERYHLEVEAGNTLVRNESHKNCDFRVKDLQYSTDYTFKAY
FHNGDYPGEPFILHHSTSYNSKALIAFLAFLIIVTSIALLVVLYKIYDLHKKRSCNLD
EQQELVERDDEKQLMNVEPIHADILLETYKRKIADEGRLFLAEFQSIPRVFSKFPIKE
ARKPFNQNKNRYVDILPYDYNRVELSEINGDAGSNYINASYIDGFKEPRKYIAAQGPR
DETVDDFWRMIWEQKATVIVMVTRCEEGNRNKCAEYWPSMEEGTRAFGDVVVKINQHK
RCPDYIIQKLNIVNKKEKATGREVTHIQFTSWPDHGVPEDPHLLLKLRRRVNAFSNFF
SGPIVVHCSAGVGRTGTYIGIDAMLEGLEAENKVDVYGYVVKLRRQRCLMVQVEAQYI
LIHQALVEYNQFGETEVNLSELHPYLHNMKKRDPPSEPSPLEAEFQRLPSYRSWRTQH
IGNQEENKSKNRNSNVIPYDYNRVPLKHELEMSKESEHDSDESSDDDSDSEEPSKYIN
ASFIMSYWKPEVMIAAQGPLKETIGDFWQMIFQRKVKVIVMLTELKHGDQEICAQYWG
EGKQTYGDIEVDLKDTDKSSTYTLRVFELRHSKRKDSRTVYQYQYTNWSVEQLPAEPK
ELISMIQVVKQKLPQKNSSEGNKHHKSTPLLIHCRDGSQQTGIFCALLNLLESAETEE
VVDIFQVVKALRKARPGMVSTFEQYQFLYDVIASTYPAQNGQVKKNNHQEDKIEFDNE
VDKVKQDANCVNPLGAPEKLPEAKEQAEGSEPTSGTEGPEHSVNGPASPALNQGS"
```

**SEQ ID NO: 3**

```
MNLAISIALLLTVLQVSRGQKVTSLTACLVDQSLRLDCRHENTS
SSPIQYEFSLTRETKKHVLFGTVGVPEHTYRSRTNFTSKYNMKVLYLSAFTSKDEGTY
TCALHHSGHSPPISSQNVTVLRDKLVKCEGISLLAQNTSWLLLLLLSLSLLQATDFMS
L
```

**SEQ ID NO: 4**

```
MVLLWLTLLLIALPCLLQTKEDPNPPITNLRMKAKAQQLTWDLN
RNVTDIECVKDADYSMPAVNNSYCQFGAISLCEVTNYTVRVANPPFSTWILFPENSGK
PWAGAENLTCWIHDVDFLSCSWAVGPGAPADVQYDLYLNVANRRQQYECLHYKTDAQG
TRIGCRFDDISRLSSGSQSSHILVRGRSAAFGIPCTDKFVVFSQIEILTPPNMTAKCN
KTHSFMHWKMRSHFNRKFRYELQIQKRMQPVITEQVRDRTSFQLLNPGTYTVQIRARE
RVYEFLSAWSTPQRFECDQEEGANTRAWRTSLLIALGTLLALVCVFVICRRYLVMQRL
FPRIPHMKDPIGDSFQNDKLVVWEAGKAGLEECLVTEVQVVQKT
```

**SEQ ID NO: 5**

MANCEFSPVSGDKPCCRLSRRAQLCLGVSILVLILVVVLAVVVP
RWRQQWSGPGTTKRFPETVLARCVKYTEIHPEMRHVDCQSVWDAFKGAFISKHPCNIT
EEDYQPLMKLGTQTVPCNKILLWSRIKDLAHQFTQVQRDMFTLEDTLLGYLADDLTWC
GEFNTSKINYQSCPDWRKDCSNNPVSVFWKTVSRRFAEAACDVVHVMLNGSRSKIFDK
NSTFGSVEVHNLQPEKVQTLEAWVIHGGREDSRDLCQDPTIKELESIISKRNIQFSCK
NIYRPDKFLQCVKNPEDSSCTSEI

**SEQ ID NO: 6**

MPPPRLLFFLLFLTPMEVRPEEPLVVKVEEGDNAVLQCLKGTSD
GPTQQLTWSRESPLKPFLKLSLGLPGLGIHMRPLAIWLFIFNVSQQMGGFYLCQPGPP
SEKAWQPGWTVNVEGSGELFRWNVSDLGGLGCGLKNRSSEGPSSPSGKLMSPKLYVWA
KDRPEIWEGEPPCLPPRDSLNQSLSQDLTMAPGSTLWLSCGVPPDSVSRGPLSWTHVH
PKGPKSLLSLELKDDRPARDMWVMETGLLLPRATAQDAGKYYCHRGNLTMSFHLEITA
RPVLWHWLLRTGGWKVSAVTLAYLIFCLCSLVGILHLQRALVLRRKRKRMTDPTRRFF
KVTPPPGSGPQNQYGNVLSLPTPTSGLGRAQRWAAGLGGTAPSYGNPSSDVQADGALG
SRSPPGVGPEEEEGEGYEEPDSEEDSEFYENDSNLGQDQLSQDGSGYENPEDEPLGPE
DEDSFSNAESYENEDEELTQPVARTMDFLSPHGSAWDPSREATSLGSQSYEDMRGILY
AAPQLRSIRGQPGPNHEEDADSYENMDNPDGPDPAWGGGGRMGTWSTR

**SEQ ID NO: 7**

WPLVAALLLGSACCGSAQLLFNKTKSVEFTFCNDTVVIPCFVT
NMEAQNTTEVYVKWKFKGRDIYTFDGALNKSTVPTDFSSAKIEVSQLLKGDASLKMDK
SDAVSHTGNYTCEVTELTREGETIIELKYRVVSWFSPNENILIVIFPIFAILLFWGQF
GIKTLKYRSGGMDEKTIALLVAGLVITVIVIVGAILFVPGEYSLKNATGLGLIVTSTG
ILILLHYYVFSTAIGLTSFVIAILVIQVIAYILAVVGLSLCIAACIPMHGPLLISGLS
ILALAQLLGLVYMKFVASNQKTIQPPRKAVEEPLNAFKESKGMMNDE"

**SEQ ID NO: 8**

MDYTLDLSVTTVTDYYYPDIFSSPCDAELIQTNGKLLLAVFYCL
LFVFSLLGNSLVILVLVVCKKLRSITDVYLLNLALSDLLFVFSFPFQTYYLLDQWVFG
TVMCKVVSGFYYIGFYSSMFFITLMSVDRYLAVVHAVYALKVRTIRMGTTLCLAVWLT
AIMATIPLLVFYQVASEDGVLQCYSFYNQQTLKWKIFTNFKMNILGLLIPFTIFMFCY
IKILHQLKRCQNHNKTKAIRLVLIVVIASLLFWVPFNVVLFLTSLHSMHILDGCSISQ
QLTYATHVTEIISFTHCCVNPVIYAFVGEKFKKHLSEIFQKSCSQIFNYLGRQMPRES
CEKSSSCQQHSSRSSSVDYIL

**SEQ ID NO: 9**

MSFPKAPLKRFNDPSGCAPSPGAYDVKTLEVLKGPVSFQKSQRF
KQQKESKQNLNVDKDTTLPASARKVKSSESKKESQKNDKDLKILEKEIRVLLQERGAQ

DRRIQDLETELEKMEARLNAALREKTSLSANNATLEKQLIELTRTNELLKSKFSENGN
QKNLRILSLELMKLRNKRETKMRGMMAKQEGMEMKLQVTQRSLEESQGKIAQLEGKLV
SIEKEKIDEKSETEKLLEYIEEISCASDQVEKYKLDIAQLEENLKEKNDEILSLKQSL
EENIVILSKQVEDLNVKCQLLEKEKEDHVNRNREHNENLNAEMQNLKQKFILEQQERE
KLQQKELQIDSLLQQEKELSSSLHQKLCSFQEEMVKEKNLFEEELKQTLDELDKLQQK
EEQAERLVKQLEEEAKSRAEELKLLEEKLKGKEAELEKSSAAHTQATLLLQEKYDSMV
QSLEDVTAQFESYKALTASEIEDLKLENSSLQEKAAKAGKNAEDVQHQILATESSNQE
YVRMLLDLQTKSALKETEIKEITVSFLQKITDLQNQLKQQEEDFRKQLEDEEGRKAEK
ENTTAELTEEINKWRLLYEELYNKTKPFQLQLDAFEVEKQALLNEHGAAQEQLNKIRD
SYAKLLGHQNLKQKIKHVVKLKDENSQLKSEVSKLRCQLAKKKQSETKLQEELNKVLG
IKHFDPSKAFHHESKENFALKTPLKEGNTNCYRAPMECQESWK

**SEQ ID NO: 10**

MDPQCTMGLSNILFVMAFLLSGAAPLKIQAYFNETADLPCQFAN
SQNQSLSELVVFWQDQENLVLNEVYLGKEKFDSVHSKYMGRTSFDSDSWTLRLHNLQI
KDKGLYQCIIHHKKPTGMIRIHQMNSELSVLANFSQPEIVPISNITENVYINLTCSSI
HGYPEPKKMSVLLRTKNSTIEYDGVMQKSQDNVTELYDVSISLSVSFPDVTSNMTIFC
ILETDKTRLLSSPFSIELEDPQPPPDHIPWITAVLPTVIICVMVFCLILWKWKKKKRP
RNSYKCGTNTMEREESEQTKKREKIHIPERSDEAQRVFKSSKTSSCDKSDTCF

**SEQ ID NO: 11**

atgctggtcc gcaggggcgc gcgcgcaggg cccaggatgc cgcggggctg gaccgcgctt
tgcttgctga gtttgctgcc ttctgggttc atgagtcttg acaacaacgg tactgctacc
ccagagttac ctacccaggg aacattttca aatgtttcta caaatgtatc ctaccaagaa
actacaacac ctagtaccct tggaagtacc agcctgcacc ctgtgtctca acatggcaat
gaggccacaa caaacatcac agaaacgaca gtcaaattca catctacctc tgtgataacc
tcagtttatg gaaacacaaa ctcttctgtc cagtcacaga cctctgtaat cagcacagtg
ttcaccaccc cagccaacgt ttcaactcca gagacaacct tgaagcctag cctgtcacct
ggaaatgttt cagacctttc aaccactagc actagccttg caacatctcc cactaaaccc
tatacatcat cttctcctat cctaagtgac atcaaggcag aaatcaaatg ttcaggcatc
agagaagtga aattgactca gggcatctgc ctggagcaaa ataagacctc cagctgtgcg
gagtttaaga aggacagggg agagggcctg gcccgagtgc tgtgtgggga ggagcaggct
gatgctgatg ctggggccca ggtatgctcc ctgctccttg cccagtctga ggtgaggcct
cagtgtctac tgctggtctt ggccaacaga acagaaattt ccagcaaact ccaacttatg
aaaaagcacc aatctgacct gaaaaagctg gggatcctag atttcactga gcaagatgtt
gcaagccacc agagctattc ccaaaagacc ctgattgcac tggtcacctc gggagccctg
ctggctgtct tgggcatcac tggctatttc ctgatgaatc gccgcagctg gagccccaca
ggagaaaggc tgggcgaaga cccttattac acggaaaacg gtggaggcca gggctatagc
tcaggacctg ggacctcccc tgaggctcag ggaaaggcca gtgtgaaccg aggggctcag
gaaaacggga ccggccaggc cacctccaga aacggccatt cagcaagaca acacgtggtg
gctgataccg aattgtga

**SEQ ID NO: 12**

atgtatttgt ggcttaaact cttggcattt ggctttgcct ttctggacac agaagtattt
gtgacagggc aaagcccaac accttccccc actggattga ctacagcaaa gatgcccagt
gttccacttt caagtgaccc cttacctact cacaccactg cattctcacc cgcaagcacc
tttgaaagag aaaatgactt ctcagagacc acaacttctc ttagtccaga caatacttcc
acccaagtat ccccggactc tttggataat gctagtgctt ttaataccac aggtgtttca
tcagtacaga cgcctcacct tcccacgcac gcagactcgc agacgccctc tgctggaact
gacacgcaga cattcagcgg ctccgccgcc aatgcaaaac tcaaccctac cccaggcagc
aatgctatct cagatgtccc aggagagagg agtacagcca gcacctttcc tacagaccca
gtttccccat tgacaaccac cctcagcctt gcacaccaca gctctgctgc cttacctgca
cgcacctcca acaccaccat cacagcgaac acctcagatg cctaccttaa tgcctctgaa

```
acaaccactc tgagcccttc tggaagcgct gtcatttcaa ccacaacaat agctactact
ccatctaagc caacatgtga tgaaaaatat gcaaacatca ctgtggatta cttatataac
aaggaaacta aattatttac agcaaagcta aatgttaatg agaatgtgga atgtggaaac
aatacttgca caaacaatga ggtgcataac cttacagaat gtaaaaatgc gtctgtttcc
atatctcata attcatgtac tgctcctgat aagacattaa tattagatgt gccaccaggg
gttgaaaagt ttcagttaca tgattgtaca caagttgaaa aagcagatac tactatttgt
ttaaaatgga aaaatattga aacctttact tgtgatacac agaatattac ctacagattt
cagtgtggta atatgatatt tgataataaa gaaattaaat tagaaaacct tgaacccgaa
catgagtata agtgtgactc agaaatactc tataataacc acaagtttac taacgcaagt
aaaattatta aaacagattt tgggagtcca ggagagcctc agattatttt ttgtagaagt
gaagctgcac atcaaggagt aattacctgg aatcccctc aaagatcatt tcataatttt
accctctgtt atataaaaga gacagaaaaa gattgcctca atctggataa aaacctgatc
aaatatgatt tgcaaaattt aaaaccttat acgaaatatg ttttatcatt acatgcctac
atcattgcaa aagtgcaacg taatggaagt gctgcaatgt gtcatttcac aactaaaagt
gctcctccaa gccaggtctg gaacatgact gtctccatga catcagataa tagtatgcat
gtcaagtgta ggcctcccag ggaccgtaat ggcccccatg aacgttacca tttggaagtt
gaagctggaa atactctggt tagaaatgag tcgcataaga attgcgattt ccgtgtaaaa
gatcttcaat attcaacaga ctacactttt aaggcctatt ttcacaatgg agactatcct
ggagaaccct ttattttaca tcattcaaca tcttataatt ctaaggcact gatagcattt
ctggcatttc tgattattgt gacatcaata gccctgcttg ttgttctcta caaaatctat
gatctacata agaaaagatc ctgcaattta gatgaacagc aggagcttgt tgaaagggat
gatgaaaaac aactgatgaa tgtggagcca atccatgcag atattttgtt ggaaacttat
aagaggaaga ttgctgatga aggaagactt tttctggctg aatttcagag catcccgcgg
gtgttcagca agtttcctat aaaggaagct cgaaagccct ttaaccagaa taaaaaccgt
tatgttgaca ttcttcctta tgattataac cgtgttgaac tctctgagat aaacggagat
gcagggtcaa actacataaa tgccagctat attgatggtt tcaaagaacc caggaaatac
attgctgcac aaggtcccag ggatgaaact gttgatgatt tctggaggat gatttgggaa
cagaaagcca cagttattgt catggtcact cgatgtgaag aaggaaacag gaacaagtgt
gcagaatact ggccgtcaat ggaagagggc actcgggctt ttggagatgt tgttgtaaag
atcaaccagc acaaaagatg tccagattac atcattcaga aattgaacat tgtaaataaa
aaagaaaaag caactggaag agaggtgact cacattcagt tcaccagctg gccagaccac
ggggtgcctg aggatcctca cttgctcctc aaactgagaa ggagagtgaa tgccttcagc
aatttcttca gtggtcccat tgtggtgcac tgcagtgctg gtgttgggcg cacaggaacc
tatatcggaa ttgatgccat gctagaaggc ctggaagccg agaacaaagt ggatgtttat
ggttatgttg tcaagctaag gcgacagaga tgcctgatgg ttcaagtaga ggcccagtac
atcttgatcc atcaggcttt ggtggaatac aatcagtttg gagaaacaga agtgaatttg
tctgaattac atccatatct acataacatg aagaaaaggg atccacccag tgagccgtct
ccactagagg ctgaattcca gagacttcct tcatatagga gctggaggac acagcacatt
ggaaatcaag aagaaaataa aagtaaaaac aggaattcta atgtcatccc atatgactat
aacagagtgc cacttaaaca tgagctggaa atgagtaaag agagtgagca tgattcagat
gaatcctctg atgatgacag tgattcagag gaaccaagca aatacatcaa tgcatctttt
ataatgagct actggaaacc tgaagtgatg attgctgctc agggaccact gaaggagacc
attggtgact tttggcagat gatcttccaa agaaaagtca aagttattgt tatgctgaca
gaactgaaac atggagacca ggaaatctgt gctcagtact ggggagaagg aaagcaaaca
tatggagata ttgaagttga cctgaaagac acagacaaat cttcaactta tacccttcgt
gtctttgaac tgagacattc caagaggaaa gactctcgaa ctgtgtacca gtaccaatat
acaaactgga gtgtggagca gcttcctgca gaacccaagg aattaatctc tatgattcag
gtcgtcaaac aaaaacttcc ccagaagaat tcctctgaag ggaacaagca tcacaagagt
acacctctac tcattcactg cagggatgga tctcagcaaa cgggaatatt ttgtgctttg
ttaaatctct tagaaagtgc ggaaacagaa gaggtagtgg atatttttca agtggtaaaa
gctctacgca aagctaggcc aggcatggtt tccacattcg agcaatatca attcctatat
gacgtcattg ccagcaccta ccctgctcag aatggacaag taaagaaaaa caaccatcaa
gaagataaaa ttgaatttga taatgaagtg gacaaagtaa agcaggatgc taattgtgtt
aatccacttg gtgccccaga aaagctccct gaagcaaagg aacaggctga aggttctgaa
cccacgagtg gcactgaggg gccagaacat tctgtcaatg gtcctgcaag tccagcttta
aatcaaggtt catag
```

**SEQ ID NO: 13**

atgaacctgg ccatcagcat cgctctcctg ctaacagtct tgcaggtctc ccgagggcag
aaggtgacca gcctaacggc ctgcctagtg gaccagagcc ttcgtctgga ctgccgccat
gagaatacca gcagttcacc catccagtac gagttcagcc tgacccgtga gacaaagaag
cacgtgctct ttggcactgt gggggtgcct gagcacacat accgctcccg aaccaacttc
accagcaaat acaacatgaa ggtcctctac ttatccgcct tcactagcaa ggacgagggc
acctacacgt gtgcactcca ccactctggc cattccccac ccatctcctc ccagaacgtc
acagtgctca gagacaaact ggtcaagtgt gagggcatca gcctgctggc tcagaacacc
tcgtggctgc tgctgctcct gctctccctc tccctcctcc aggccacgga tttcatgtcc
ctgtga

**SEQ ID NO: 14**

atggtcctcc tttggctcac gctgctcctg atcgccctgc cctgtctcct gcaaacgaag
gaagatccaa acccaccaat cacgaaccta aggatgaaag caaaggctca gcagttgacc
tgggacctta acagaaatgt gaccgatatc gagtgtgtta aagacgccga ctattctatg
ccggcagtga acaatagcta ttgccagttt ggagcaattt ccttatgtga agtgaccaac
tacaccgtcc gagtggccaa cccaccattc tccacgtgga tcctcttccc tgagaacagt
gggaagcctt gggcaggtgc ggagaatctg acctgctgga ttcatgacgt ggatttcttg
agctgcagct gggcggtagg cccgggggcc cccgcggacg tccagtacga cctgtacttg
aacgttgcca acaggcgtca acagtacgag tgtcttcact acaaaacgga tgctcaggga
acacgtatcg ggtgtcgttt cgatgacatc tctcgactct ccagcggttc tcaaagttcc
cacatcctgg tgcggggcag gagcgcagcc ttcggtatcc cctgcacaga taagtttgtc
gtcttttcac agattgagat attaactcca cccaacatga ctgcaaagtg taataagaca
cattccttta tgcactggaa aatgagaagt catttcaatc gcaaatttcg ctatgagctt
cagatacaaa agagaatgca gcctgtaatc acagaacagg tcagagacag aacctccttc
cagctactca atcctggaac gtacacagta caaataagag cccgggaaag agtgtatgaa
ttcttgagcg cctggagcac cccccagcgc ttcgagtgcg accaggagga gggcgcaaac
acacgtgcct ggcggacgtc gctgctgatc gcgctgggga cgctgctggc cctggtctgt
gtcttcgtga tctgcagaag gtatctggtg atgcagagac tctttccccg catccctcac
atgaaagacc ccatcggtga cagcttccaa aacgacaagc tggtggtctg ggaggcgggc
aaagccggcc tggaggagtg tctggtgact gaagtacagg tcgtgcagaa aacttga

**SEQ ID NO: 15**

atggccaact gcgagttcag cccggtgtcc ggggacaaac cctgctgccg gctctctagg
agagcccaac tctgtcttgg cgtcagtatc ctggtcctga tcctcgtcgt ggtgctcgcg
gtggtcgtcc cgaggtggcg ccagcagtgg agcggtccgg gcaccaccaa gcgctttccc
gagaccgtcc tggcgcgatg cgtcaagtac actgaaattc atcctgagat gagacatgta
gactgccaaa gtgtatggga tgctttcaag ggtgcattta tttcaaaaca tccttgcaac
attactgaag aagactatca gccactaatg aagttgggaa ctcagaccgt accttgcaac
aagattcttc tttggagcag aataaaagat ctggcccatc agttcacaca ggtccagcgg
gacatgttca ccctggagga cacgctgcta ggctaccttg ctgatgacct cacatggtgt
ggtgaattca acacttccaa aataaactat caatcttgcc cagactggag aaaggactgc
agcaacaacc ctgtttcagt attctggaaa acggtttccc gcaggtttgc agaagctgcc
tgtgatgtgg tccatgtgat gctcaatgga tcccgcagta aaatctttga caaaaacagc
acttttggga gtgtggaagt ccataatttg caaccagaga aggttcagac actagaggcc
tgggtgatac atggtggaag agaagattcc agagacttat gccaggatcc caccataaaa
gagctggaat cgattataag caaaaggaat attcaatttt cctgcaagaa tatctacaga
cctgacaagt ttcttcagtg tgtgaaaaat cctgaggatt catcttgcac atctgagatc
tga

**SEQ ID NO: 16**

atgccacctc ctcgcctcct cttcttcctc ctcttcctca cccccatgga agtcaggccc
gaggaacctc tagtggtgaa ggtggaagag ggagataacg ctgtgctgca gtgcctcaag
gggacctcag atggccccac tcagcagctg acctggtctc gggagtcccc gcttaaaccc
ttcttaaaac tcagcctggg gctgccaggc ctgggaatcc acatgaggcc cctggccatc
tggcttttca tcttcaacgt ctctcaacag atgggggct tctacctgtg ccagccgggg
cccccctctg agaaggcctg gcagcctggc tggacagtca atgtggaggg cagcggggag
ctgttccggt ggaatgtttc ggacctaggt ggcctgggct gtggcctgaa gaacaggtcc
tcagagggcc ccagctcccc ttccgggaag ctcatgagcc ccaagctgta tgtgtgggcc
aaagaccgcc ctgagatctg ggagggagag cctccgtgtc tcccaccgag ggacagcctg
aaccagagcc tcagccagga cctcaccatg gcccctggct ccacactctg gctgtcctgt
ggggtacccc ctgactctgt gtccaggggc cccctctcct ggacccatgt gcaccccaag
gggcctaagt cattgctgag cctagagctg aaggacgatc gcccggccag agatatgtgg
gtaatggaga cgggtctgtt gttgccccgg gccacagctc aagacgctgg aaagtattat
tgtcaccgtg gcaacctgac catgtcattc cacctggaga tcactgctcg gccagtacta
tggcactggc tgctgaggac tggtggctgg aaggtctcag ctgtgacttt ggcttatctg
atcttctgcc tgtgttccct tgtgggcatt cttcatcttc aaagagccct ggtcctgagg
aggaaaagaa agcgaatgac tgaccccacc aggagattct tcaaagtgac gcctccccca
ggaagcgggc cccagaacca gtacgggaac gtgctgtctc tccccacacc cacctcaggc
ctcggacgcg cccagcgttg ggccgcaggc ctgggggca ctgccccgtc ttatggaaac
ccgagcagcg acgtccaggc ggatggagcc ttggggtccc ggagcccgcc gggagtgggc
ccagaagaag aggaagggga gggctatgag gaacctgaca gtgaggagga ctccgagttc
tatgagaacg actccaacct tgggcaggac cagctctccc aggatggcag cggctacgag
aaccctgagg atgagcccct gggtcctgag gatgaagact ccttctccaa cgctgagtct
tatgagaacg aggatgaaga gctgacccag ccggtcgcca ggacaatgga cttcctgagc
cctcatgggt cagcctggga ccccagccgg gaagcaacct ccctggggtc ccagtcctat
gaggatatga gaggaatcct gtatgcagcc ccccagctcc gctccattcg ggccagcct
ggacccaatc atgaggaaga tgcagactct tatgagaaca tggataatcc cgatgggcca
gacccagcct ggggaggagg gggccgcatg ggcacctgga gcaccaggtg a

**SEQ ID NO: 17**

atgtggcccc tggtagcggc gctgttgctg ggctcggcgt gctgcggatc agctcagcta
ctatttaata aaacaaaatc tgtagaattc acgttttgta atgacactgt cgtcattcca
tgctttgtta ctaatatgga ggcacaaaac actactgaag tatacgtaaa gtggaaattt
aaaggaagag atatttacac ctttgatgga gctctaaaca agtccactgt ccccactgac
tttagtagtg caaaaattga agtctcacaa ttactaaaag gagatgcctc tttgaagatg
gataagagtg atgctgtctc acacacagga aactacactt gtgaagtaac agaattaacc
agagaaggtg aaacgatcat cgagctaaaa tatcgtgttg tttcatggtt ttctccaaat
gaaaatattc ttattgttat tttcccaatt tttgctatac tcctgttctg ggacagttt
ggtattaaaa cacttaaata tagatccggt ggtatggatg agaaaacaat tgctttactt
gttgctggac tagtgatcac tgtcattgtc attgttggag ccattctttt cgtcccaggt
gaatattcat taaagaatgc tactggcctt ggtttaattg tgacttctac agggatatta
atattacttc actactatgt gtttagtaca gcgattggat taacctcctt cgtcattgcc
atattggtta ttcaggtgat agcctatatc ctcgctgtgg ttggactgag tctctgtatt
gcggcgtgta taccaatgca tggccctctt ctgatttcag gtttgagtat cttagctcta
gcacaattac ttggactagt ttatatgaaa tttgtggctt ccaatcagaa gactatacaa
cctcctagga aagctgtaga ggaacccctt aatgcattca aagaatcaaa aggaatgatg
aatgatgaat aa

SEQ ID NO: 18

tttgtagtgg gaggatacct ccagagaggc tgctgctcat tgagctgcac tcacatgagg
atacagactt tgtgaagaag gaattggcaa cactgaaacc tccagaacaa aggctgtcac
taaggtcccg ctgccttgat ggattataca cttgacctca gtgtgacaac agtgaccgac
tactactacc ctgatatctt ctcaagcccc tgtgatgcgg aacttattca gacaaatggc
aagttgctcc ttgctgtctt ttattgcctc ctgtttgtat tcagtcttct gggaaacagc

ctggtcatcc tggtccttgt ggtctgcaag aagctgagga gcatcacaga tgtatacctc
ttgaacctgg ccctgtctga cctgcttttt gtcttctcct tcccctttca gacctactat
ctgctggacc agtgggtgtt tgggactgta atgtgcaaag tggtgtctgg cttttattac
attggcttct acagcagcat gtttttcatc accctcatga gtgtggacag gtacctggct
gttgtccatg ccgtgtatgc cctaaaggtg aggacgatca ggatgggcac aacgctgtgc
ctggcagtat ggctaaccgc cattatggct accatcccat tgctagtgtt ttaccaagtg
gcctctgaag atggtgttct acagtgttat tcattttaca atcaacagac tttgaagtgg
aagatcttca ccaacttcaa aatgaacatt ttaggcttgt tgatcccatt caccatcttt
atgttctgct acattaaaat cctgcaccag ctgaagaggt gtcaaaacca caacaagacc
aaggccatca ggttggtgct cattgtggtc attgcatctt tacttttctg ggtcccattc
aacgtggttc ttttcctcac ttccttgcac agtatgcaca tcttggatgg atgtagcata
agccaacagc tgacttatgc cacccatgtc acagaaatca tttcctttac tcactgctgt
gtgaaccctg ttatctatgc ttttgttggg gagaagttca agaaacacct ctcagaaata
tttcagaaaa gttgcagcca aatcttcaac tacctaggaa gacaaatgcc tagggagagc
tgtgaaaagt catcatcctg ccagcagcac tcctcccgtt cctccagcgt agactacatt
ttgtgaggat caatgaagac taaatataaa aaacattttc ttgaatggca tgctagtagc
agtgagcaaa ggtgtgggtg tgaaaggttt ccaaaaaaag ttcagcatga aggatgccat
atatgttgtt gccaacactt ggaacacaat gactaaagac atagttgtgc atgcctggca
caacatcaag cctgtgattg tgtttattga tgatgttgaa caagtggtaa ctttaaagga
ttctgtatgc caagtgaaaa aaaaagatgt ctgacctcct tacatat

**SEQ ID NO: 19**

attctttctt cgtgttcctg tgcgggattg gtgtgcccag ggtttggct ttccaattgg
ctaacgccgg ggtgggtggg gaatgtgggg agatttgaat ttgaaaccgg tagggagtga
taatccgcat tcagttgtcg aggagtgcca gtcaccttca gtttctggag ctggccgtca
acatgtcctt tcctaaggcg cccttgaaac gattcaatga cccttctggt tgtgcaccat
ctccaggtgc ttatgatgtt aaaactttag aagtattgaa aggaccagta tcctttcaga
aatcacaaag atttaaacaa caaaaagaat ctaaacaaaa tcttaatgtt gacaaagata
ctaccttgcc tgcttcagct agaaaagtta agtcttcgga atcaaagaag gaatctcaaa
agaatgataa agatttgaag atattagaga aagagattcg tgttcttcta caggaacgtg
gtgcccagga caggcggatc caggatctgg aaactgagtt ggaaaagatg gaagcaaggc
taaatgctgc actaagggaa aaaacatctc tctctgcaaa taatgctaca ctggaaaaac
aacttattga attgaccagg actaatgaac tactaaaatc taagttttct gaaaatggta
accagaagaa tttgagaatt ctaagcttgg agttgatgaa acttagaaac aaaagagaaa
caaagatgag ggtatgatg gctaagcaag aaggcatgga gatgaagctg caggtcaccc
aaaggagtct cgaagagtct caagggaaaa tagcccaact ggagggaaaa cttgtttcaa
tagagaaaga aaagattgat gaaaaatctg aaacagaaaa actcttggaa tacatcgaag
aaattagttg tgcttcagat caagtggaaa aatacaagct agatattgcc cagttagaag
aaaatttgaa agagaagaat gatgaaattt taagccttaa gcagtctctt gaggagaata
ttgttatatt atctaaacaa gtagaagatc taaatgtgaa atgtcagctg cttgaaaaag
aaaaagaaga ccatgtcaac aggaatagag aacacaacga aaatctaaat gcagagatgc
aaaacttaaa acagaagttt attcttgaac aacaggaacg tgaaaagctt caacaaaaag
aattacaaat tgattcactt ctgcaacaag agaaagaatt atcttcgagt cttcatcaga
agctctgttc ttttcaagag gaaatggtta aagagaagaa tctgtttgag gaagaattaa
agcaaacact ggatgagctt gataaattac agcaaaagga ggaacaagct gaaaggctgg
tcaagcaatt ggaagaggaa gcaaaatcta gagctgaaga attaaaactc ctagaagaaa
agctgaaagg gaaggaggct gaactggaga aaagtagtgc tgctcatacc caggccaccc
tgcttttgca ggaaaagtat gacagtatgg tgcaaagcct tgaagatgtt actgctcaat
ttgaaagcta taaagcgtta acagccagtg agatagaaga tcttaagctg gagaactcat
cattacagga aaaagcggcc aaggctggga aaaatgcaga ggatgttcag catcagattt
tggcaactga gagctcaaat caagaatatg taaggatgct tctagatctg cagaccaagt
cagcactaaa ggaaacagaa attaaagaaa tcacagtttc ttttcttcaa aaaataactg
atttgcagaa ccaactcaag caacaggagg aagactttag aaaacagctg gaagatgaag
aaggaagaaa agctgaaaaa gaaaatacaa cagcagaatt aactgaagaa attaacaagt
ggcgtctcct ctatgaagaa ctatataata aaacaaaacc ttttcagcta caactagatg
cttttgaagt agaaaaacag gcattgttga atgaacatgg tgcagctcag gaacagctaa

ataaaataag agattcatat gctaaattat tgggtcatca gaatttgaaa caaaaaatca
agcatgttgt gaagttgaaa gatgaaaata gccaactcaa atcggaagta tcaaaactcc
gctgtcagct tgctaaaaaa aaacaaagtg agacaaaact tcaagaggaa ttgaataaag
ttctaggtat caaacacttt gatccttcaa aggcttttca tcatgaaagt aaagaaaatt
ttgccctgaa gaccccatta aaagaaggca atacaaactg ttaccgagct cctatggagt
gtcaagaatc atggaagtaa acatctgaga aacctgttga agattatttc attcgtcttg
ttgttattga tgttgctgtt attatatttg acatgggtat tttataatgt tgtatttaat
tttaactgcc aatccttaaa tatgtgaaag gaacattttt taccaaagtg tcttttgaca
ttttattttt tcttgcaaat acctcctccc taatgctcac ctttatcacc tcattctgaa
ccctttcgct ggctttccag cttagaatgc atctcatcaa cttaaaagtc agtatcatat
tattatcctc ctgttctgaa accttagttt caagagtcta aaccccagat tcttcagctt
gatcctggag gtcttttcta gtctgagctt ctttagctag gctaaaacac cttggcttgt
tattgcctct actttgattc tgataatgct cacttggtcc tacctattat ccttctactt
gtccagttca aataagaaat aaggacaagc ctaacttcat agaaacctct ctatttttaa
tcagttgttt aataatttac aggttcttag gctccatcct gtttgtatga aattataatc
tgtggattgg cctttaagcc tgcattctta acaaactctt cagttaattc ttagatacac
taaaaatctg agaaactcta catgtaacta tttcttcaga gtttgtcata tactgcttgt
catctgcatg tctactcagc atttgattaa catttgtgta atatgaaata aaattacaca
gtaagtcatt taaccaatta aaaa

**SEQ ID NO: 20**

ggaaggcttg cacagggtga aagctttgct tctctgctgc tgtaacaggg actagcacag
acacacggat gagtggggtc atttccagat attaggtcac agcagaagca gccaaaatgg
atccccagtg cactatggga ctgagtaaca ttctctttgt gatggccttc ctgctctctg
gtgctgctcc tctgaagatt caagcttatt tcaatgagac tgcagacctg ccatgccaat
ttgcaaactc tcaaaaccaa agcctgagtg agctagtagt attttggcag gaccaggaaa
acttggttct gaatgaggta tacttaggca aagagaaatt tgacagtgtt cattccaagt
atatgggccg cacaagtttt gattcggaca gttggaccct gagacttcac aatcttcaga
tcaaggacaa gggcttgtat caatgtatca tccatcacaa aaagcccaca ggaatgattc
gcatccacca gatgaattct gaactgtcag tgcttgctaa cttcagtcaa cctgaaatag
taccaatttc taatataaca gaaaatgtgt acataaattt gacctgctca tctatacacg
gttacccaga acctaagaag atgagtgttt tgctaagaac caagaattca actatcgagt
atgatggtgt tatgcagaaa tctcaagata atgtcacaga actgtacgac gtttccatca
gcttgtctgt ttcattccct gatgttacga gcaatatgac catcttctgt attctggaaa
ctgacaagac gcggctttta tcttcacctt tctctataga gcttgaggac cctcagcctc
ccccagacca cattccttgg attacagctg tacttccaac agttattata tgtgtgatgg
ttttctgtct aattctatgg aaatggaaga agaagaagcg gcctcgcaac tcttataaat
gtggaaccaa cacaatggag agggaagaga gtgaacagac caagaaaaga gaaaaaatcc
atatacctga aagatctgat gaagcccagc gtgtttttaa aagttcgaag acatcttcat
gcgacaaaag tgatacatgt ttttaattaa agagtaaagc ccatacaagt attcattttt
tctacccttt cctttgtaag ttcctgggca acctttttga tttcttccag aaggcaaaaa
gacattacca tgagtaataa gggggctcca ggactccctc taagtggaat agcctccctg
taactccagc tctgctccgt atgccaagag gagactttaa ttctcttact gcttcttttc
acttcagagc acacttatgg gccaagccca gcttaatggc tcatgacctg gaaataaaat
ttaggaccaa tacctcctcc agatcagatt cttctcttaa tttcatagat tgtgtttttt
ttttaaatag acctctcaat ttctggaaaa ctgcctttta tctgcccaga attctaagct
ggtgccccac tgaattttgt gtgtacctgt gactaaacaa ctacctcctc agtctgggtg
ggacttatgt atttatgacc ttatagtgtt aatatcttga aacatagaga tctatgtact
gtaatagtgt gattactatg ctctagagaa aagtctaccc ctgctaagga gttctcatcc
ctctgtcagg gtcagtaagg aaaacggtgg cctagggtac aggcaacaat gagcagacca
acctaaattt ggggaaatta ggagaggcag agatagaacc tggagccact tctatctggg
ctgttgctaa tattgaggag gcttgcccca cccaacaagc catagtggag agaactgaat
aaacaggaaa atgccagagc ttgtgaaccc tgtttctctt gaagaactga ctagtgagat
ggcctgggga agctgtgaaa gaaccaaaag agatcacaat actcaaaaga gagagagaga
gaaaaaagag agatcttgat ccacagaaat acatgaaatg tctggtctgt ccaccccatc
aacaagtctt gaaacaagca acagatggat agtctgtcca aatggacata agacagacag

```
cagtttccct ggtggtcagg gaggggtttt ggtgataccc aagttattgg gatgtcatct
tcctggaagc agagctgggg agggagagcc atcaccttga taatgggatg aatggaagga
ggcttaggac tttccactcc tggctgagag aggaagagct gcaacggaat taggaagacc
aagacacaga tcacccgggg cttacttagc ctacagatgt cctacgggaa cgtgggctgg
cccagcatag ggctagcaaa tttgagttgg atgattgttt ttgctcaagg caaccagagg
aaacttgcat acagagacag atatactggg agaaatgact ttgaaaacct ggctctaagg
tgggatcact aagggatggg gcagtctctg cccaaacata aagagaactc tggggagcct
gagccacaaa aatgttcctt tattttatgt aaaccctcaa gggttataga ctgccatgct
agacaagctt gtccatgtaa tattcccatg tttttaccct gcccctgcct tgattagact
cctagcacct ggctagtttc taacatgttt tgtgcagcac agtttttaat aaatgcttgt
tacattcaaa aaaaaaaaaa aaaaaaaaaa aaaaaaaaaa aaaaaaaaaa aaaaaaaaaa
aaaaaaaaaa aaaaaaaaaa aaaa
```

## EXAMPLES

### Example 1 - AML diagnostic FACS screen

Patient samples

Bone marrow samples from AML patients and patients undergoing orthopaedic surgery with normal blood counts/films were obtained with informed consent (protocols 06/Q1606/110 and 05/MRE07/74). Bone marrow samples were received in EDTA (Heparin is a suitable alternative) and were lysed with ammonium chloride prior to immunostaining.

Samples received in culture medium were washed and the sample resuspended in PBS before analysis. To do this the sample was transferred to a conical tube (10ml), which was topped up with PBS and centrifuged for 5 minutes at 3000rpm. The supernatant was removed using a pipette and the cells resuspended in PBS to ~2ml.

Occasionally diagnostic panels were run on peripheral blood samples if the blast count was high enough. The technique used was identical to that for bone marrow.

Samples were prepared as soon as possible after receipt.

Lysis

Ammonium Chloride was used for lysis:

| | |
|---|---|
| $NH_4CL_2$: | 8.30g |
| $KHCO_3$: | 1.00g |
| $Na_2EDTa$: | 0.38g |

Made up to 1 Litre with Distilled Water for a working solution which was stable for 1 month.

1. Diluted BM/PB with a least a 10 fold excess of Ammonium Chloride Lyse, in an appropriate centrifuge tube (20 or 50ml conical tubes)
2. Incubated for 5-10 minutes, examined for red cell lysis. If red cells showed resistance to lysis incubated for a further 5-10 minutes at 37 or 4°c
3. Spun sample down at 300g for 5min
4. Tipped off supernatant and topped up with PBS
5. Spun sample down at 300g for 5 min
6. Resuspended pellet in 1 ml of PBS and obtained white cell count

Adjusted the white cell count of samples for diagnostic studies to ~5 x $10^9$/l by dilution with PBS.

Red cell contamination will interfere with scatter plots and reduce overall MRD %

Excessive lysis will result in changes to FSC/SSC scatter properties

Panel Set Up

1. Added 5ul of the following antibodies to a FACS tube:

CD34 Percp Becton Dickinson 345803
CD45 APC-H7 Becton Dickinson 641389
CD47 PE Pharmingen 556046
CD38 PE-CY7 Becton Dickinson 335825
CD19 Pacific Bleu E Biosciences 87-0199-33
CD90
CD123
CRR8
RHAMM
CD86

2. Added 100μl of pre lysed sample to each of tubes
3. Vortexed each tube
4. Incubated at RT in dark for 15 minutes
5. Topped up each tube with PBS
6. Centrifuged at 300g for 5 minutes
7. Tipped off/aspirate supernatant
8. Resuspended cell pellet in 500μl PBS
9. Vortexed well
10. The samples were now ready for acquisition

Acquisition

1. Acquired 500,000 cells using a FacsDiva Protocol as follows:
2. Define a CD45 vs. SSC plot
3. Gate all CD45 positive cells (Gate P1)
4. Project these cells onto a CD34 vs. SSC plot
5. Gate CD34 positive events (Gate P2)
6. Refine the CD34 positive population on a CD45 vs. SSC plot (Gate P3).
7. Further refine the CD34 population on a FSC vs. SSC plot (Gate P4)
8. The combined information satisfying gates P1, 2, 3 and 4 are projected on a CD19 vs. CD34 plot. CD34+CD19- cells are gated (Gate P5)
9. Events from P5 (CD34+CD19-) are projected onto a CD45 vs. CD34 gate.
10. Events from P5 (CD34+CD19-) are projected onto an exponential CD38 vs. CD34 gate.
11. Define regions for CD38-CD34+ (gate P7) and CD38-CD34+ cells (gate P8)
12. Events from P5 (CD34+CD19-) are projected onto a CD38 vs. CD47 plot.

Results

**Immunophenotype of Lin-CD34+CD38- and Lin-CD34+CD38+ compartments in human AML: there are two major immunophenotypic groups of AML.**

We used cell surface markers to compare patterns of stem/progenitor-cell immunophenotypes in CD34+ primary AML and normal control samples. The immunophenotypic gating strategy is illustrated in Figure 1 was validated by reanalyzing purity of FACS-sorted stem/progenitor populations and showed that the sorted populations were >99% pure (Figure 2). In vitro colony assays and in vivo assays confirmed the functional potential of sorted normal stem/progenitor cells. Using this approach, we immunophenotyped 82 primary AML samples (57 de novo AML, 14 secondary AML, 6 relapsed AML and 5 refractory AML - spanning a range of FAB subtypes, cytogenetic categories and *FLT3* and *NPM1* mutation states and 8 age-matched control marrow samples.

For the first time, we showed that there are two major immunophenotypic groups in primary human CD34+ AML with respect to these markers (Figure 3). 82% of the 56 AML samples (and 77.8% of the 45 de novo AML samples), most of the Lin-CD34+CD38-cells are CD34+CD38-CD90-CD45RA+ (hereafter termed "CD38-CD45RA+") (57%-100% of CD34+CD38- cells and in most cases >90%). Hereafter, this group is named "CD45RA+ expanded" group (Figure 3Aii). Less commonly, in 22.2% of the 56 primary AML samples there is dominant Lin-CD34+CD38-CD90-CD45RA- population (66%-99% of CD34+CD38- cells and in most cases >90%). This group was only seen in de novo AML cases. Hereafter, this group is named "MPP-like expanded" group (Figure 3Aiii).

Within these two major groups there are variations of CD38 expression between AML samples (Figure 3B). Within the both groups nearly all cells can be CD38-(Figure 3Bii and iv), or a proportion of cells can express CD38 (Figure 3Biii and v) and finally in the 45RA+-like expanded group the majority of cells can be CD38+.

The central novel finding is that 82% of a broad range of primary CD34+ expressing AML samples have one major distinct CD38-CD45RA+ population within the CD34+CD38- compartment and a corresponding GMP-like population within the CD34+CD38+ compartment.

**Leukaemic stem cell hierarchy in CD45RA+ expanded AML**

Given the CD45RA+ expanded group is the major group, we focused on dissecting which populations within this group had LSC activity in a xenotransplant assay. Previous data had shown that in CD34+ AML LSC activity resides in the CD34 compartment. Therefore, FACS-sorted CD38-CD45RA+ and GMP-like cells from 6 patients (10 populations in total) were injected intravenously into NOD-SCID mice treated with anti-CD122 antibody to remove residual NK-cells (Figure 4A). Samples details are in Figure 12. 105 cells/mouse of each population were injected into 4 mice. Cells from 5 out of 6 patients were detected in bone marrow in all 4 mice injected for each of the 10 populations from these 5 patients. There was a similar level of engraftment from CD38-CD45RA+ and GMP-like populations, consistent with LSC activity in both the CD38- and CD38+ compartments. FISH analysis on FACS-sorted human engrafted cells from mice injected with CD38-CD45RA+ and GMP-like cells from 2 AML patients with cytogenetic abnormalities showed that nearly all cells were leukaemic (Figure 4B and Figure 13).

FACS analysis confirmed that for both CD38-CD45RA+ and GMP-like injected populations, CD34+ and CD34- cells were detected in bone marrow (Figure 4Ci and iv). In both CD34+ and CD34- populations in mice injected with CD38-CD45RA+ (Figure 4Cii and iii) and GMP-like (Figure 4Cv and vi) populations, nearly all cells expressed CD33 but not CD19, consistent with leukaemic myeloid-restricted engraftment as opposed to multi-potential myelo-lymphoid engraftment from normal blood cells. In mice injected with CD38-CD45RA+ cells, Lin-CD34+CD38-CD90-CD45RA+ cells were detected consistent with self-renewal of this population (Figure 4Di and ii). CD38-CD45RA+ cells also gave rise to GMP-like cells in vivo (Figure 4Diii). In contrast, when GMP-like cells are injected in mice, <0.1% of the CD34+ cells are CD34+CD38-CD45RA+ (Figure 4Div). Taken together, this is consistent with an in vivo hierarchy where a minority of CD34+CD38- cells self renew and give rise to GMP-like cells whereas GMP-like cells cannot give rise to CD34+CD38-CD45RA+ cells.

To prove that CD38-CD45RA+ and GMP-like AML populations have leukemic stem cell activity defined by secondary engraftment, human cells were harvested from primary recipients and injected into secondary NOD-SCID hosts treated with anti-CD122 antibody (Figure 4E). Engraftment in secondary hosts was seen at 12 weeks from all 5 samples with no difference in engraftment level between cells taken from primary hosts transplanted with either CD38-CD45RA+ or GMP-like populations. The overall engraftment level of was lower in secondary hosts compared to primary transplanted animals as previously reported in the literature. Detailed immunophenotypic analysis of the engrafted cells showed that in all cases >99% of the human cells were CD33+ and CD19- consistent with engraftment of myeloid LSC rather than normal HSC (Figure 5). The majority of the hCD34+ cells were CD38+CD110+CD45RA+ (i.e. GMP-like) regardless of whether the injected cells were from animals initially injected with CD38-CD45RA+ or GMP-like LSC. This would be consistent with differentiation of CD38-CD45RA+ cells into GMP-like cells but not in the reverse direction. In summary, both CD38-CD45RA+ and GMP-like populations, within the same patient, have LSC activity. In vivo, CD38-CD45RA+ LSC give rise to cells with a GMP-like phenotype and the converse does not occur.

**In vitro differentiation of AML LSC populations**

To confirm the in vivo observations, CD38-CD45RA+ cells and GMP-like populations were FACS-sorted from 5 AML patients (10 populations) and each population was cultured on MS5 stroma with cytokines (Figure 6). The cultures were analyzed 4 and 8 days after culture initiation. After 4 days, most GMP-like cells remained CD38- and were CD90-CD45RA+ (Figure 6Bii). The remaining input CD38- cells gained CD38 expression.-By day 8, the original CD38-CD45RA+ cells were nearly all CD38+ and a proportion of cells has lost CD34 expression; consistent with differentiation into a CD34- blast population (Figure 6Biii). The small numbers of CD34+CD38- cells were all CD90-CD45RA+, consistent with the immunophenotype of input cells.

After 4 days of culture of CD38+CD45RA+ cells nearly all cells remained CD38+ and some cells had already lost CD34 expression (Figure 6Bii). There was little differentiation of CD34+CD38+ cells into CD34+CD38- cells.

Thus, the sum of the in vivo and in vitro data suggest that a CD38-CD45RA+ population lies at the top of hierarchy in most cases of primary human AML and differentiates into GMP-like population but notably both populations have LSC activity.

## Gene expression profiles of AML LSC and normal haemopoietic stem/progenitor cells

We then obtained global mRNA expression profiles from 22 FACS-sorted AML CD38-CD45RA+, 21 GMP-like populations from 22 patients. In 18 patients we were able to obtain both CD38-CD45RA- and GMP-like AML populations allowing us to compare expression profiles between the two populations within each patient, thus negating the effect of genetic and epigenetic changes between patients. We also obtained from 5 normal HSC, MPP, CMP, GMP and CD38-CD45RA+ populations from 5 different age-matched human marrow samples. We asked two questions: first, are the two AML LSC populations (CD38-CD45RA+ and GMP-like) molecularly distinct; and second which normal populations are the two AML LSC populations most closely related to at a molecular level.

We used two approaches to determine if the expression profiles for the two AML LSC populations were distinct. First, we used a paired t-test (cut-off 0.01) to obtain a list of differentially expressed genes (917 probes; 748 mapped genes) between CD38-CD45RA+ and GMP-like cells from the subset of 18 AML cases where both populations were available from the same patient. The expression profiles of these differential genes was displayed by 3D Principal Component Analysis (PCA) (Figure 7A). This shows that though the majority of the two populations are separated, 5/18 of the GMP-like populations lie interspersed with the CD38-CD45RA+ populations. This was confirmed standard t-test (cut-off 0.01) when applied to all AML population expression profiles - 22 CD38-CD45RA+ and 21 GMP-like populations) was used to obtain a list of differentially expressed genes (458 probes; 360 genes) (Figure 8A). We also examined the relationship between CD38-CD45RA+ AML and GMP-like AML populations by hierarchical clustering with this minimum gene set (Figure 8B). In this analysis, 5/21 GMP-like AML populations lie amongst CD38-CD45RA+ AML populations and 1/22 CD38-CD45RA+ AML population within the GMP-like AML populations.

Secondly, to obtain a more quantitative measure of the difference in gene expression between the two AML populations with LSC activity, we compared expression between CD38-CD45RA+ and GMP-like populations from the same patient, by a non-parametric (rank product) method (Figure 7B). With a false discovery rate of 0.05 (pfp=0.5), 443 mapped genes were expressed more highly in CD38-CD45RA+ compared to GMP-like populations (Figure 7Bi). Similarly, 1496 genes were more highly expressed in GMP-like compared to CD38-CD45RA+ populations (Figure 7Bii). Using more stringent false discovery rate cut off of 0.01, 200 genes are expressed more highly in CD38-CD45RA+ populations and 943 genes are expressed more highly in GMP-like populations (data not shown). CD38 was amongst the top twenty most differentially expressed genes. Taken together, CD38-CD45RA+ and GMP-like LSC populations are molecularly distinct but show some overlap in gene expression and may not be fully separated.

To determine which normal populations the two AML LSC populations most closely resemble molecularly, we used ANOVA to curate a 2628 gene set (2789 probes) that maximally distinguished normal stem and progenitor populations. 3-D PCA was then used to display the profiles from the ANOVA curated gene set from normal populations (Figure 7C). The signature of normal HSC was most closely related to the normal MPPs, with the normal CD38-CD45RA+, CMP and GMP populations more widely scattered. Each normal immunophenotypic population are relative closely co-located. Next, using this same 2628 ANOVA gene the expression profile of 22 CD38-CD45RA+ and 21 GMP-like populations AML populations were then distributed (Figure 7Ci and ii). Both AML populations are more widely dispersed (presumably reflecting heterogeneity of genetic/epigenetic changes in AML). CD38-CD45RA+ and GMP-like AML populations mainly cluster around their normal counterpart population. However, some GMP-like AML populations are located closer to normal CD38-CD45RA+ cells.

Next, we used the same ANOVA gene set as a classifier to ask which normal population did each individual AML LSC populations most closely resemble (Figure 7E-G). 17/22 (77%) CD38-CD45RA+ AML populations were classified as normal CD38-CD45RA+ cells and 5/22 (28%) as GMPs. Only 278 probes (corresponding to 272 genes) (threshold 4.0) were required for the classifier. Additional genes did not provide further discriminatory power. Likewise for the GMP-like AML populations, 13/21 samples were called as GMP (62%), 7 as CD38-CD45RA+ (33%) and 1 sample as CMP (5%) using 241 probes (corresponding to 235 genes) (threshold 4.23). Similar results were obtained by hierarchical clustering using these same minimum gene sets that minimises misclassification error (thresholds of 4 and 4.23) (Figure 8C and D). 4/22 CD38-CD45RA+ AML populations were closer to normal GMP than normal CD38-CD45RA+ (Figure 8C); whereas 5/21 GMP AMLs were closer to normal CD38-CD45RA+ than to normal GMP (Figure 8D).

Taken together, the gene expression profiles of both AML populations with LSC activity do not map most closely to normal HSC.

## Normal CD38-CD45RA+ population has cells with lymphoid primed multipotential (LMPP) potential.

The gene expression data above showed that global expression profiles of CD38-CD45RA+ AML most closely resemble normal CD38-CD45RA+ cells. The expression data suggested that the normal CD38-CD45RA+ population was distinct fro other stem/progenitor cells. Previous studies had not shed light on the function of normal CD38-CD45RA+ cells. To investigate the lineage potential of CD38-CD45RA+ cells, we performed colony assays (Figure 9A). HSC, MPP, CMP, GMP and MEP populations produced the expected lineage output. The CD38-CD45RA+ population had a cloning efficiency of -30% and produced granulocyte, macrophage and mixed granulocyte-macrophage colonies. Importantly, there was no erythroid output. When cells from the initial colony assay were replated, cells from CD38-CD45RA+ colonies had a replating potential intermediate between that of cells from MPP- and CMP-derived colonies (Figure 5B). CD38-CD45RA+ cells exhibited no megakaryocyte potential, in contrast to MEP, CMP, HSC and MPP (Figure 9C).

The B-lymphoid potential of CD38-CD45RA+ cells was tested by co-culture on MS5 stroma with cytokines. As positive control both HSC and MPP populations differentiated into myeloid (CD33-expressing) and B-lymphoid cells (CD19-expressing) cells whereas CMPs only gave rise to CD33+ cells (data not shown). CD38-CD45RA+ cells differentiated into both CD33+ and CD19+ cells (data not shown). The frequency of cells with myeloid, B- and T-lymphoid potential in the CD38-CD45RA+ population was determined by limiting dilution analysis (Figure 9D-H). On MS5 stroma, 1/3.79 CD38-CD45RA+ cells had myeloid potential (compared to 1/12.4 GMP cells) (Figure 9D). In conditions that promote both B-cell and myeloid output, 1/6.38 CD38-CD45RA+ cells differentiated into CD19 expressing B-cells and myeloid cells, whereas 1/95.79 GMP cells showed similar potential (Figures 9E and 9F). B-cell and myeloid potential was always seen together. Finally, the T-cell potential of the CD38-CD45RA+ cells was tested in an OP9-DL1 co-culture assay. 1/12 CD38-CD45RA+ cells expressed CD1a, CD7 and CD3 (Figures 9G and 9H) compared to 1/32 GMP cells. We also searched for early TCRd□ VD, DD or DJ and IgH DJ gene rearrangements in CD38-CD45RA+ cells, as compared to HSC, MPP, CMP and GMP controls but there was no evidence of significant polyclonal rearrangements at either loci in CD38-CD45RA+ DNA or in control DNA (data not shown).

**Expression of lymphoid- and GM-specific genes in CD38-CD45RA+ cells.**

Murine multi-potential stem/progenitor cells express low levels of multiple lineage-affiliated gene expression programmes concordant with their lineage potentials (termed multilineage priming). As these cells pass through lineage restriction points, losing lineage potential, there is gradual, concomitant, extinction of lineage-affiliated gene expression programmes. In a refinement of this concept it has been suggested that there is a cascade of lineage-affiliated transcriptional signatures, initiated in HSCs and propagated in a differential manner in lineage-restricted progenitors (Figure 10A).

Whether this also occurs in human haemopoietic stem/progenitor cells has not been previously reported. Therefore, we used quantitative RT-PCR to study expression of select lineage-affiliated genes shown in mouse to be representative of lineage-affiliated gene expression programmes, in 10 and 100 FACS sorted normal HSC, CD38- CD45RA+, GMP and MEP cells (Figure 10B and Figure 11). The aim was two-fold. First, to establish if lineage-affiliated patterns of gene expression seen in the mouse held true in human and, second, to determine if CD38-CD45RA+ cells expressed a lineage affiliated transcriptional programmes similar to murine LMPP cells (myelo-lymphoid gene expression and diminishing levels, or a lack of expression, of erythroid-megakaryocyte affiliated genes). The gene set chosen for study was based on previous published data and our own transcriptional profiling.

Consistent with previous data we found that in 10 FACS-sorted cells, MPL and HLF were most highly expressed in HSC (Figure 10Bi) (representative of stem-cell only genes). Using our own gene expression data we confirmed that the stem/progenitor regulators BMI1 and MEIS1 were expressed in HSC and CD38-CD45R+ cells, with markedly lower expression in GMP and MEP. Similarly, KIT, IKZF1 (IKAROS) and RUNX1 are also expressed in HSC and CD38-CD45RA+ cells but also are expressed in GMP and MEP. Finally, HOXA9 and, to a lesser extent, IL3RA, are expressed principally only in CD38-CD45RA+ cells.

Turning to the myeloid lineage affiliated genes, the early myeloid genes CEBPA, CSF3R, SPI1 (PU.1) are all expressed in HSC with expression retained in CD38-CD45RA+ and GMP cells but extinguished, as expected, in MEP (Figure 10Bii).

These are reminiscent of stem cell/myelolymphoid genes. The next pattern of myeloid gene expression is seen primarily in CD38-CD45RA+ and GMP cells (CSFR2A and GFI1) (i.e. restricted myelo-lymphoid) and, finally, the last layer of myeloid gene expression are late myeloid-specific genes (MPO and CSFR1), which are mainly expressed in GMPs only (differentiated myeloid). The pattern of early lymphoid gene expression is more focused; CD79A, ETS1, VPREB1, sterile IGHM, FLT3, NOTCH1 and RUNX3 all maximally expressed in CD38-CD45RA+ with little expression in other cell types (Figure 10Biii). Finally, early stem-erythroid-megakaryocyte gene expression is epitomized by VWF, TAL1 and GATA2, which are all expressed in HSC and MEP. Both VWF and TAL1 are expressed at much lower levels in CD38-CD45RA+ cells, though GATA2 continues to be expressed in these cells, consistent with its broader role in haemopoietic progenitors (including GMP) biology. The erythroid-specific cytokine receptor EPOR, the erythroid-megakaryocyte transcription factor GATA1 and late erythroid transcription factor KLF1 show a more restricted pattern with expression principally in MEPs. Importantly, none of these three genes is expressed in CD38-CD45RA+ cells. Identical patterns of gene expression were obtained when either 10 or 100 FACS sorted cells where used (Figure 10 and Figure 11).

The sum of the gene expression data is consistent with multilineage priming in primary human stem/progenitors in a manner analogous to mouse. Moreover, these data are consistent with lymphoid and myeloid (GM), but lack of erythroid-megakaryocyte lineage potential of CD38-CD45RA+ cells described earlier. The sum of the functional and molecular data suggests that CD38-CD45RA+ cells are most similar to mouse LMPP cells.

**Example 2 - Prognostic application of diagnostic screen**

A 49 year old male suffering from symptoms of pancytopenia presents himself to hospital. 10ml of blood and/or 2 mls of bone marrow is removed for diagnostic.
Are for flow cytometery evaluation. The biological samples are treated either as in Example 1 or with red cell lysis buffer to remove red cells. Then the nucleated cells are incubated with antibodies as described in Example 1 that are either directly conjugated or indirectly conjugated. Excess unbound antibody is washed off. The stained cells are then put through a flow cytometer. Data is then collected and prognosis is made.

**Example 3 - Use of the diagnostic screen in an in vitro assay to identify a therapeutic candidate**

A 33 year old with known Acute Myeloid Leukaemia present himself in hospital. 10ml of blood and/or 2 mls of bone marrow is removed to monitor residual leukaemia stem cells for flow cytometery evlauation. The biological samples are treated either as in Example 1 or with red cell lysis buffer to remove red cells. Then the nucleated cells are incubated with antibodies as described in Example 1 that are either directly conjugated or indirectly conjugated. Excess unbound antibody is washed off. The stained cells are then put through a flow cytometer. Data is then collected and the effect of a therapeutic candidate assessed.

## Claims

**1.** A method for diagnosis of acute myeloid leukaemia comprising:

i. contacting an isolated sample containing a blood cell population with a screen that detects at least the following cell surface polypeptide markers: $CD34^+$; $CD45RA^+$; $CD123^+$; and $CD38^-$; wherein (+) indicates the presence and (-) indicates the absence of said cell surface polypeptide markers; and
ii. confirming the presence of a blood cell that has a cell surface phenotype comprising:

a. $CD34^+$;
b. $CD45RA^+$;
c. $CD123^+$; and
d. $CD38^-$;

wherein acute myeloid leukaemia is diagnosed when the presence of said blood cell is confirmed.

**2.** A method for determining prognosis of acute myeloid leukaemia comprising:

i. contacting an isolated sample containing a blood cell population with a screen that detects at least the following cell surface polypeptide markers: $CD34^+$; $CD45RA^+$; $CD123^+$; and $CD38^-$; wherein (+) indicates the presence and (-) indicates the absence of said cell surface polypeptide markers; and
ii. confirming the presence of a blood cell that has a cell surface phenotype comprising:

a. $CD34^+$;
b. $CD45RA^+$;
c. $CD123^+$; and
d. $CD38^-$;

wherein prognosis of acute myeloid leukaemia is determined when the presence of said blood cell is confirmed.

**3.** A method of identifying a therapeutic candidate for the treatment of acute myeloid leukaemia comprising:

i. contacting the therapeutic candidate with an isolated sample containing a population of blood cells, wherein said population of blood cells has a cell surface phenotype comprising:

a. $CD34^+$;
b. $CD45RA^+$;
c. $CD123^+$; and
d. $CD38^-$;

ii. incubating said therapeutic candidate with said isolated sample;
iii. contacting said isolated sample after step ii. with a screen that detects at least the following cell surface polypeptide markers: $CD34^+$; $CD45RA^+$; $CD123^+$; and $CD38^-$; wherein (+) indicates the presence and (-) indicates the absence of said cell surface polypeptide markers;
iv. identifying blood cells by step iii. that have a cell surface phenotype comprising:

a. $CD34^+$;
b. $CD45RA^+$;
c. $CD123^+$; and
d. $CD38^-$;

v. correlating the number of blood cells identified by step iv. with the number of blood cells present in an isolated sample prior to step i. that have a cell surface phenotype comprising:

a. $CD34^+$;
b. $CD45RA^+$;
c. $CD123^+$; and
d. $CD38^-$; and

vi. confirming the presence of a therapeutic candidate having anti-acute myeloid leukaemia cell activity by identifying a relative decrease in the number of blood cells in step v. after contact with the therapeutic candidate; or confirming the absence of a therapeutic candidate having anti-acute myeloid leukaemia cell activity by identifying no significant relative decrease in the number of blood cells in step v. after contact with the therapeutic candidate.

**4.** A method of monitoring efficacy of a therapeutic molecule in treating acute myeloid leukaemia comprising:

i. contacting an isolated sample from a patient, wherein said patient has been administered the therapeutic molecule, with a screen that detects at least the following cell surface polypeptide markers: $CD34^+$; $CD45RA^+$; $CD123^+$; and $CD38^-$; wherein (+) indicates the presence and (-) indicates the absence of said cell surface polypeptide markers;
ii. identifying blood cells by step i. that have a cell surface phenotype comprising:

a. $CD34^+$;
b. $CD45RA^+$;
c. $CD123^+$; and
d. $CD38^-$;

iii. correlating the number of blood cells identified by step ii. with the number of blood cells present in an isolated sample taken from a patient prior to administration of the therapeutic molecule, wherein said blood cells taken prior to administration of the therapeutic molecule have a cell surface phenotype comprising:

a. $CD34^+$;
b. $CD45RA^+$;
c. $CD123^+$; and
d. $CD38^-$; and

iv. confirming efficacy of the therapeutic molecule by identifying a relative decrease in the number of blood cells in step iii. after contact with the therapeutic molecule; or
confirming the absence of efficacy of the therapeutic molecule by identifying no significant relative decrease in the number of blood cells in step iii. after contact with the therapeutic molecule.

**5.** The method according to any one of the preceding claims, wherein the cell surface phenotype further comprises: $CD19^-$, $CD47^{+/-}$, $CCR8^{+/-}$, $RHAMM^{+/-}$, and/or $CD86^-$.

**6.** The method according to any one of the preceding claims, wherein the method comprises the use of one or more antibodies that bind to one or more cell surface polypeptide markers selected from: CD34, CD45RA, CD123, CD38, CD47, CD19, CCR8, RHAMM and CD86.

**7.** The method according to any one of the preceding claims, wherein the cell surface polypeptide markers are detected by antibodies, said antibodies comprising:

a first antibody that binds to CD34; and
a second antibody that binds to CD45RA; and
a third antibody that binds to CD123; and
a fourth antibody that binds to CD38.

**Patentansprüche**

**1.** Ein Verfahren zur Diagnose von akuter myeloischer Leukämie, das Folgendes beinhaltet:

i. In-Kontakt-Bringen einer isolierten Probe, die eine Blutzellenpopulation enthält, mit einem Screening-Test, der mindestens die folgenden Zelloberflächen-Polypeptidmarker nachweist: $CD34^+$; $CD45RA^+$; $CD123^+$; und $CD38^-$; wobei (+) das Vorhandensein und (-) das Fehlen der Zelloberflächen-Polypeptidmarker anzeigt; und
ii. Bestätigen des Vorhandenseins einer Blutzelle, die einen Zelloberflächen-Phänotyp aufweist, der die Folgenden beinhaltet:

a. $CD34^+$;
b. $CD45RA^+$;
c. $CD123^+$; und
d. $CD38^-$;

wobei akute myeloische Leukämie diagnostiziert wird, wenn das Vorhandensein der Blutzelle bestätigt wird.

**2.** Ein Verfahren zum Bestimmen der Prognose von akuter myeloischer Leukämie, das Folgendes beinhaltet:

i. In-Kontakt-Bringen einer isolierten Probe, die eine Blutzellenpopulation enthält, mit einem Screening-Test, der mindestens die folgenden Zelloberflächen-Polypeptidmarker nachweist: $CD34^+$; $CD45RA^+$; $CD123^+$; und $CD38^-$; wobei (+) das Vorhandensein und (-) das Fehlen der Zelloberflächen-Polypeptidmarker anzeigt; und
ii. Bestätigen des Vorhandenseins einer Blutzelle, die einen Zelloberflächen-Phänotyp aufweist, der die Folgenden beinhaltet:

a. $CD34^+$;
b. $CD45RA^+$;
c. $CD123^+$; und

d. $CD38^-$;

wobei die Prognose von akuter myeloischer Leukämie bestimmt wird, wenn das Vorhandensein der Blutzelle bestätigt wird.

**3.** Ein Verfahren zum Identifizieren eines therapeutischen Kandidaten für die Behandlung von akuter myeloischer Leukämie, das Folgendes beinhaltet:

i. In-Kontakt-Bringen des therapeutischen Kandidaten mit einer isolierten Probe, die eine Population von Blutzellen enthält, wobei die Population von Blutzellen einen Zelloberflächen-Phänotyp aufweist, der die Folgenden beinhaltet:

a. $CD34^+$;
b. $CD45RA^+$;
c. $CD123^+$; und
d. $CD38^-$;

ii. Inkubieren des therapeutischen Kandidaten mit der isolierten Probe;
iii. In-Kontakt-Bringen der isolierten Probe nach Schritt ii. mit einem Screening-Test, der mindestens die folgenden Zelloberflächen-Polypeptidmarker nachweist: $CD34^+$; $CD45RA^+$; $CD123^+$; und $CD38^-$; wobei (+) das Vorhandensein und (-) das Fehlen der Zelloberflächen-Polypeptidmarker anzeigt;
iv. Identifizieren von Blutzellen durch Schritt iii., die einen Zelloberflächen-Phänotyp aufweisen, der die Folgenden beinhaltet:

a. $CD34^+$;
b. $CD45RA^+$;
c. $CD123^+$; und
d. $CD38^-$;

v. Korrelieren der durch Schritt iv. identifizierten Anzahl von Blutzellen mit der in einer isolierten Probe vor Schritt i. vorhandenen Anzahl von Blutzellen, die einen Zelloberflächen-Phänotyp aufweisen, der die Folgenden beinhaltet:

a. $CD34^+$;
b. $CD45RA^+$;
c. $CD123^+$; und
d. $CD38^-$; und

vi. Bestätigen des Vorhandenseins eines therapeutischen Kandidaten mit einer gegen akute myeloische Leukämie gerichteten Zellaktivität durch Identifizieren einer relativen Abnahme der Anzahl von Blutzellen in Schritt v. nach dem Kontakt mit dem therapeutischen Kandidaten; oder

Bestätigen des Fehlens eines therapeutischen Kandidaten mit einer gegen akute myeloische Leukämie gerichteten Zellaktivität durch Identifizieren keiner signifikanten relativen Abnahme der Anzahl von Blutzellen in Schritt v. nach Kontakt mit dem therapeutischen Kandidaten.

**4.** Ein Verfahren zum Überwachen der Wirksamkeit eines therapeutischen Moleküls bei der Behandlung der akuten myeloischen Leukämie, das Folgendes beinhaltet:

i. In-Kontakt-Bringen einer isolierten Probe von einem Patienten, wobei dem Patienten das therapeutische Molekül verabreicht worden ist, mit einem Screening-Test, der mindestens die folgenden Zelloberflächen-Polypeptidmarker nachweist: $CD34^+$; $CD45RA^+$; $CD123^+$; und $CD38^-$; wobei (+) das Vorhandensein und (-) das Fehlen der Zelloberflächen-Polypeptidmarker anzeigt;
ii. Identifizieren von Blutzellen durch Schritt i., die einen Zelloberflächen-Phänotyp aufweisen, der die Folgenden beinhaltet:

a. $CD34^+$;
b. $CD45RA^+$;

c. $CD123^+$; und
d. $CD38^-$;

iii. Korrelieren der durch Schritt ii. identifizierten Anzahl von Blutzellen mit der Anzahl von Blutzellen, die in einer isolierten Probe vorhanden sind, die einem Patienten vor der Verabreichung des therapeutischen Moleküls entnommen wurde, wobei die vor der Verabreichung des therapeutischen Moleküls entnommenen Blutzellen einen Zelloberflächen-Phänotyp aufweisen, der die Folgenden beinhaltet:

a. $CD34^+$;
b. $CD45RA^+$;
c. $CD123^+$; und
d. $CD38^-$; und

iv. Bestätigen der Wirksamkeit des therapeutischen Moleküls durch Identifizieren einer relativen Abnahme der Anzahl von Blutzellen in Schritt iii. nach Kontakt mit dem therapeutischen Molekül; oder

Bestätigen des Fehlens einer Wirksamkeit des therapeutischen Moleküls durch Identifizieren keiner signifikanten relativen Abnahme der Anzahl von Blutzellen in Schritt iii. nach Kontakt mit dem therapeutischen Molekül.

**5.** Verfahren nach einem der vorhergehenden Ansprüche, wobei der Zelloberflächen-Phänotyp ferner die Folgenden beinhaltet: $CD19^-$, $CD47^{+/-}$, $CCR8^{+/-}$, $RHAMM^{+/-}$ und/oder $CD86^-$.

**6.** Verfahren nach einem der vorhergehenden Ansprüche, wobei das Verfahren die Verwendung von einem oder mehreren Antikörpern beinhaltet, die an einen oder mehrere Zelloberflächen-Polypeptidmarker binden, die aus den Folgenden ausgewählt sind: CD34, CD45RA, CD123, CD38, CD47, CD19, CCR8, RHAMM und CD86.

**7.** Verfahren nach einem der vorhergehenden Ansprüche, wobei die Zelloberflächen-Polypeptidmarker durch Antikörper nachgewiesen werden, wobei die Antikörper die Folgenden beinhalten:

einen ersten Antikörper, der an CD34 bindet; und
einen zweiten Antikörper, der an CD45RA bindet; und
einen dritten Antikörper, der an CD123 bindet; und
einen vierten Antikörper, der an CD38 bindet.

**Revendications**

**1.** Procédé de diagnostic d'une leucémie myéloïde aiguë, comprenant :

i. la mise en contact d'un échantillon isolé contenant une population de cellules sanguines avec un écran qui détecte au moins les marqueurs polypeptidiques de surface cellulaire suivants : $CD34^+$ ; $CD45RA^+$ ; $CD123^+$ ; et $CD38^-$ ; (+) indiquant la présence et (-) indiquant l'absence desdits marqueurs polypeptidiques de surface cellulaire ; et
ii. la confirmation de la présence d'une cellule sanguine ayant un phénotype de surface cellulaire comprenant :

a. $CD34^+$ ;
b. $CD45RA^+$ ;
c. $CD123^+$ ; et
d. $CD38^-$ ;

dans lequel une leucémie myéloïde aiguë est diagnostiquée lorsque la présence de ladite cellule sanguine est confirmée.

**2.** Procédé d'établissement d'un pronostic de leucémie myéloïde aiguë, comprenant :

i. la mise en contact d'un échantillon isolé contenant une population de cellules sanguines avec un écran qui détecte au moins les marqueurs polypeptidiques de surface cellulaire suivants : $CD34^+$ ; $CD45RA^+$ ; $CD123^+$ ; et $CD38^-$ ; (+) indiquant la présence et (-) indiquant l'absence desdits marqueurs polypeptidiques de surface cellulaire ; et
ii. la confirmation de la présence d'une cellule sanguine ayant un phénotype de surface cellulaire comprenant :

a. $CD34^+$ ;
b. $CD45RA^+$ ;
c. $CD123^+$ ; et
d. $CD38^-$ ;

dans lequel le pronostic de leucémie myéloïde aiguë est établi lorsque la présence de ladite cellule sanguine est confirmée.

**3.** Procédé d'identification d'un candidat thérapeutique pour le traitement d'une leucémie myéloïde aiguë, comprenant :

i. la mise en contact du candidat thérapeutique avec un échantillon isolé contenant une population de cellules sanguines, ladite population de cellules sanguines ayant un phénotype de surface cellulaire comprenant :

a. $CD34^+$ ;
b. $CD45RA^+$ ;
c. $CD123^+$ ; et
d. $CD38^-$ ;

ii. la mise en incubation dudit candidat thérapeutique avec ledit échantillon isolé ;
iii. la mise en contact dudit échantillon isolé après l'étape ii. avec un écran qui détecte au moins les marqueurs polypeptidiques de surface cellulaire suivants : $CD34^+$ ; $CD45RA^+$ ; $CD123^+$ ; et $CD38^-$ ; (+) indiquant la présence et (-) indiquant l'absence desdits marqueurs polypeptidiques de surface cellulaire ;
iv. l'identification des cellules sanguines à l'étape iii. qui ont un phénotype de surface cellulaire comprenant :

a. $CD34^+$ ;
b. $CD45RA^+$ ;
c. $CD123^+$ ; et
d. $CD38^-$ ;

v. la mise en corrélation du nombre de cellules sanguines identifiées à l'étape iv. avec le nombre de cellules sanguines présentes dans un échantillon isolé avant l'étape i. qui ont un phénotype de surface cellulaire comprenant :

a. $CD34^+$ ;
b. $CD45RA^+$ ;
c. $CD123^+$ ; et
d. $CD38^-$ ; et

vi. la confirmation de la présence d'un candidat thérapeutique ayant une activité cellulaire anti-leucémie myéloïde aiguë en identifiant une diminution relative du nombre de cellules sanguines à l'étape v. après la mise en contact avec le candidat thérapeutique ; ou

la confirmation de l'absence de candidat thérapeutique ayant une activité cellulaire anti-leucémie myéloïde aiguë en n'identifiant aucune diminution relative significative du nombre de cellules sanguines à l'étape v. après la mise en contact avec le candidat thérapeutique.

**4.** Procédé de contrôle de l'efficacité d'une molécule thérapeutique dans le traitement d'une leucémie myéloïde, aiguë comprenant :

i. la mise en contact d'un échantillon isolé provenant d'un patient, la molécule thérapeutique ayant été administrée audit patient, avec un écran qui détecte au moins les marqueurs polypeptidiques de surface cellulaire suivants : $CD34^+$ ; $CD45RA^+$ ; $CD123^+$ ; et $CD38^-$ ; (+) indiquant la présence et (-) indiquant l'absence desdits marqueurs polypeptidiques de surface cellulaire ;
ii. l'identification des cellules sanguines à l'étape i. qui ont un phénotype de surface cellulaire comprenant :

a. $CD34^+$ ;
b. $CD45RA^+$ ;
c. $CD123^+$ ; et
d. $CD38^-$ ;

iii. la mise en corrélation du nombre de cellules sanguines identifiées à l'étape ii. avec le nombre de cellules sanguines présentes dans un échantillon isolé prélevé sur un patient avant l'administration de la molécule thérapeutique, lesdites cellules sanguines prélevées avant l'administration de la molécule thérapeutique ayant un phénotype de surface cellulaire comprenant :

a. $CD34^+$ ;
b. $CD45RA^+$ ;
c. $CD123^+$ ; et
d. $CD38^-$ ; et

iv. la confirmation de l'efficacité de la molécule thérapeutique en identifiant une diminution relative du nombre de cellules sanguines à l'étape iii. après la mise en contact avec la molécule thérapeutique ; ou

la confirmation de l'absence d'efficacité de la molécule thérapeutique en n'identifiant aucune diminution relative significative du nombre de cellules sanguines à l'étape iii. après la mise en contact avec la molécule thérapeutique.

**5.** Procédé selon l'une quelconque des revendications précédentes, dans lequel le phénotype de surface cellulaire comprend en outre : $CD19^-$, $CD47^{+/-}$, $CCR8^{+/-}$, $RHAMM^{+/-}$, et/ou $CD86^-$.

**6.** Procédé selon l'une quelconque des revendications précédentes, le procédé comprenant l'utilisation d'un ou plusieurs anticorps qui se lient à un ou plusieurs marqueurs polypeptidiques de surface cellulaire sélectionnés parmi : CD34, CD45RA, CD123, CD38, CD47, CD19, CCR8, RHAMM et CD86.

**7.** Procédé selon l'une quelconque des revendications précédentes, dans lequel les marqueurs polypeptidiques de surface cellulaire sont détectés par des anticorps, lesdits anticorps comprenant :

un premier anticorps qui se lie à CD34 ; et
un deuxième anticorps qui se lie à CD45RA ; et
un troisième anticorps qui se lie à CD123 ; et
un quatrième anticorps qui se lie à CD38.

*Fig. 1*

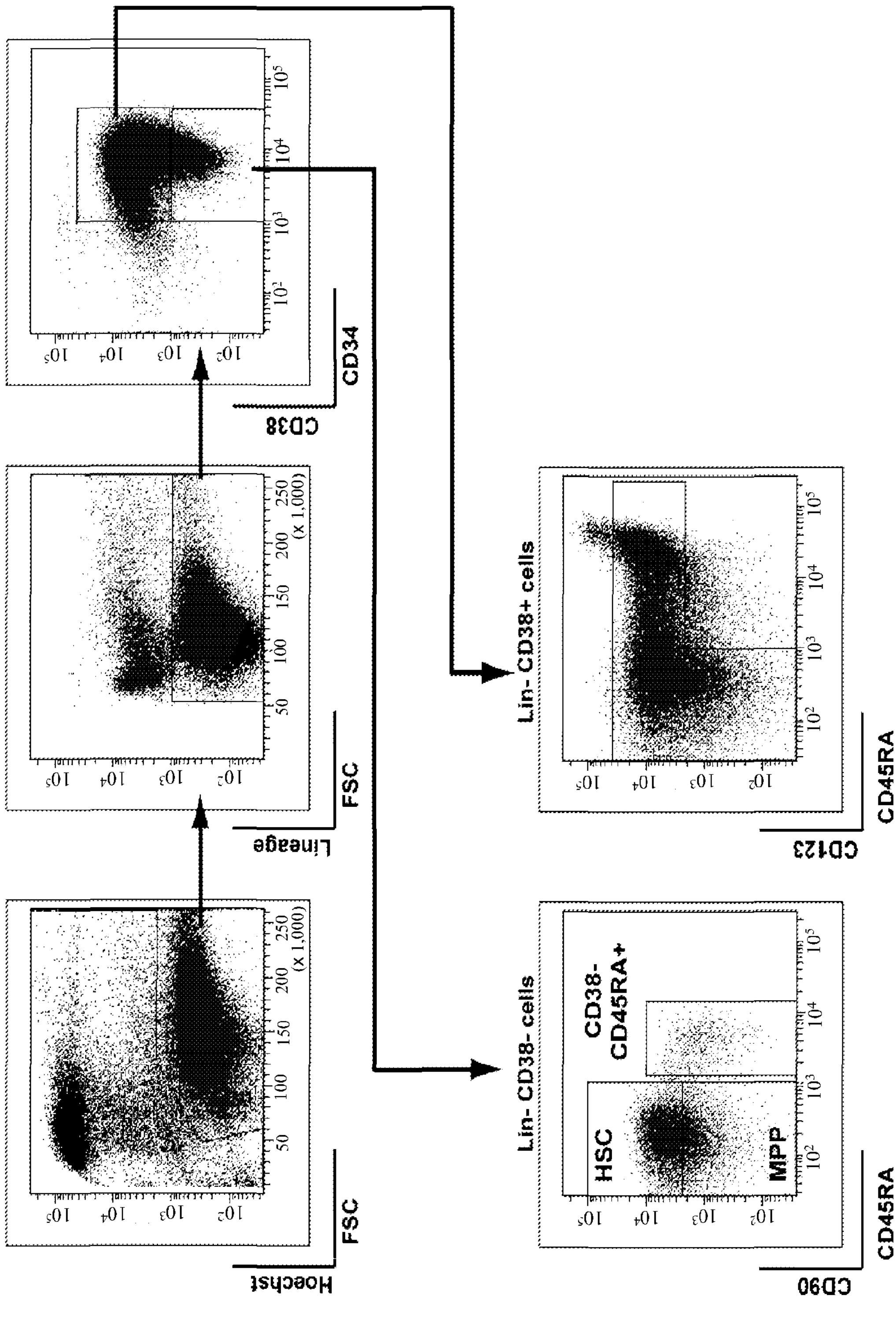
Hoechst
FSC
Lineage
FSC
Lin- cells
CD38
CD34
Lin- CD38- cells
CD38-
CD45RA+
HSC
MPP
CD90
CD45RA
Lin- CD38+ cells
CD123
CD45RA

*Fig. 2*

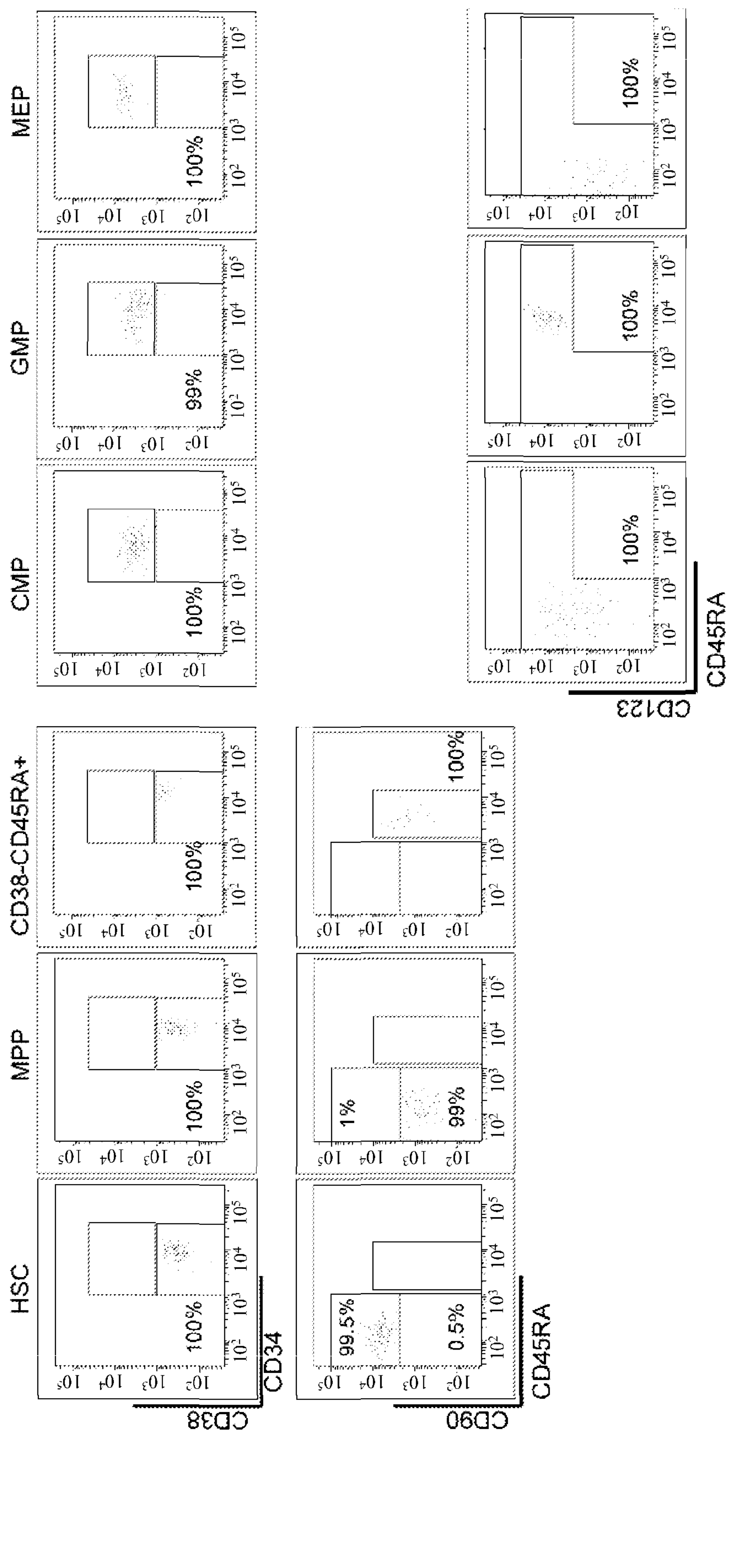
HSC
MPP
CD38-CD45RA+
CMP
GMP
MEP
CD38
CD34
CD90
CD45RA
CD123
100%
99.5%
0.5%
1%
99%

*Fig. 3*

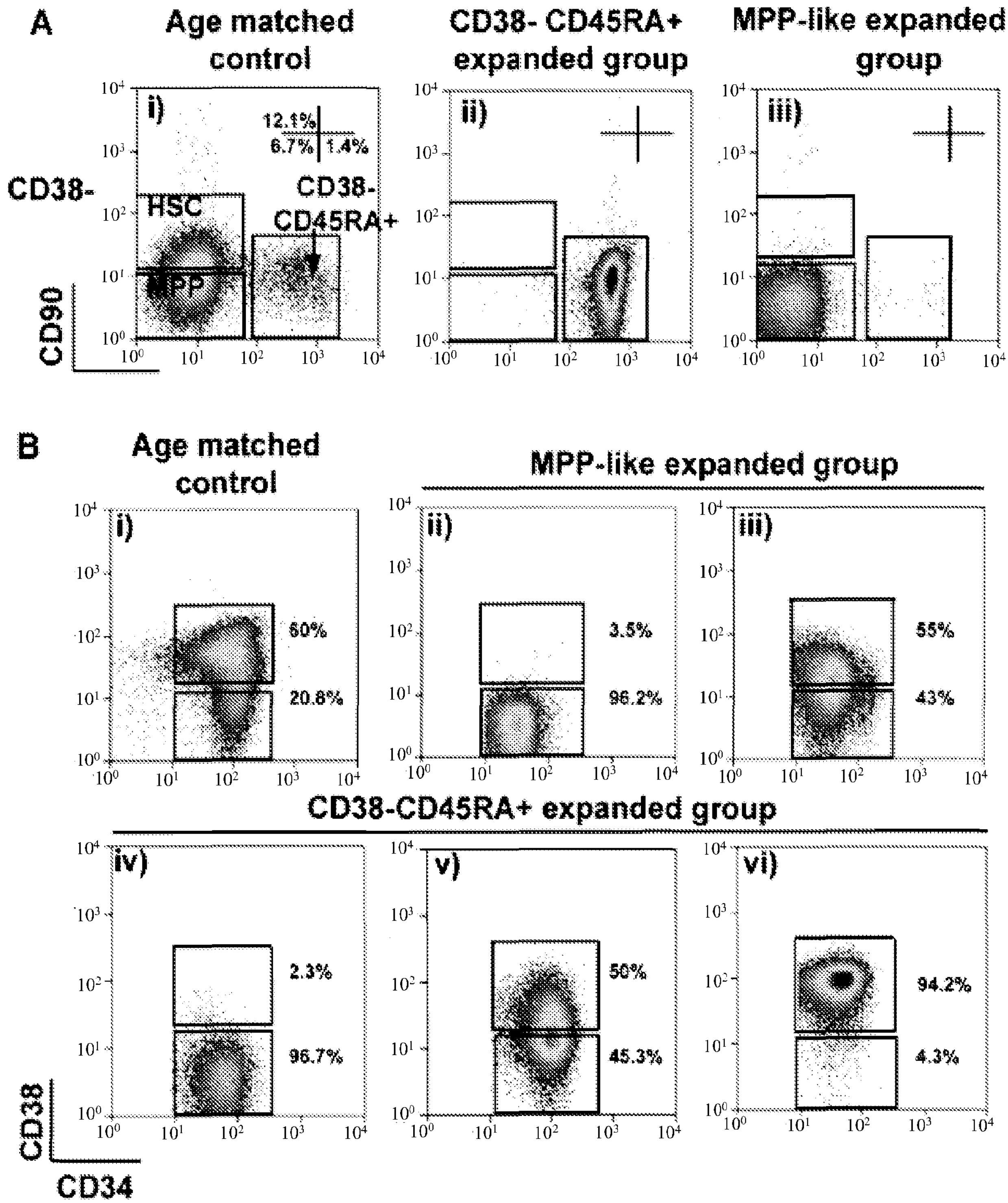
A
Age matched control
CD38- CD45RA+ expanded group
MPP-like expanded group
i)
ii)
iii)
12.1%
6.7%
1.4%
CD38-
HSC
MPP
CD38-
CD45RA+
CD90
B
Age matched control
MPP-like expanded group
CD38-CD45RA+ expanded group
i)
ii)
iii)
iv)
v)
vi)
60%
20.8%
3.5%
96.2%
55%
43%
2.3%
96.7%
50%
45.3%
94.2%
4.3%
CD38
CD34

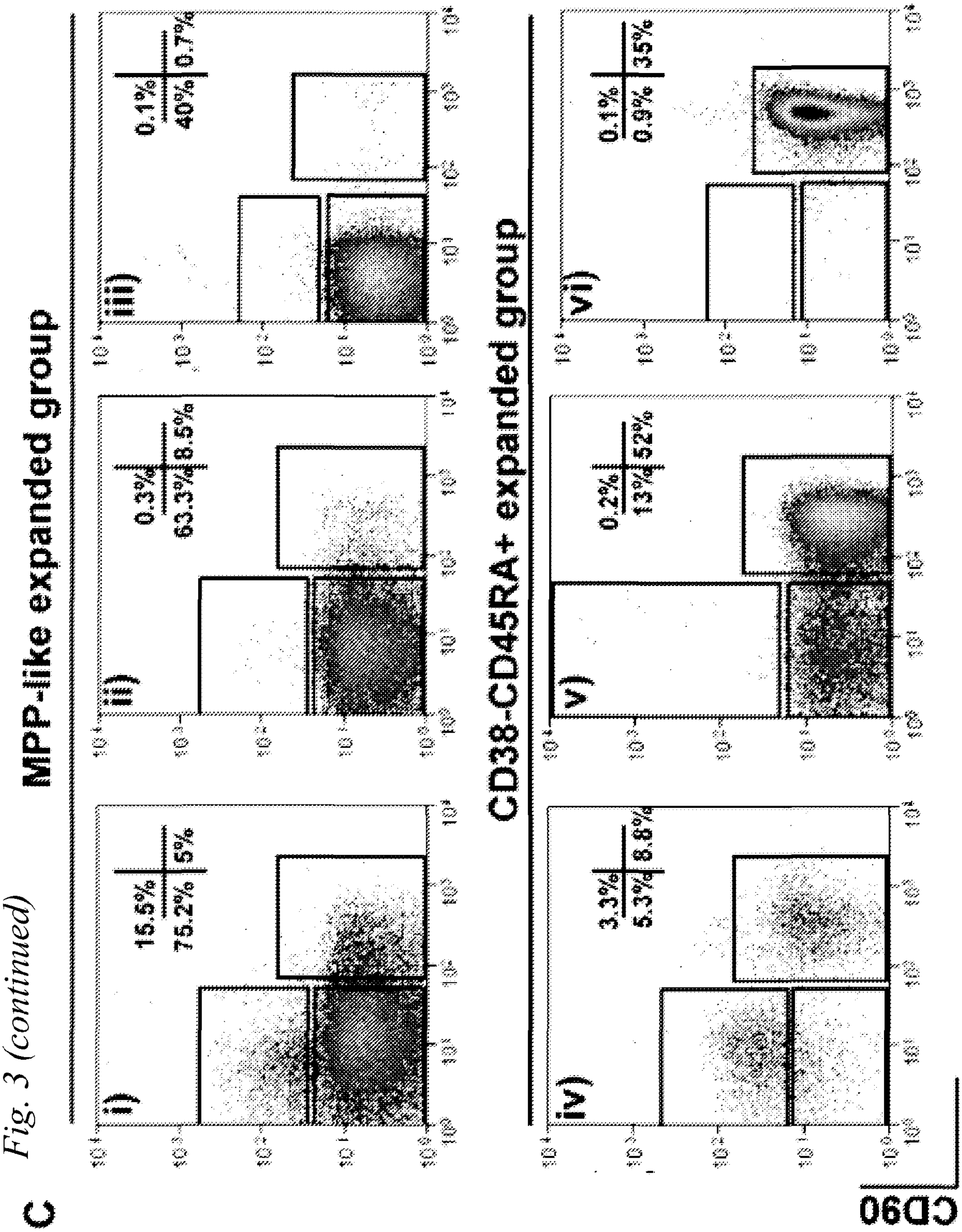


Fig. 3 (continued)

*Fig. 4*

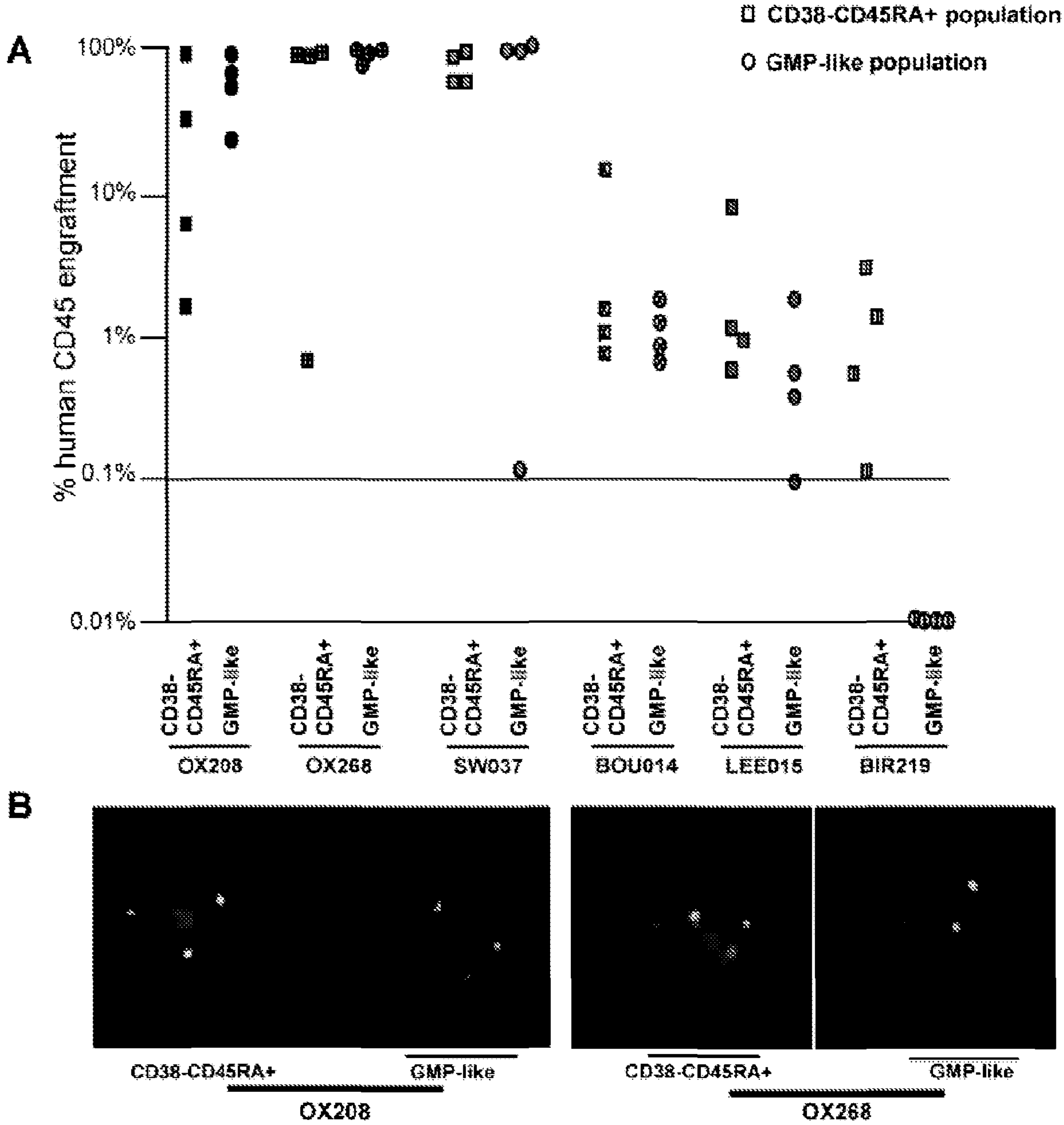
A
CD38-CD45RA+ population
GMP-like population
% human CD45 engraftment
100%
10%
1%
0.1%
0.01%
CD38-CD45RA+
GMP-like
OX208
OX268
SW037
BOU014
LEE015
BIR219
B
CD38-CD45RA+
GMP-like
OX208
CD38-CD45RA+
GMP-like
OX268

*Fig. 4 (continued)*

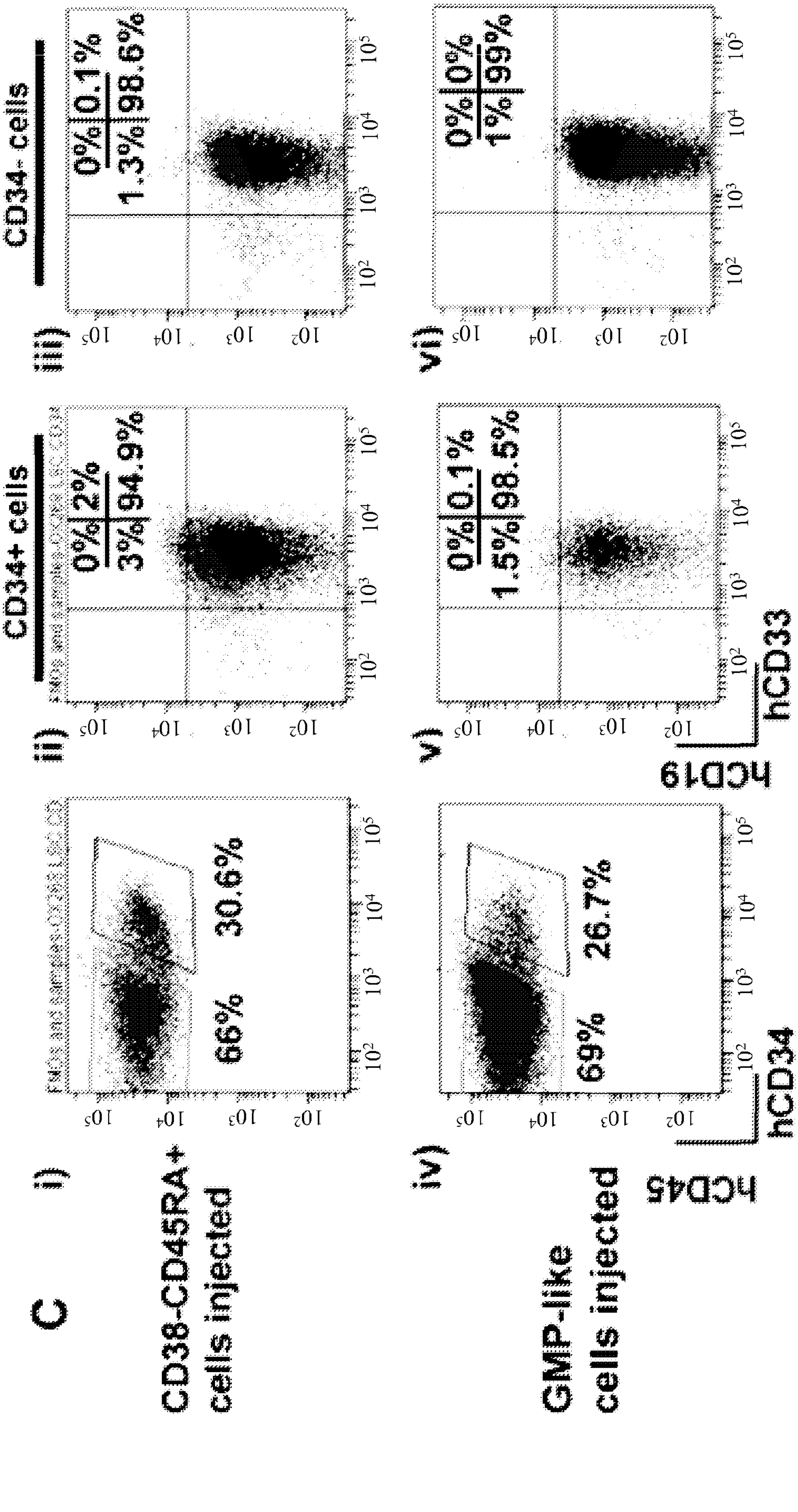

*Fig. 4 (continued)*

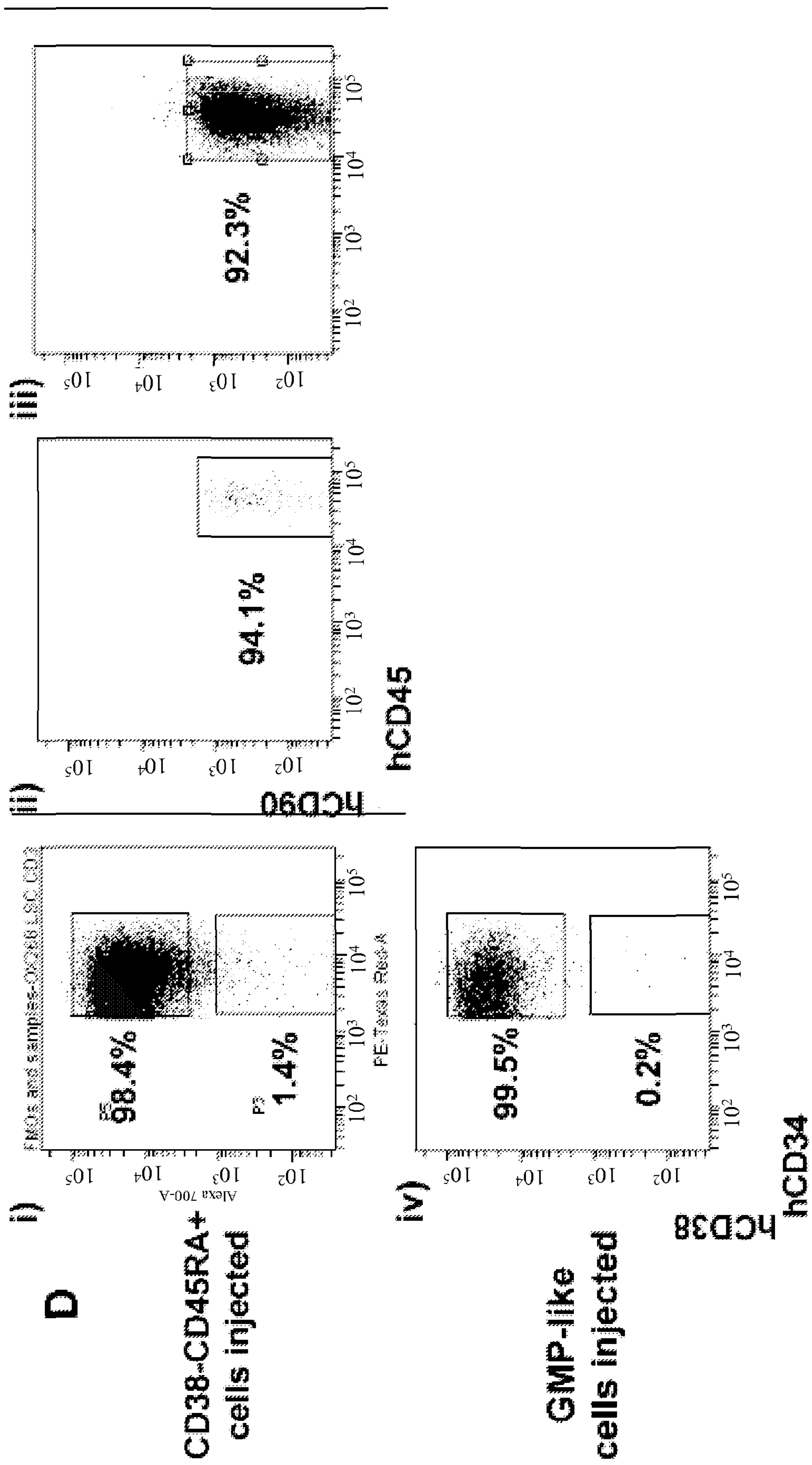

*Fig. 4 (continued)*

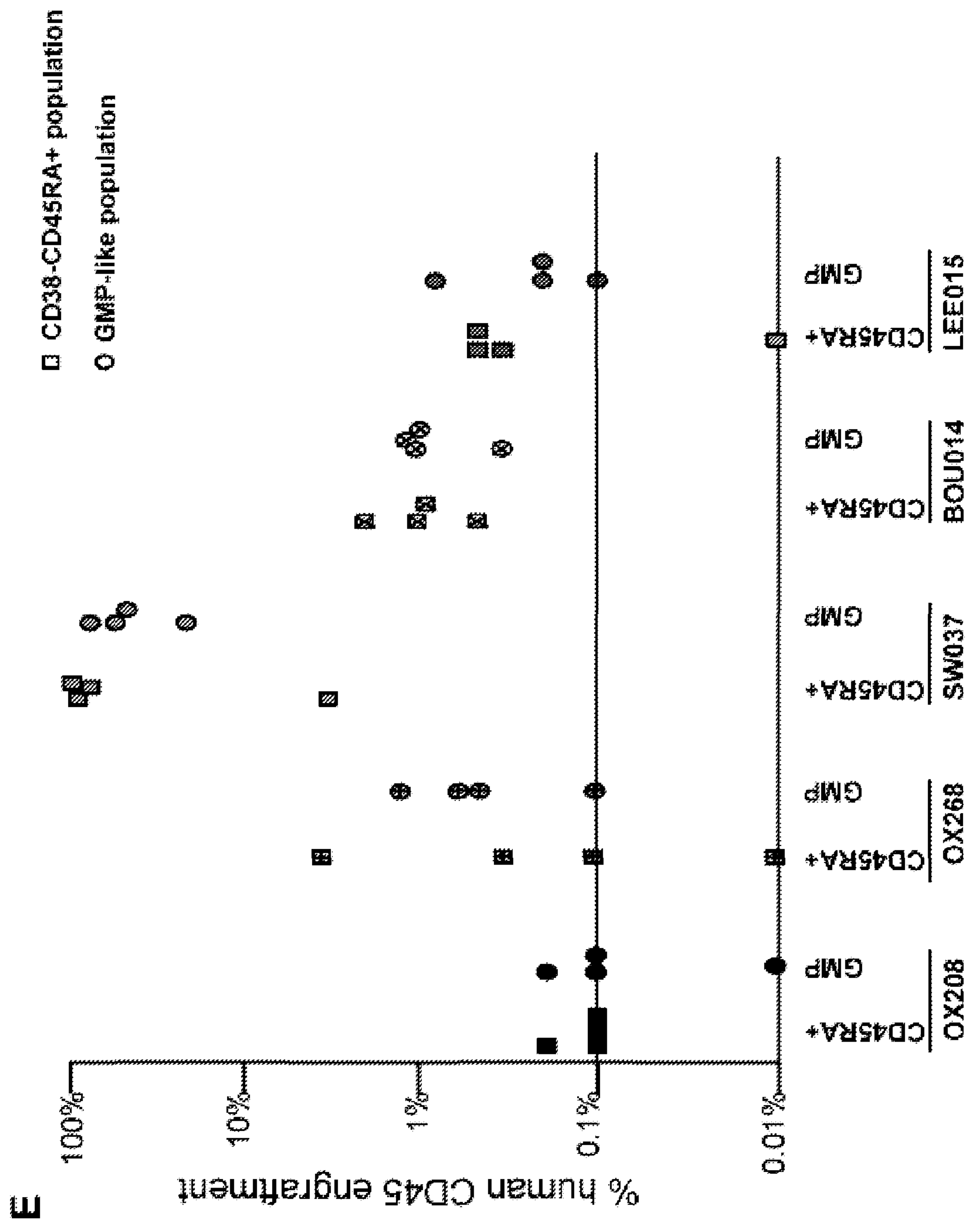

*Fig. 5*

A

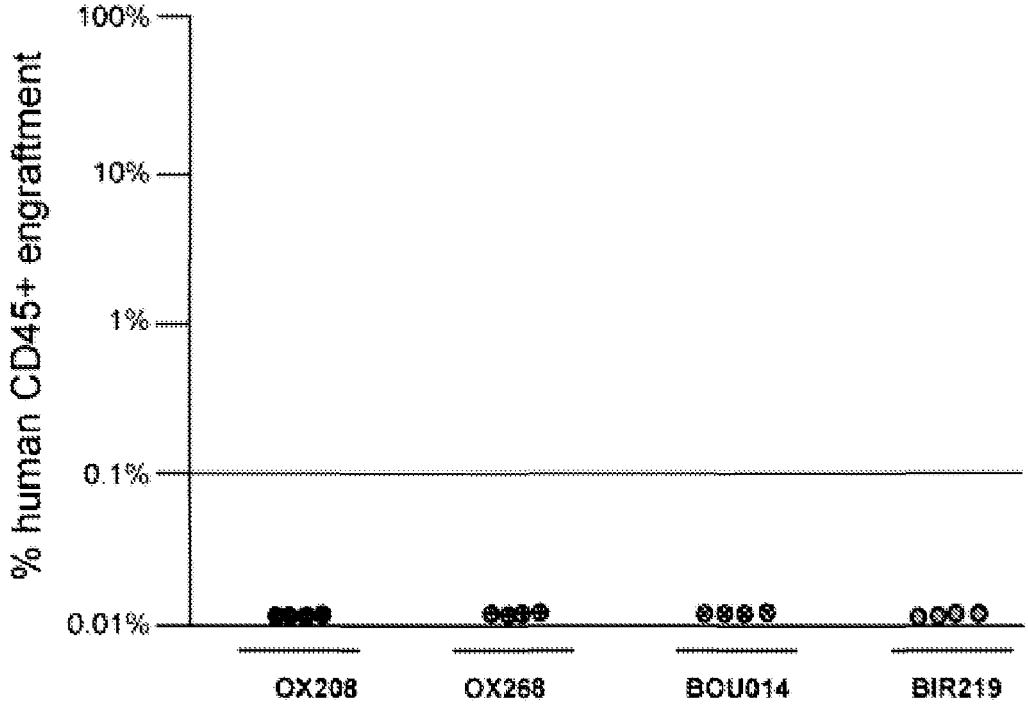
100%
10%
1%
0.1%
0.01%
% human CD45+ engraftment
OX208
OX268
BOU014
BIR219

D

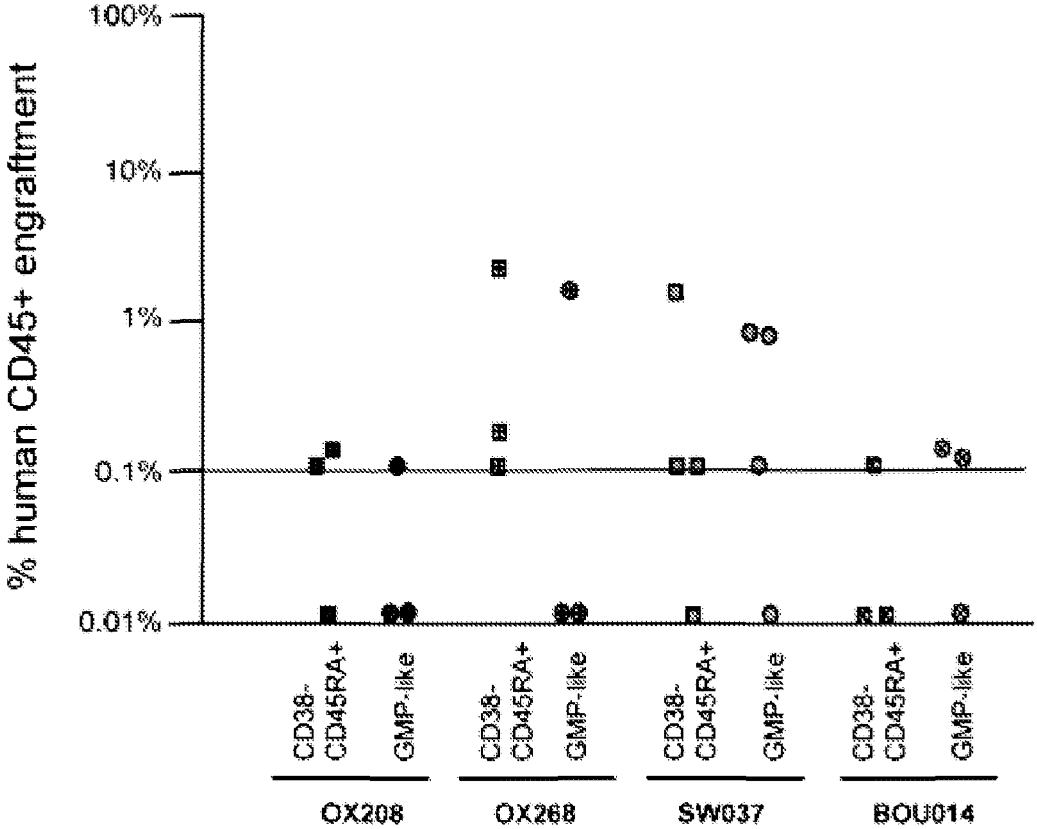
100%
10%
1%
0.1%
0.01%
% human CD45+ engraftment
CD38-CD45RA+
GMP-like
CD38-CD45RA+
GMP-like
CD38-CD45RA+
GMP-like
CD38-CD45RA+
GMP-like
OX208
OX268
SW037
BOU014

*Fig. 5 (continued)*

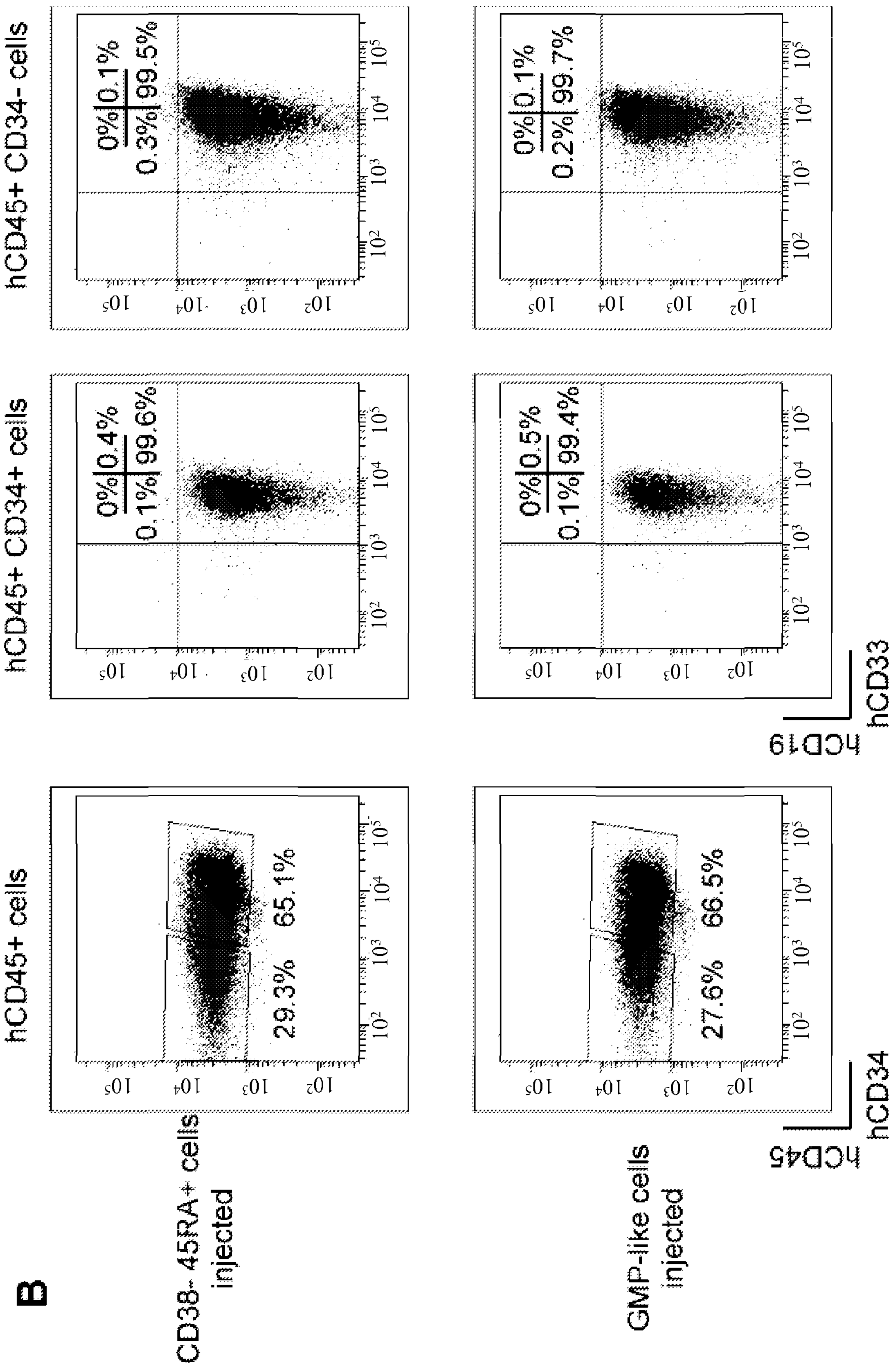

*Fig. 5 (continued)*

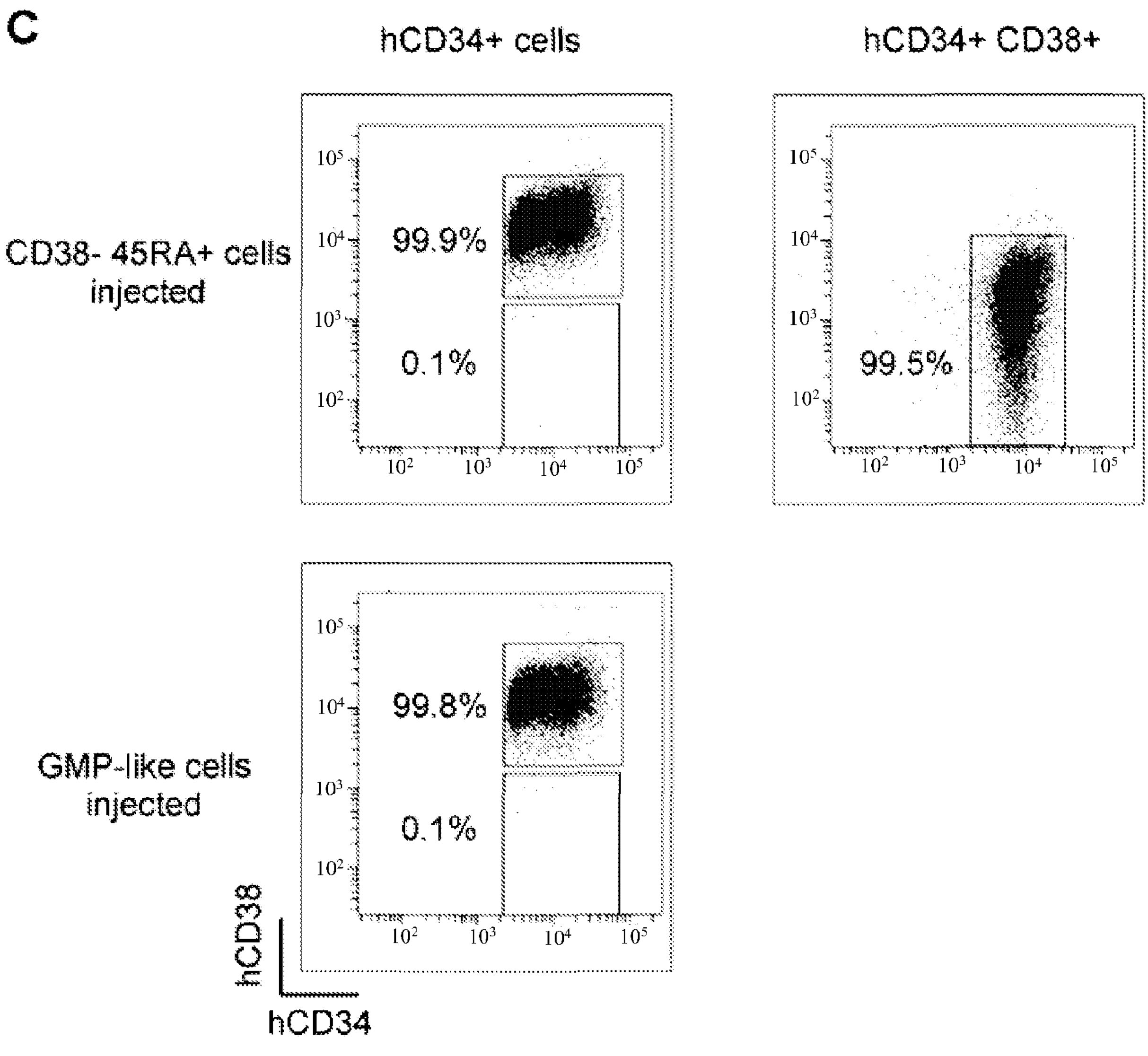

*Fig. 6*

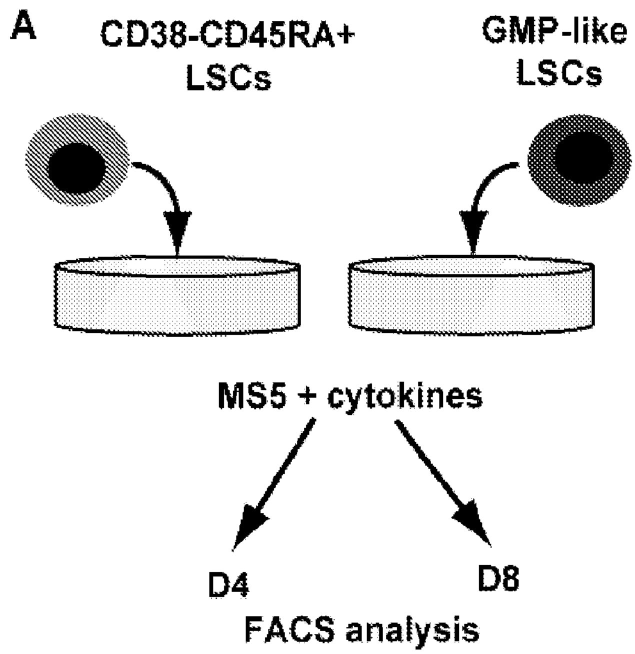
A
CD38-CD45RA+
LSCs
GMP-like
LSCs
MS5 + cytokines
D4
D8
FACS analysis

*Fig. 6 (continued)*

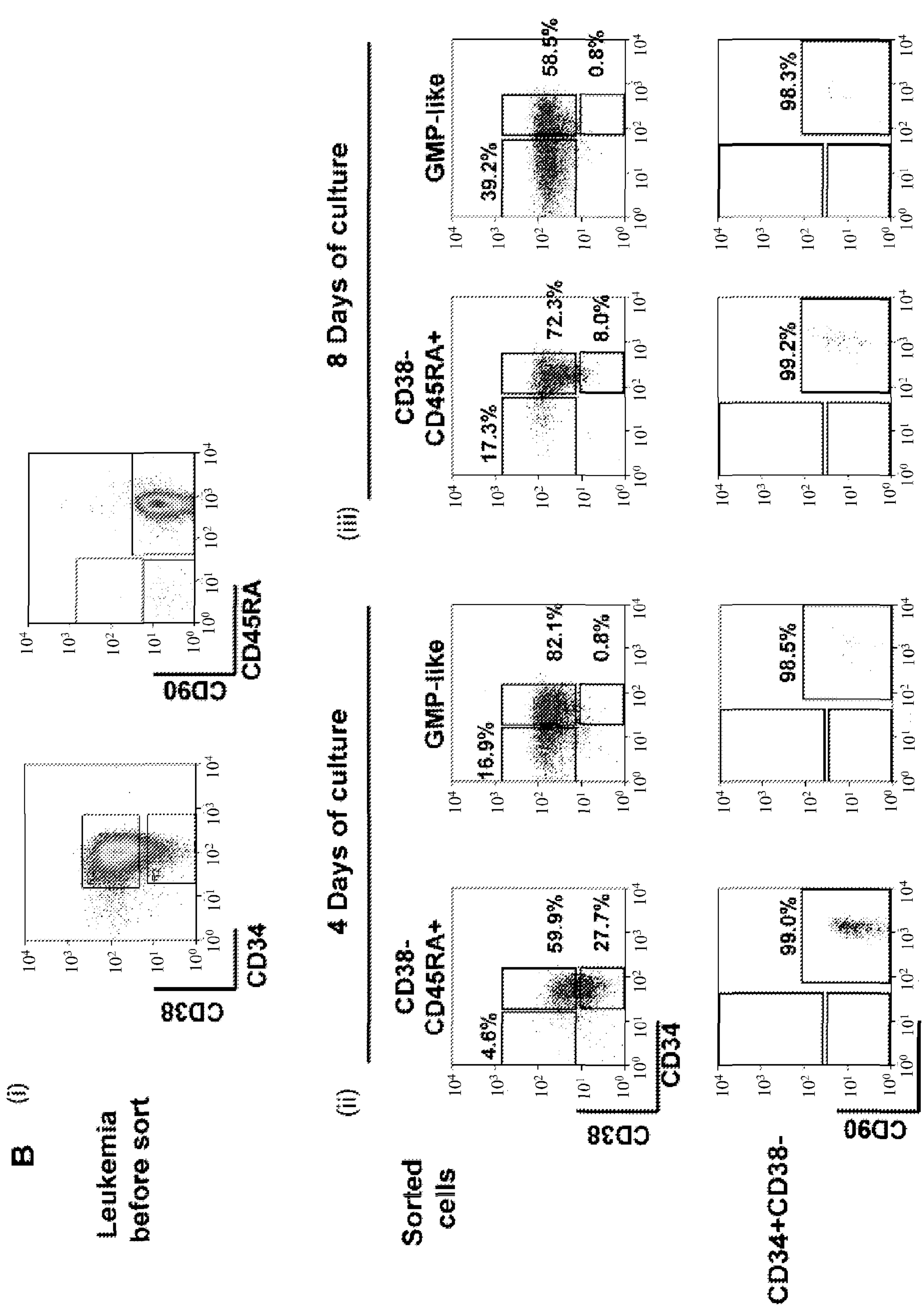

*Fig. 7*

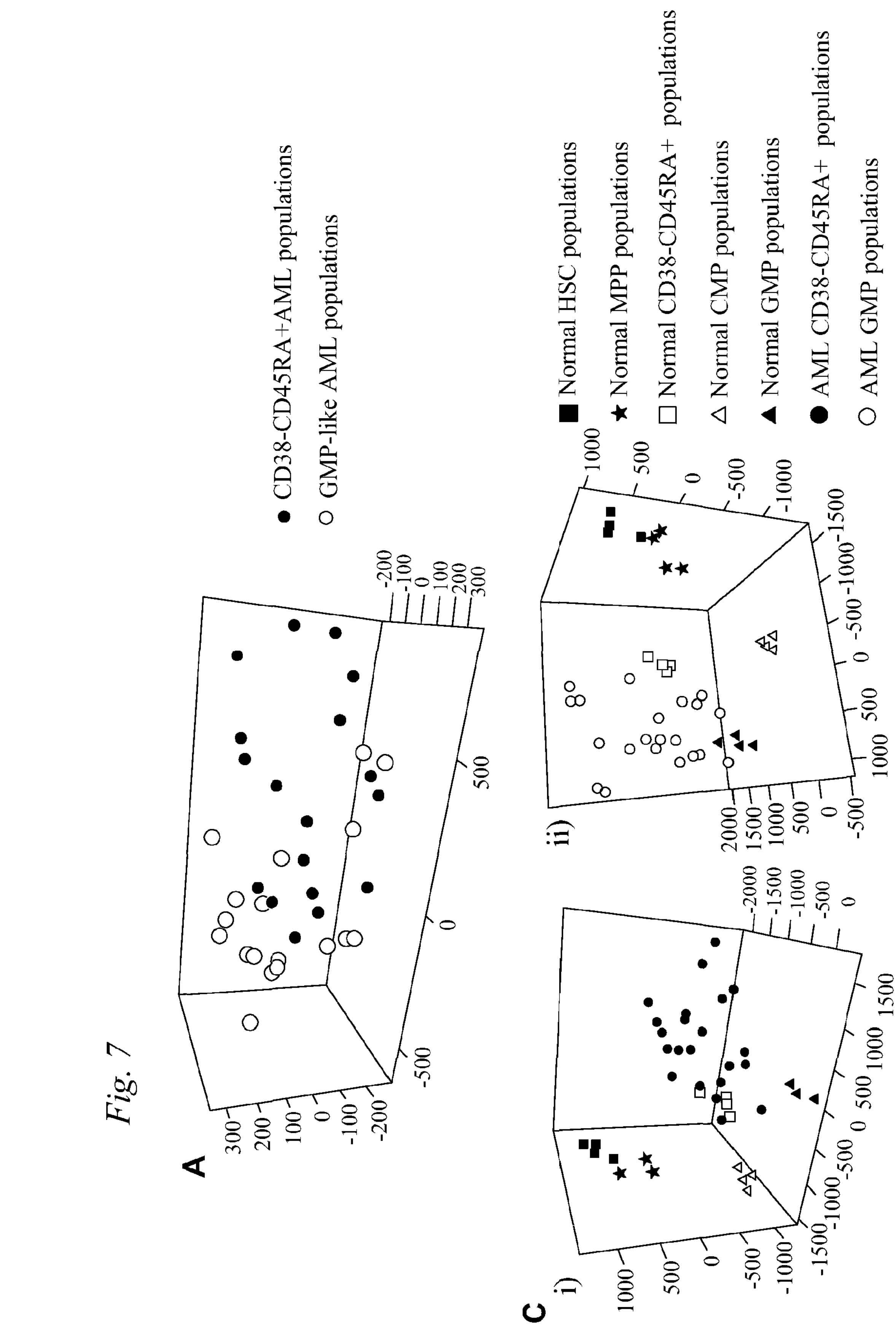
A
● CD38-CD45RA+AML populations
○ GMP-like AML populations
C
i)
ii)
■ Normal HSC populations
★ Normal MPP populations
□ Normal CD38-CD45RA+ populations
△ Normal CMP populations
▲ Normal GMP populations
● AML CD38-CD45RA+ populations
○ AML GMP populations

*Fig. 7 (continued)*

**D**

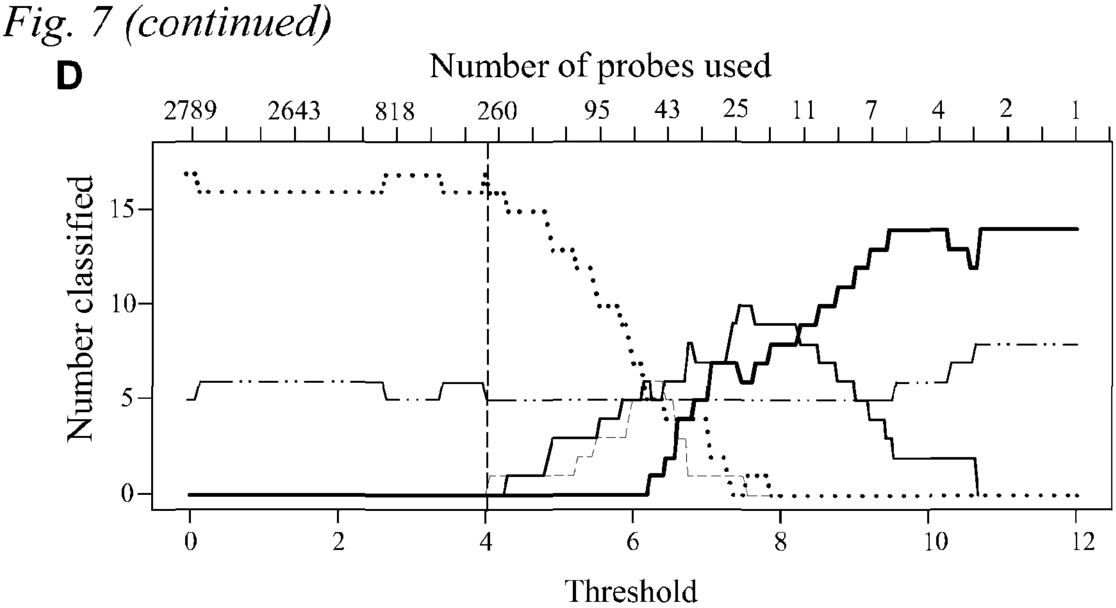


Predicted nearest normal populations for AML CD38-CD45RA+ (22 samples)

**F**

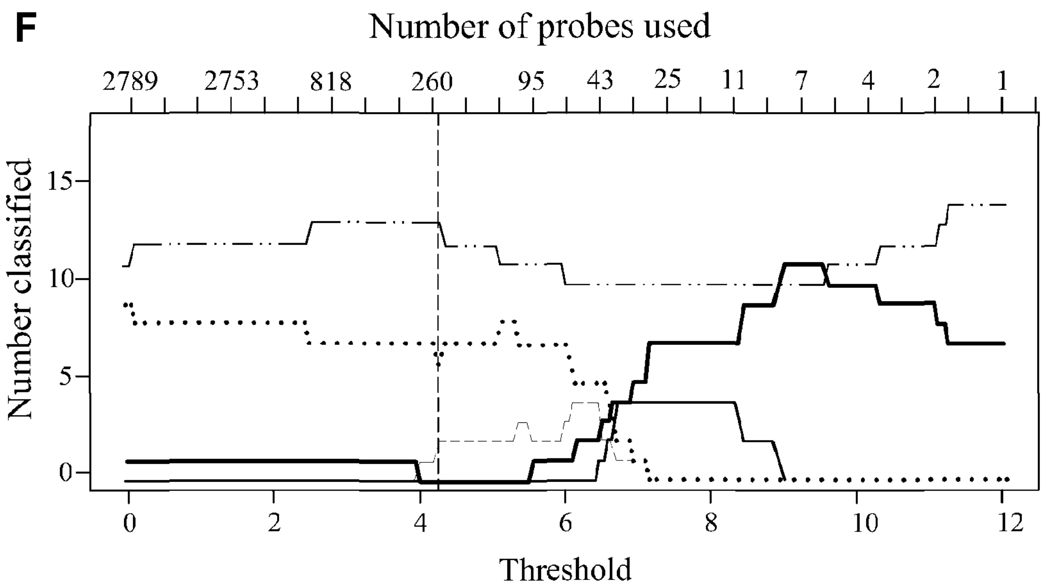


Predicted nearest normal populations for AML GMP-like (21 samples)

*Fig. 7 (continued)*

**B** i) ii)

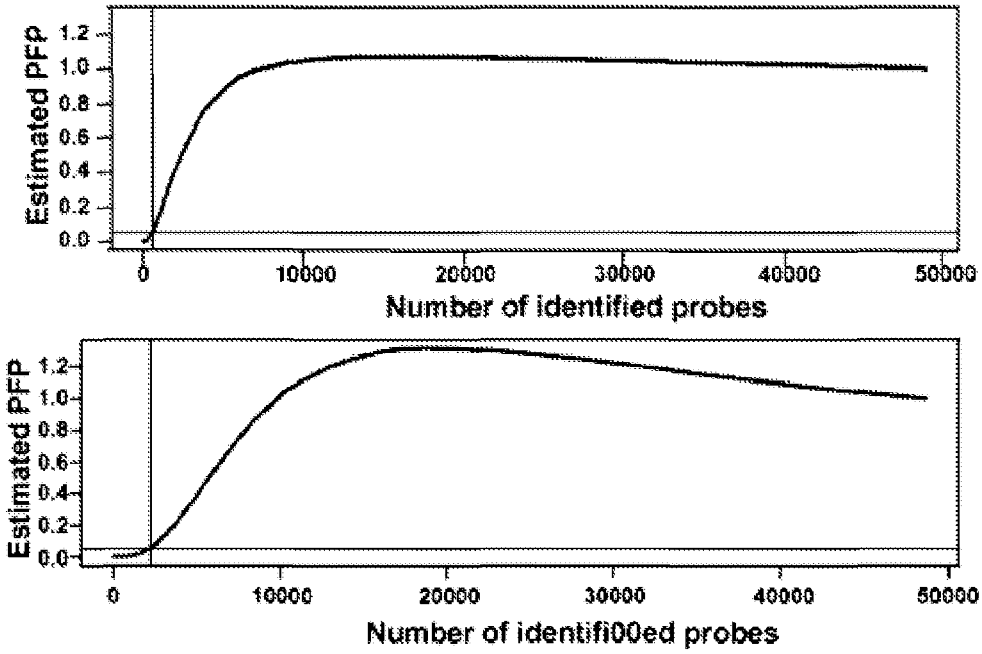


**E**

Predicted nearest normal populations for AML CD38-CD45RA+ (22 samples)

| | HSC | MPP | CD38-CD45RA+ | CMP | GMP | % recognised as normal CD38-CD45RA+ |
|---|---|---|---|---|---|---|
| Thr=5.4 (97probes) | 3 | 2 | 12 | 0 | 5 | 54% |
| Thr=4.0 (278 probes) | 0 | 0 | 17 | 0 | 5 | 77% |
| Thr=3.0 (661 probes) | 0 | 0 | 17 | 0 | 5 | 77% |
| Thr=0 (2789 probes) | 0 | 0 | 17 | 0 | 5 | 77% |

**G**

| | HSC | MPP | CD38-CD45RA+ | CMP | GMP | % recognised as normal GMP |
|---|---|---|---|---|---|---|
| Thr=5.4 (97 probes) | 0 | 3 | 7 | 0 | 11 | 52% |
| Thr=4.27 (241 probes) | 0 | 2 | 6 | 0 | 13 | 62% |
| Thr=3.0 (661 probes) | 0 | 0 | 7 | 1 | 13 | 62% |
| Thr=0 (2789 probes) | 0 | 0 | 9 | 1 | 11 | 52% |

*Fig. 8*

Figure S4: Analysis of normal stem/progenitor and AML LSC global gene expression profiles, related to Figure 4.

**A)**

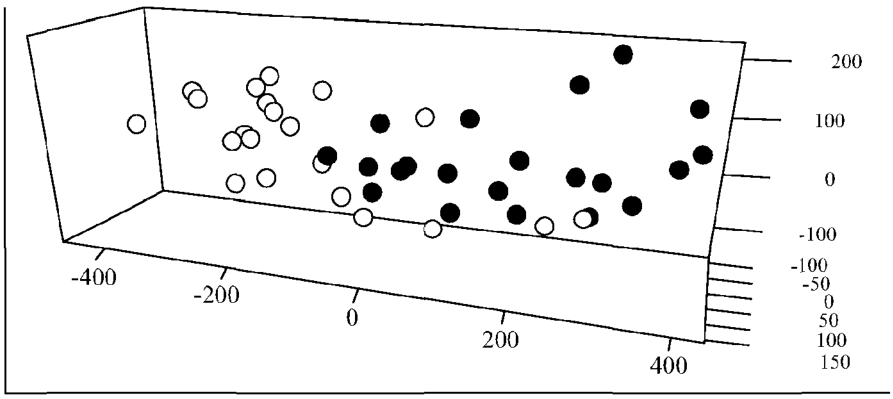


● CD38-CD45RA+AML populations

○ GMP-like AML populations

*Fig. 8 (continued)*

**B)**

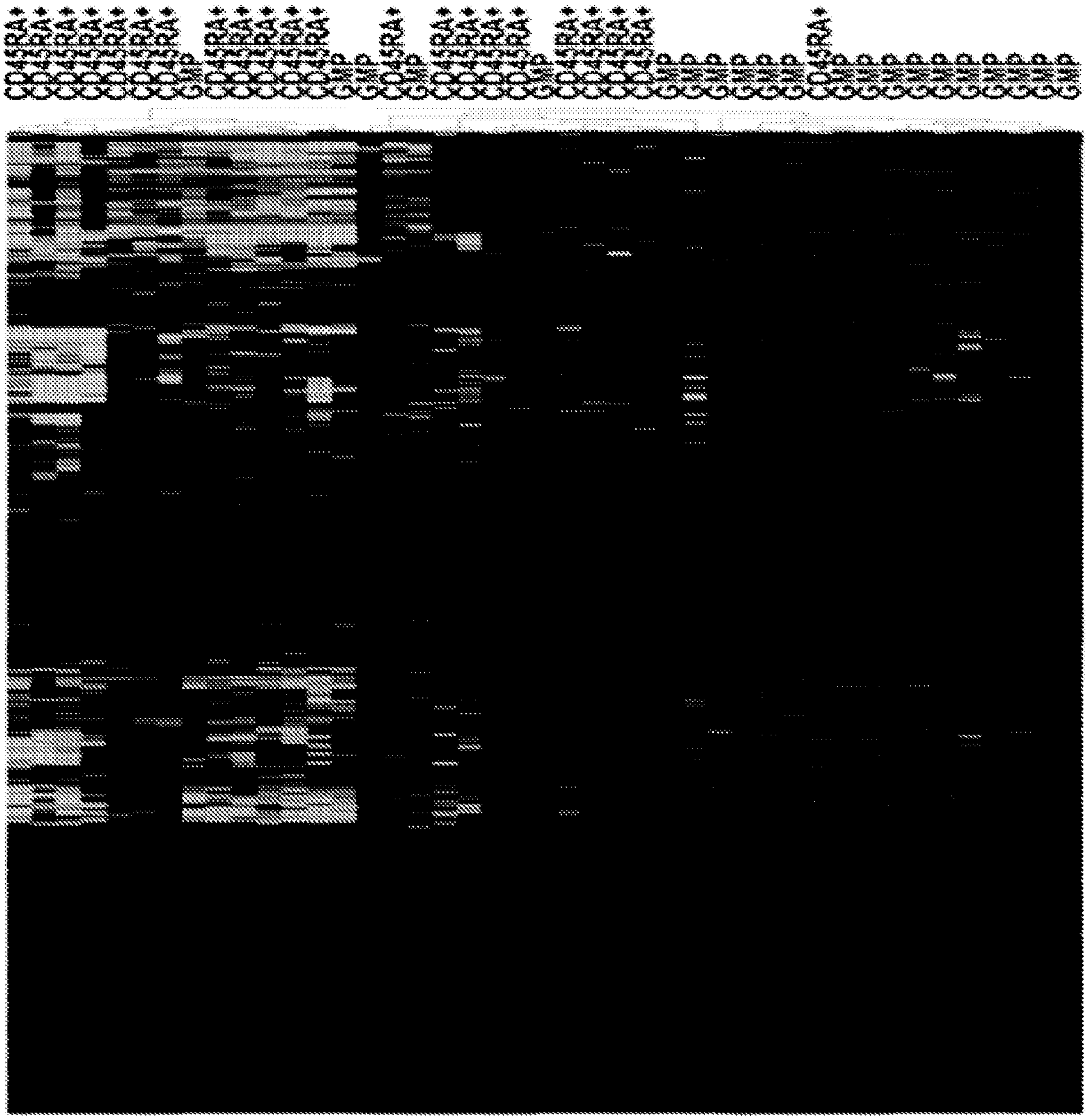

*Fig. 8 (continued)*

C)

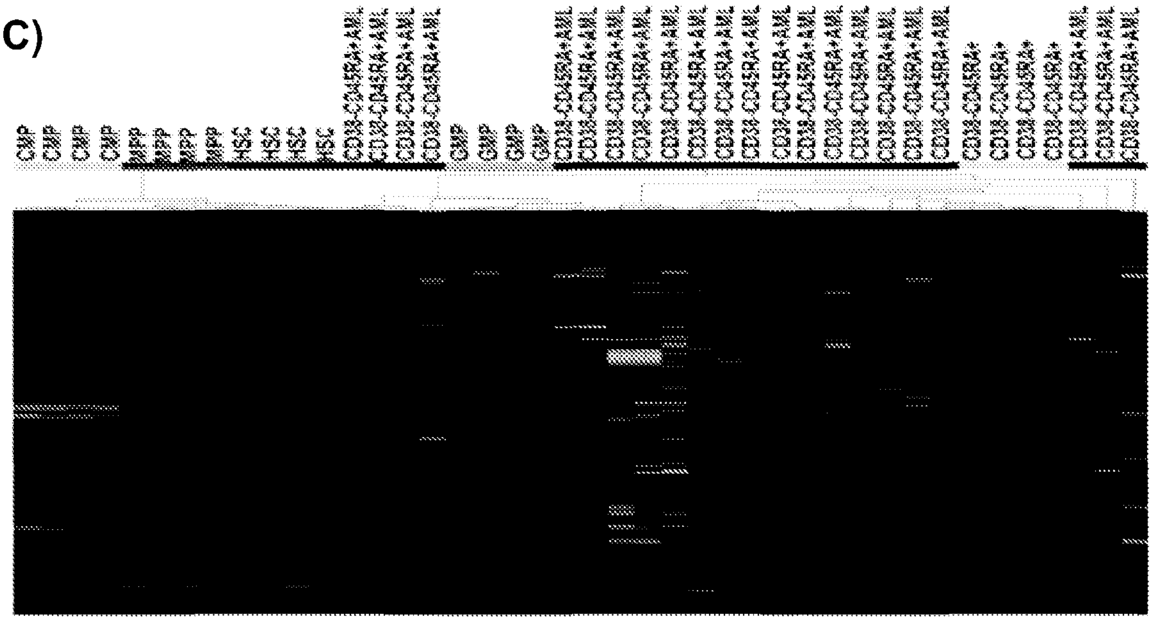

D)

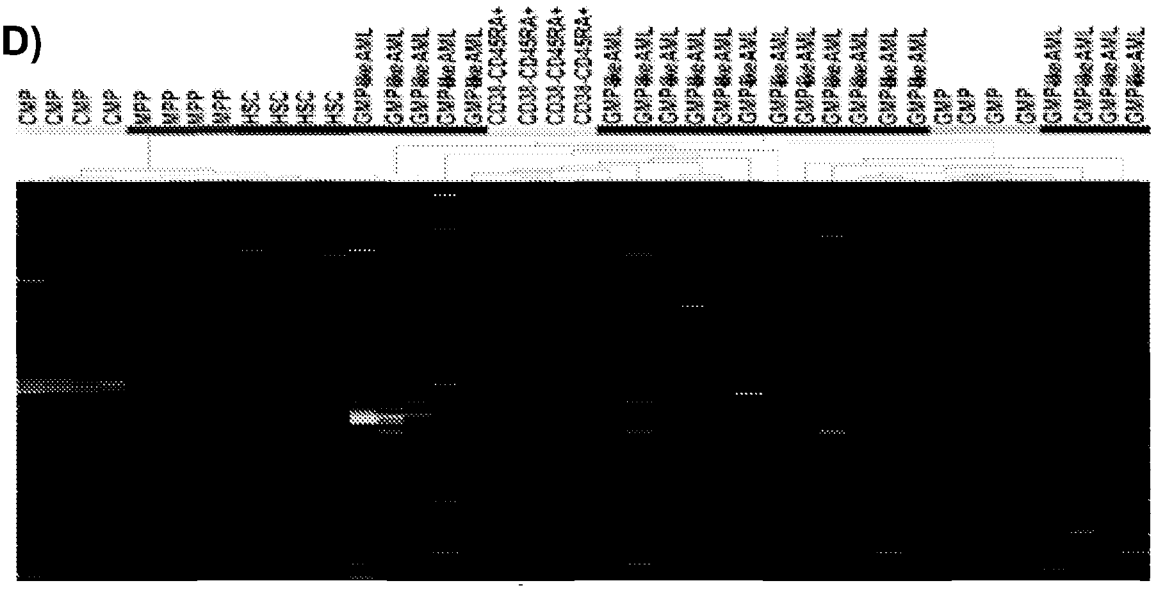

*Fig. 9*

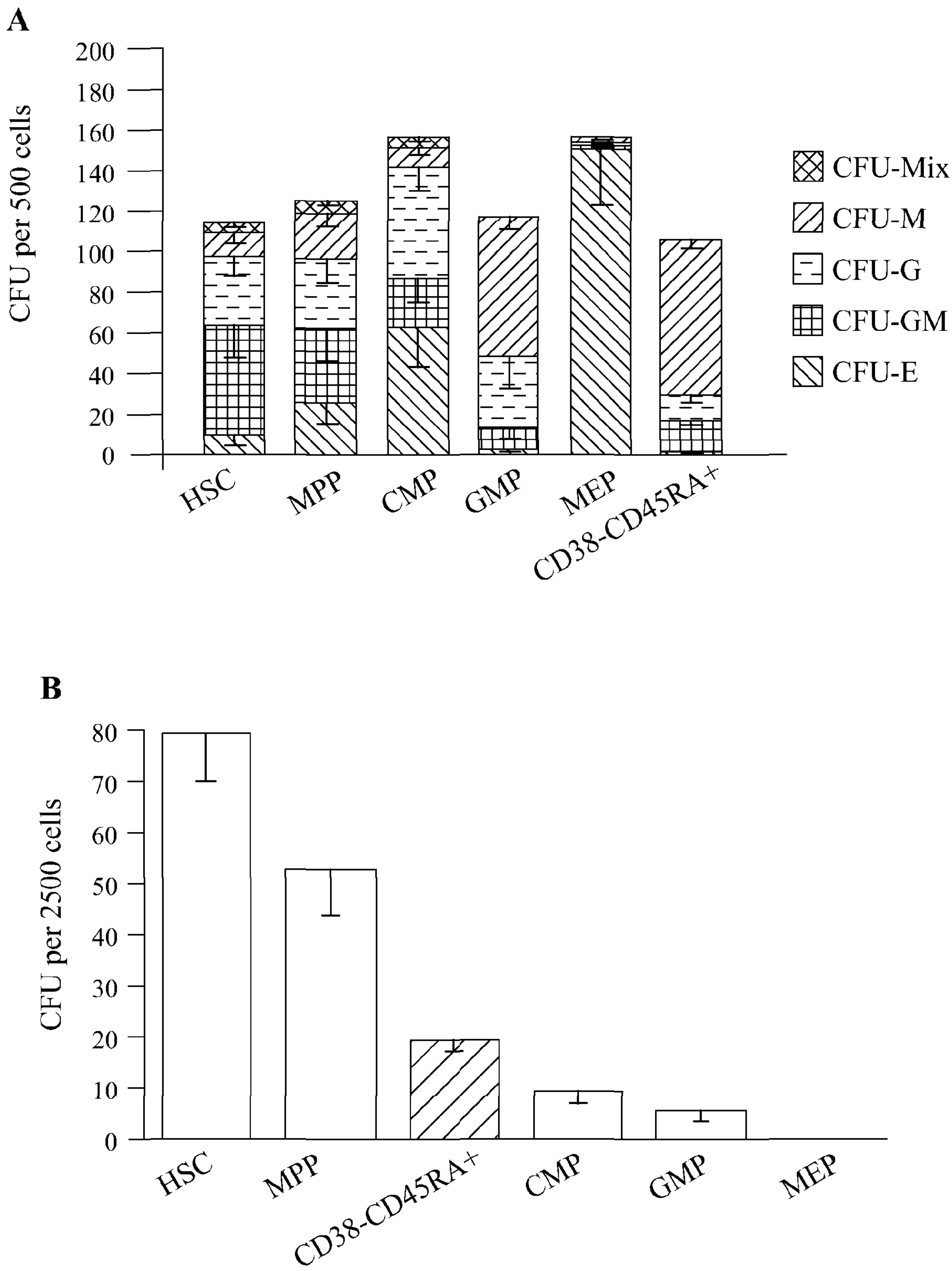
A
CFU per 500 cells
0
20
40
60
80
100
120
140
160
180
200
HSC
MPP
CMP
GMP
MEP
CD38-CD45RA+
CFU-Mix
CFU-M
CFU-G
CFU-GM
CFU-E
B
CFU per 2500 cells
0
10
20
30
40
50
60
70
80
HSC
MPP
CD38-CD45RA+
CMP
GMP
MEP

*Fig. 9 (continued)*

**C**

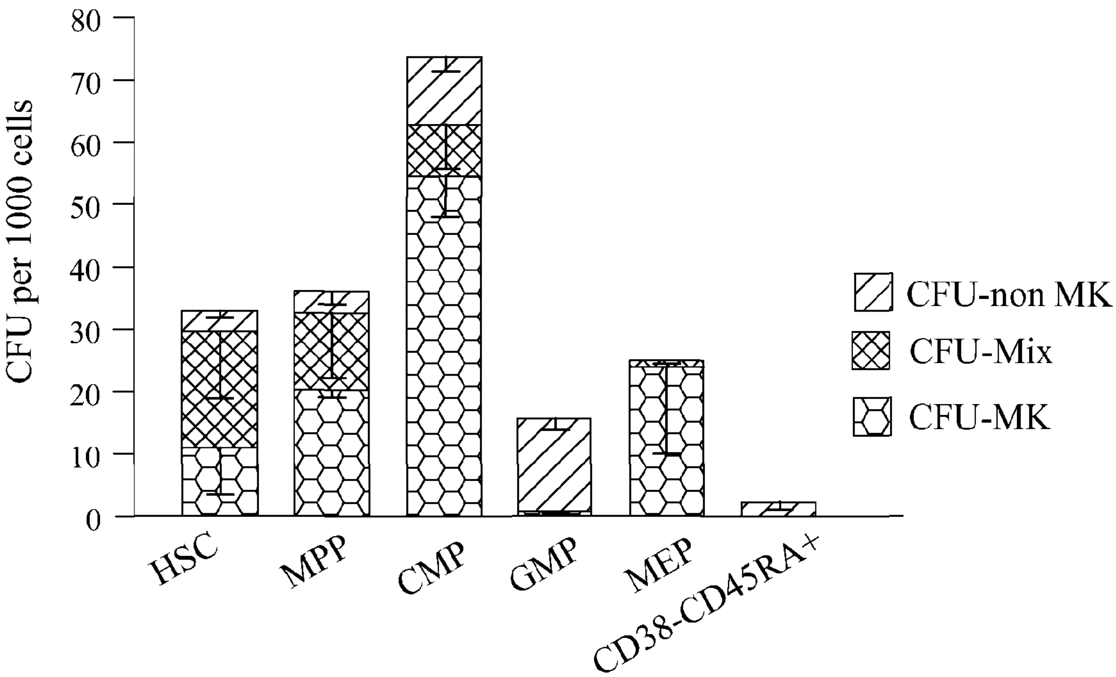

*Fig. 9 (continued)*

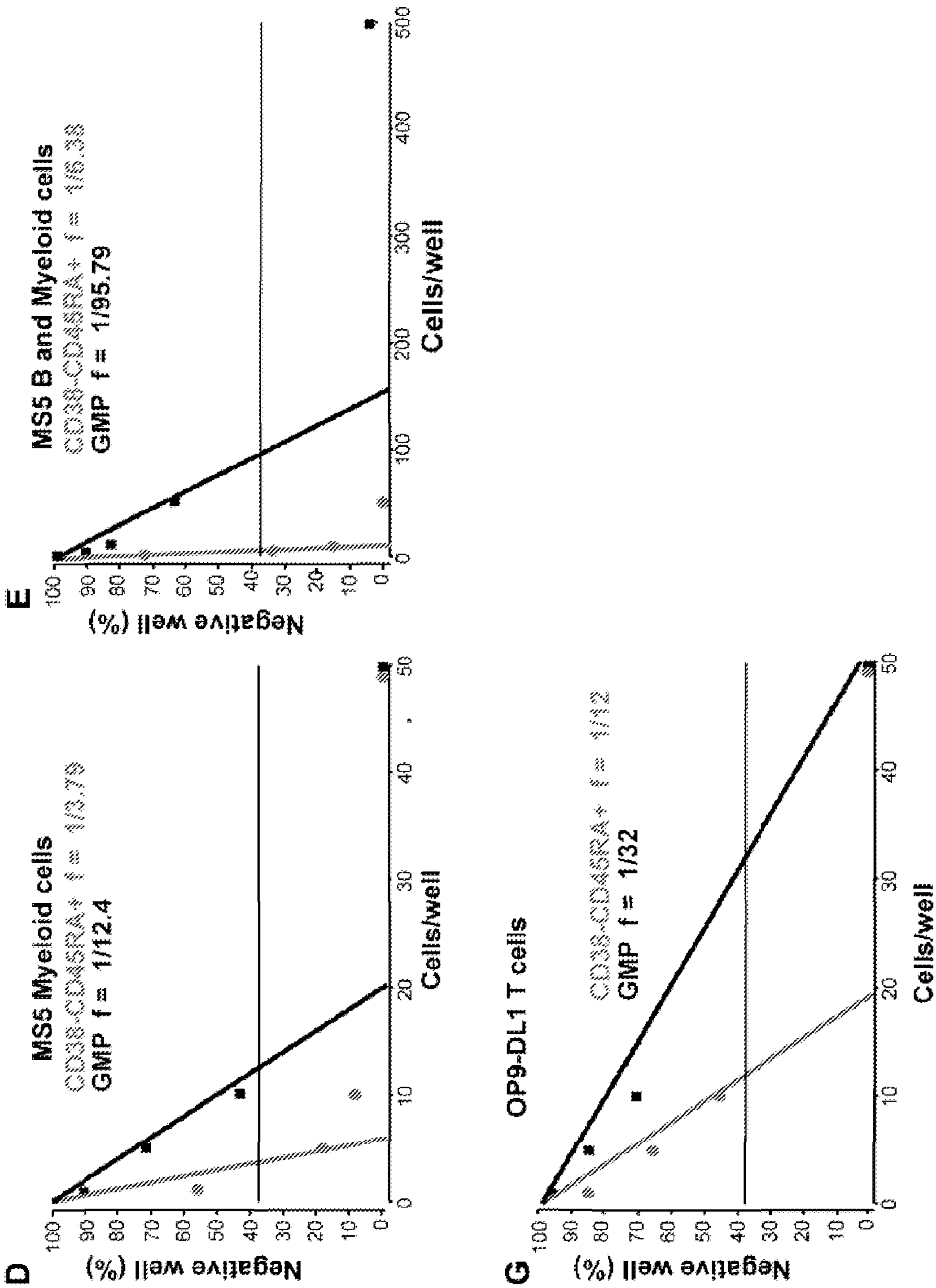

*Fig. 9 (continued)*

F

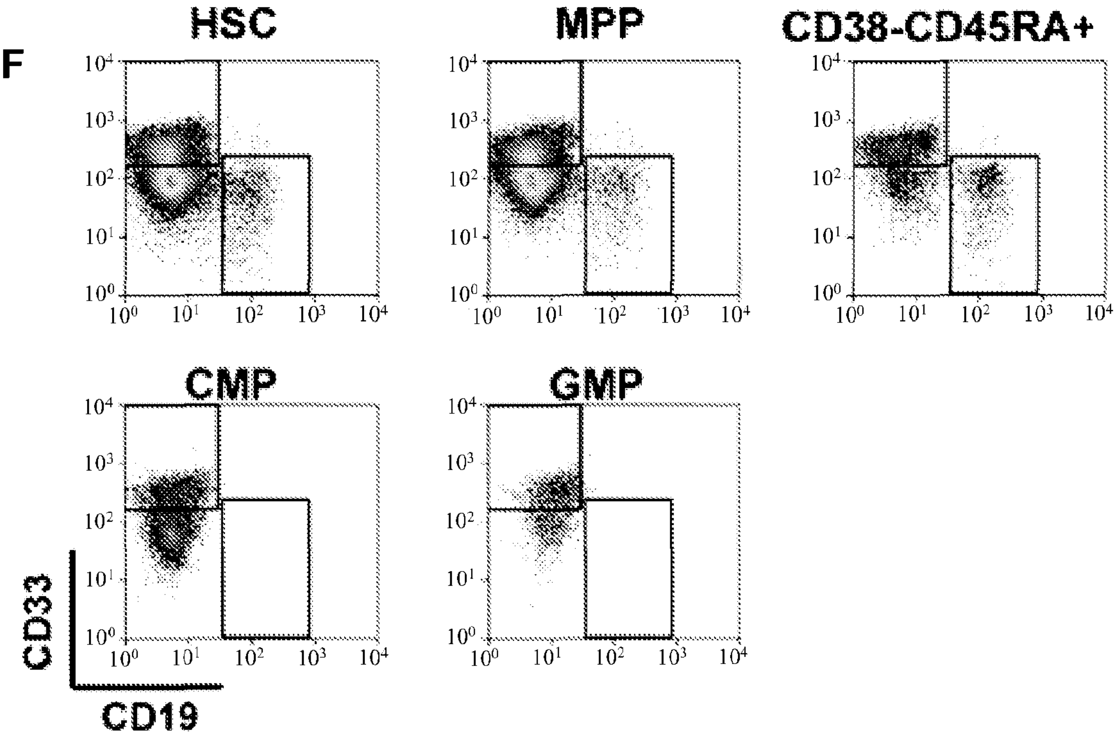


H

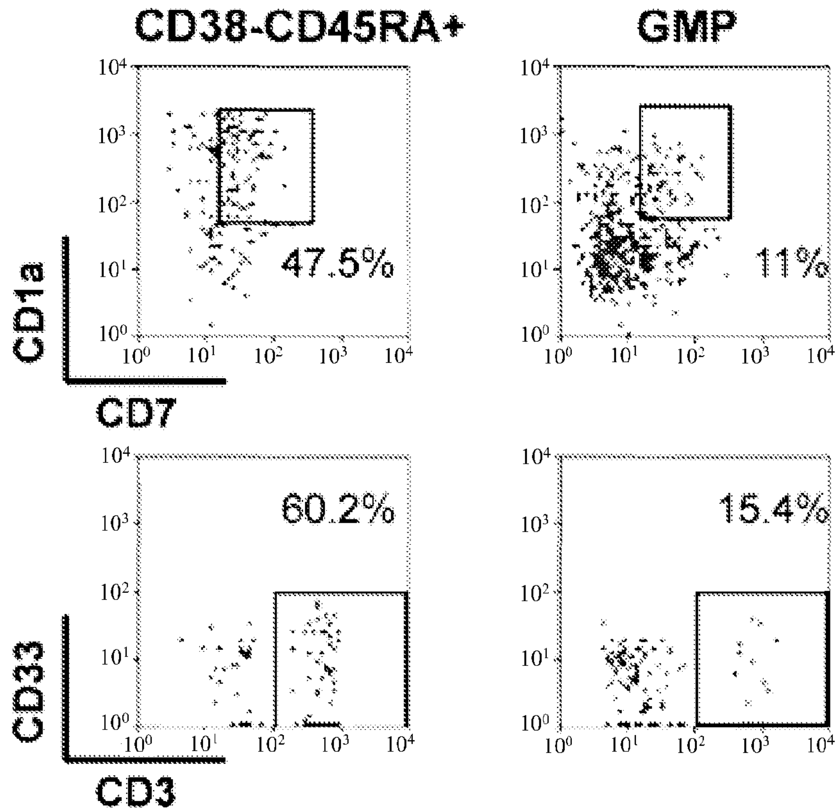

*Fig. 10*

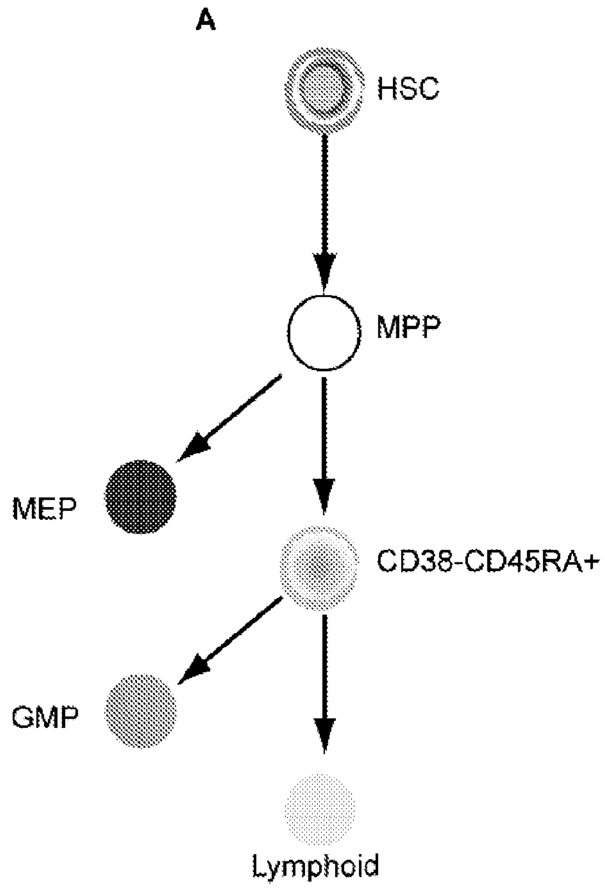

Fig. 10 (continued)

B ii)

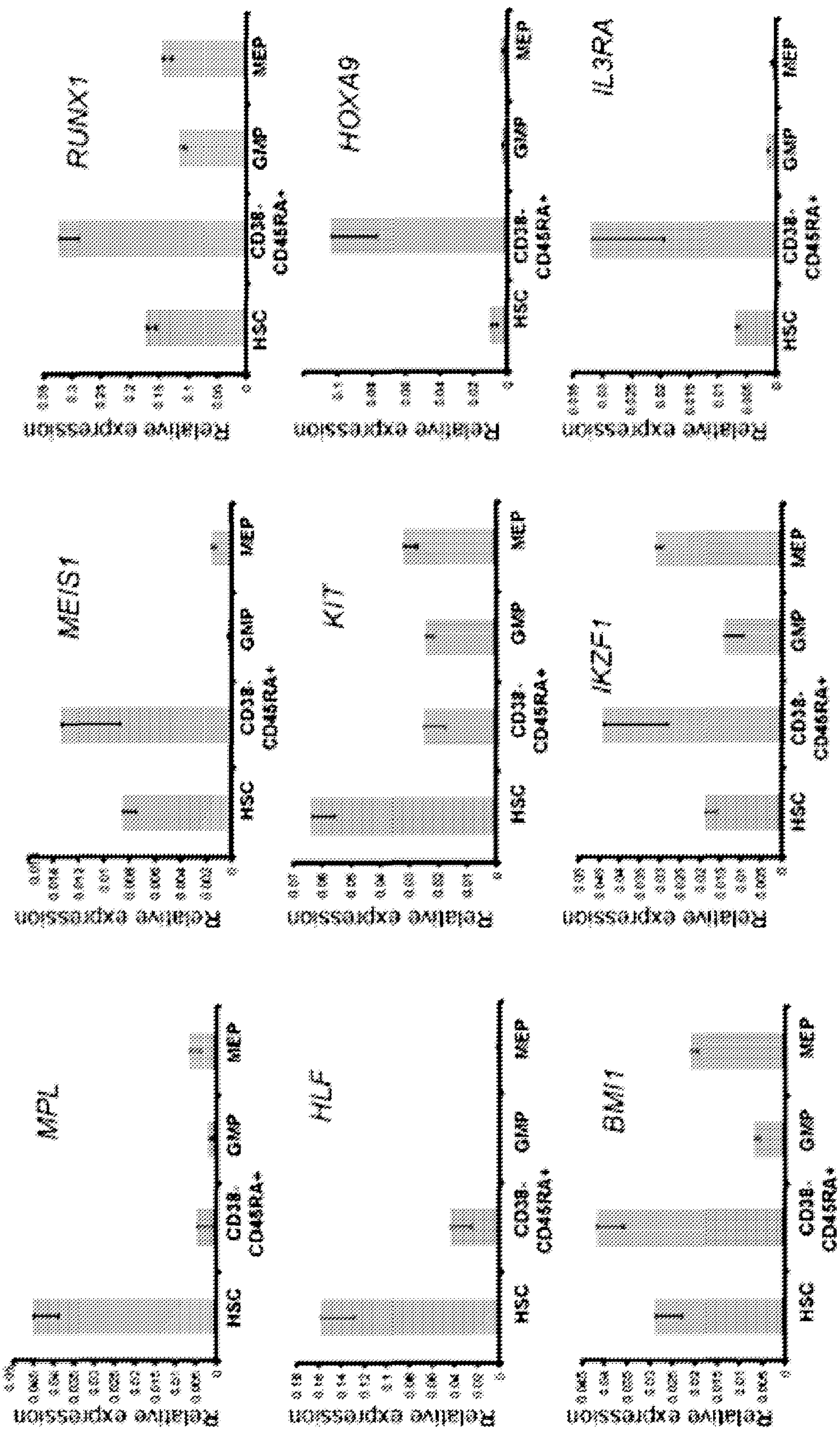

*Fig. 10 (continued)*

**B**

**ii)**

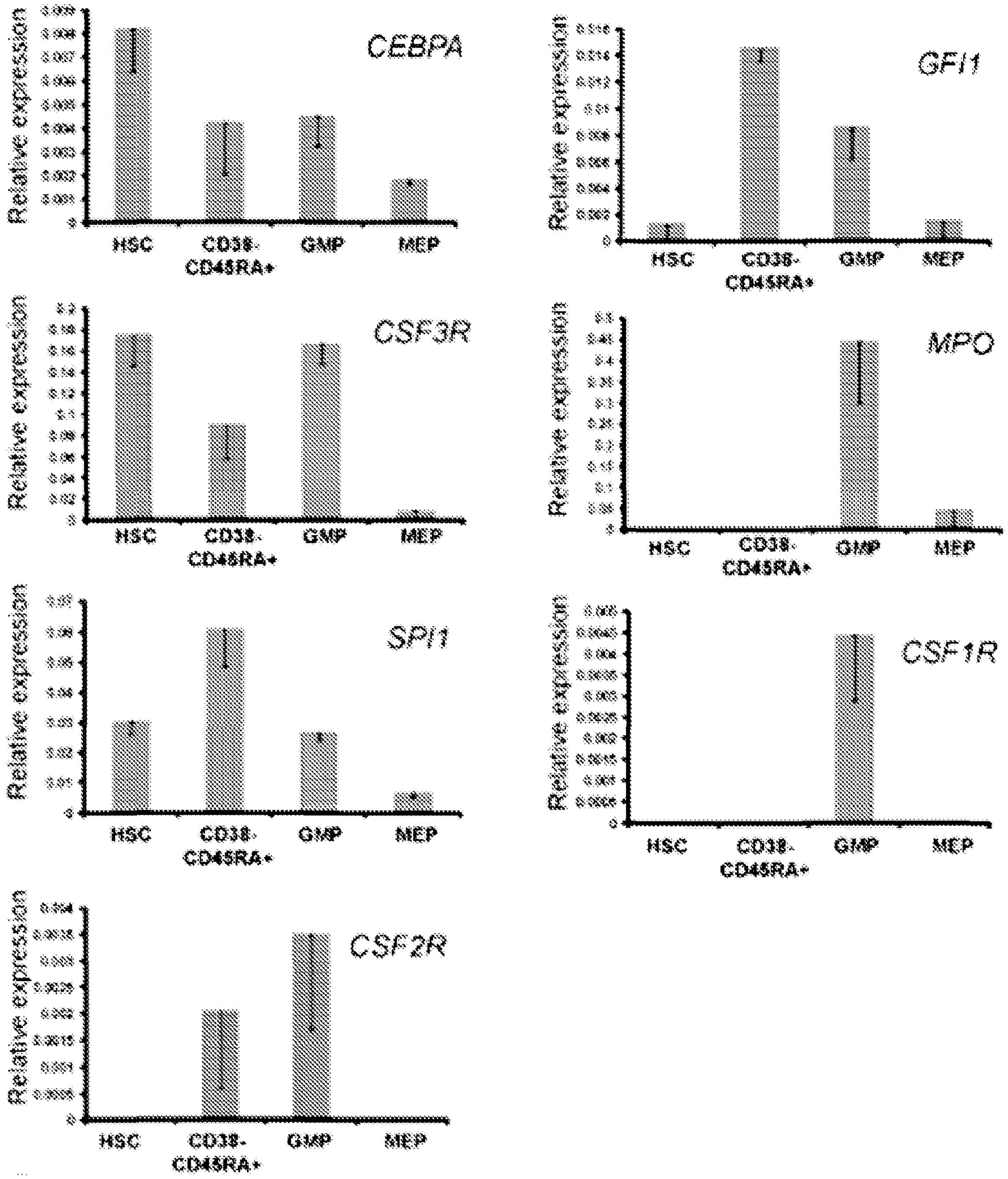

*Fig. 10 (continued)*

**B**

**iii)**

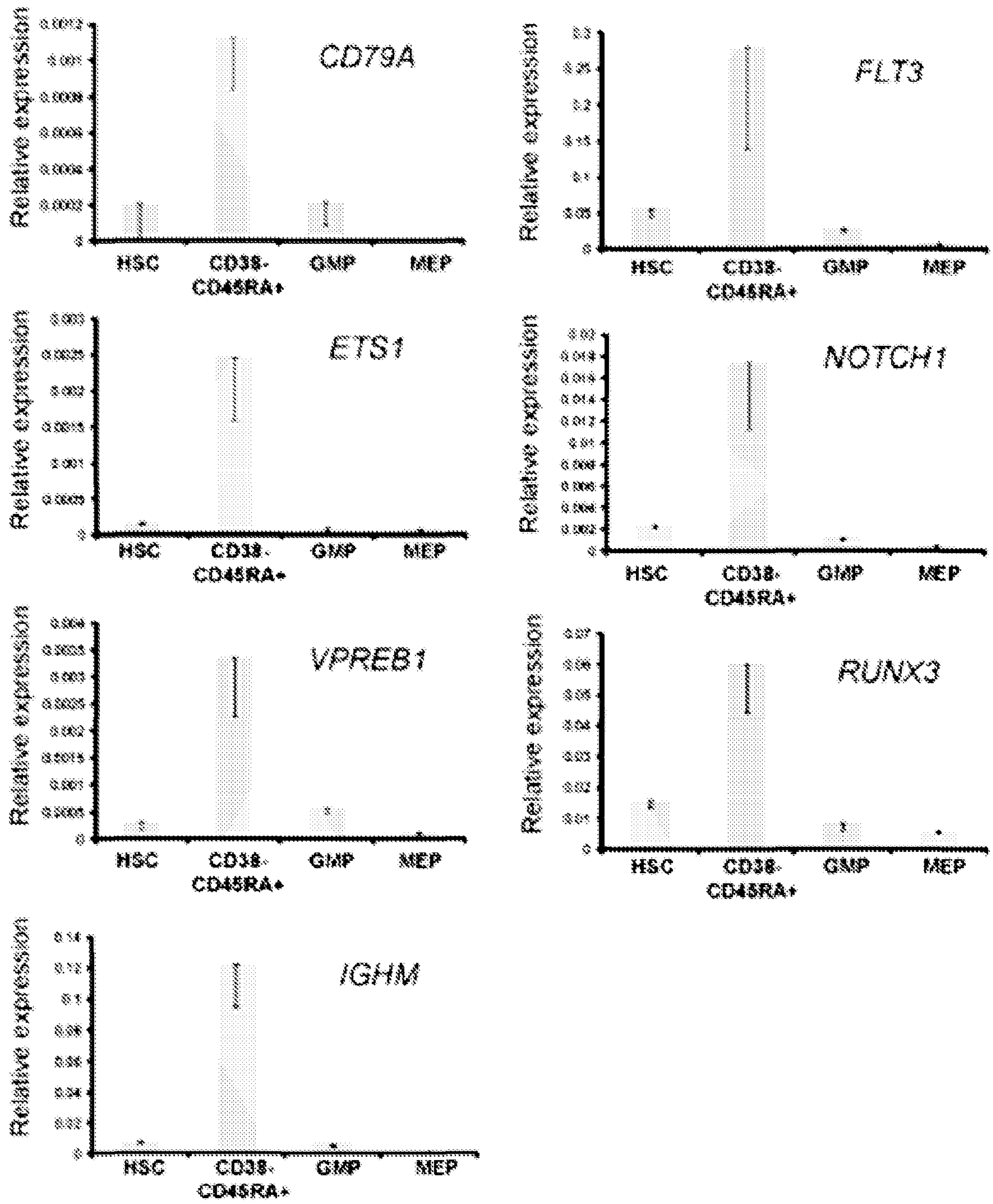

*Fig. 10 (continued)*

**B**

**iv)**

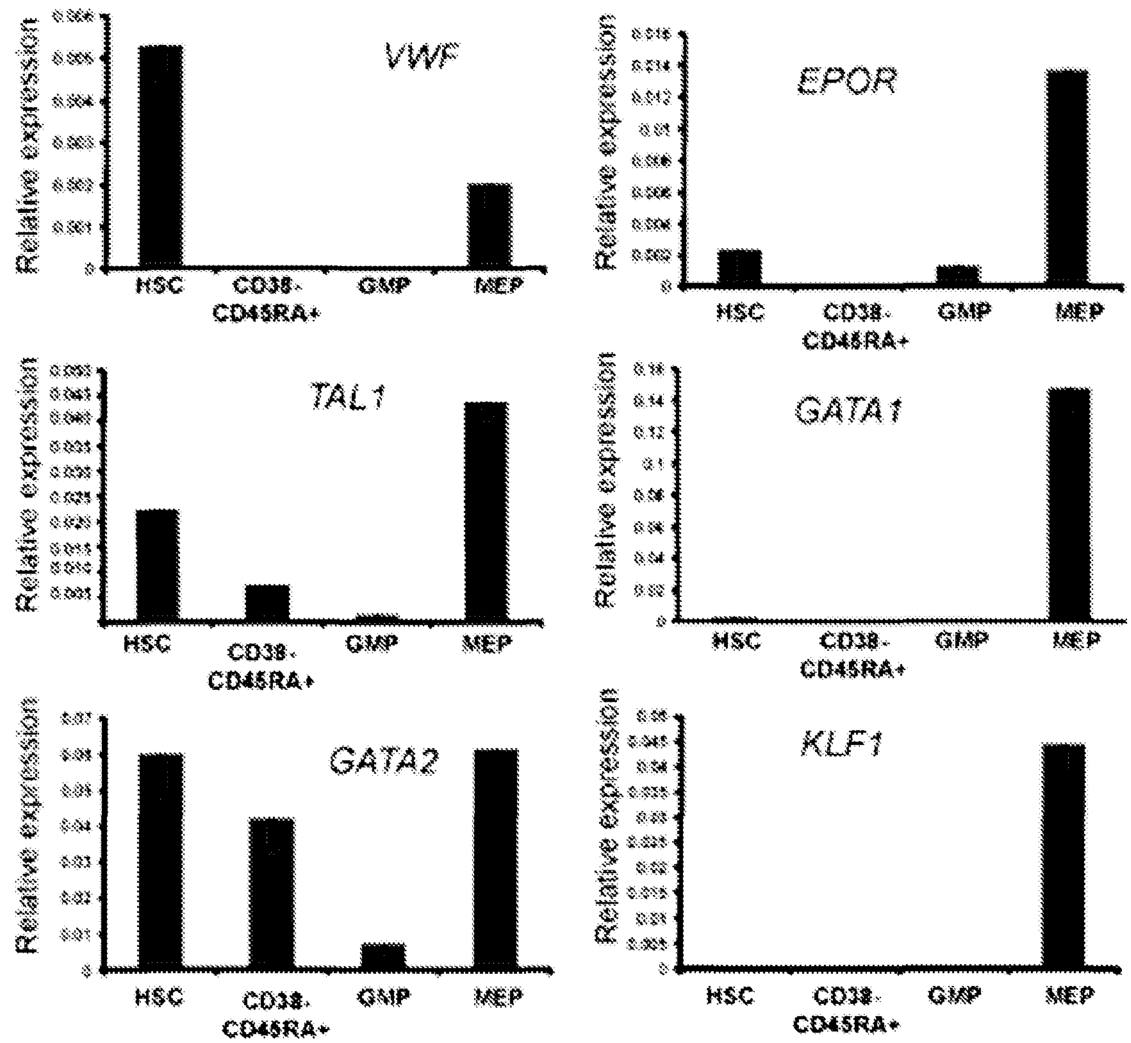

*Fig. 11*

**Figure S5: gene expression in 10 and 100 FACS-sorted normal human stem/progenitor cells, related to Figure 6.**

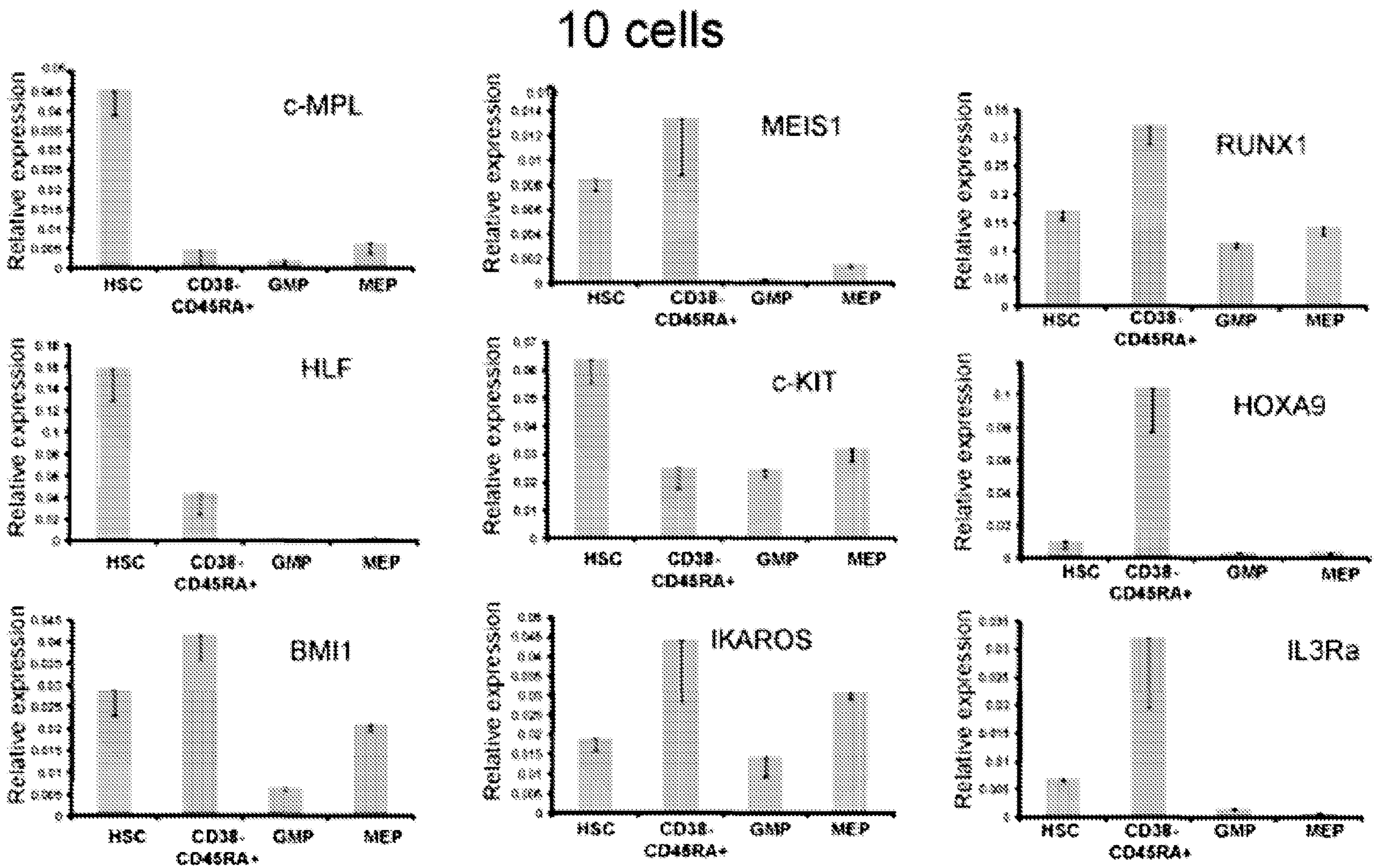

Fig. 11 (continued)

100 cells

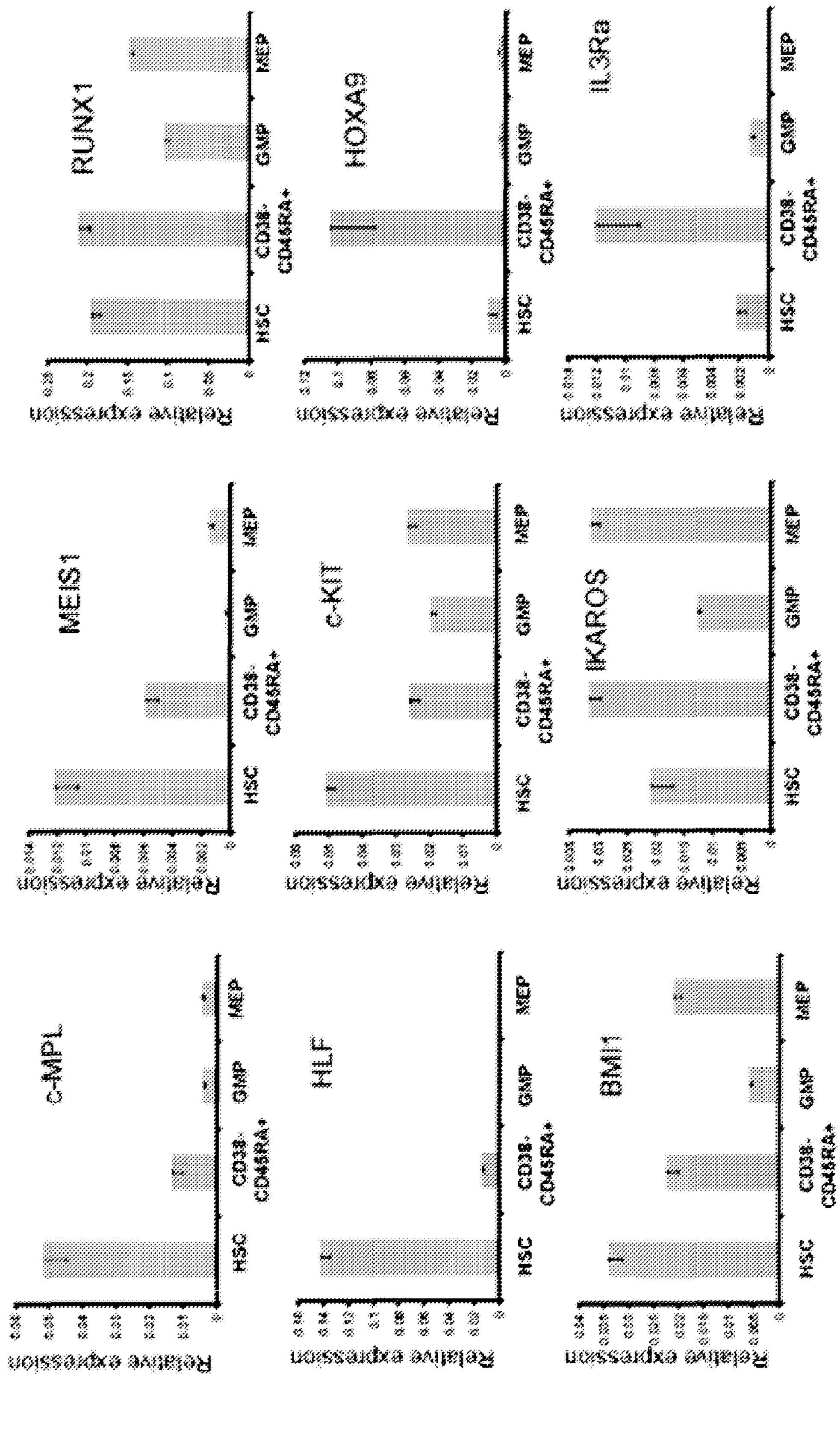

*Fig. 11 (continued)*

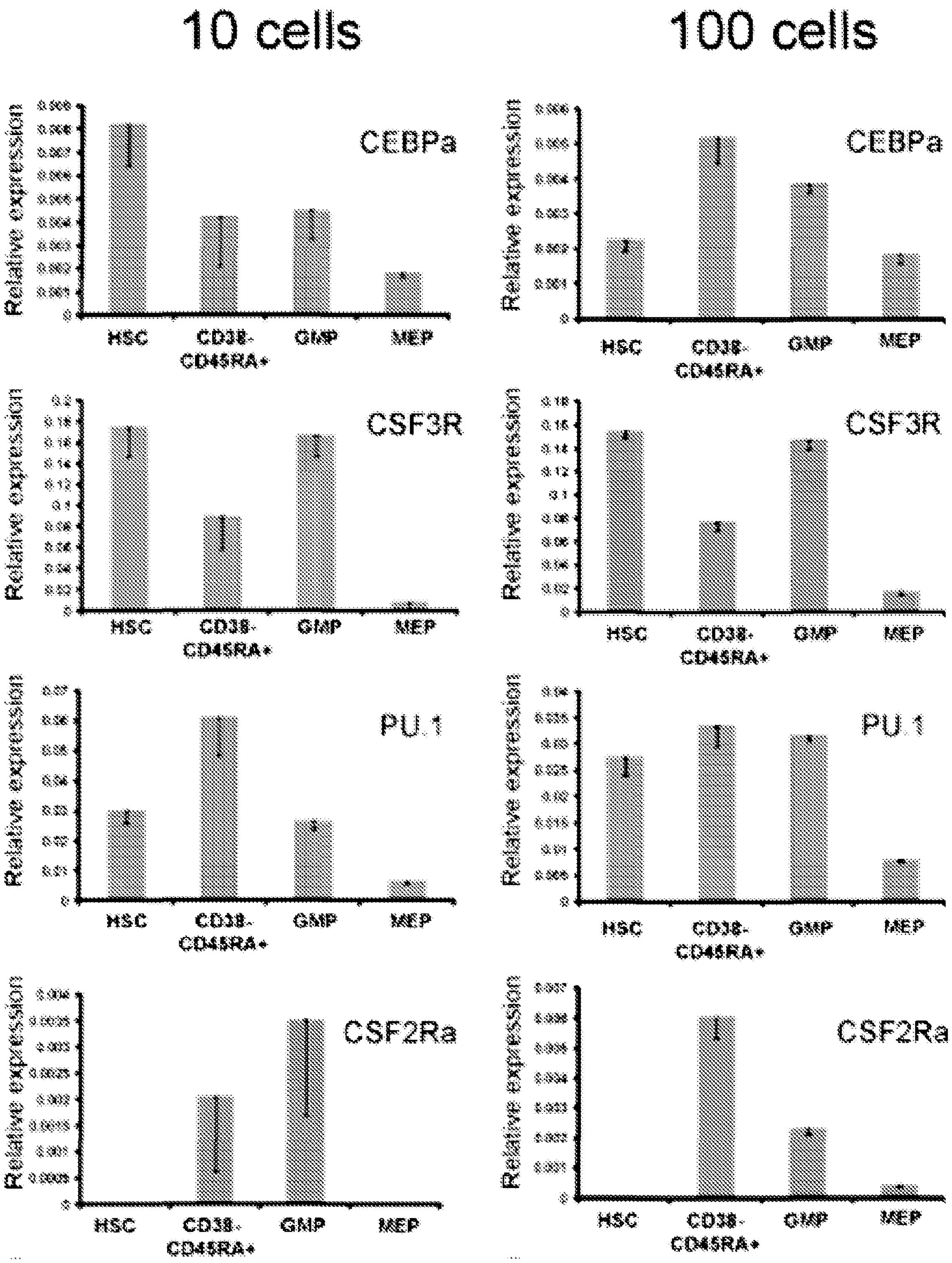

*Fig. 11 (continued)*

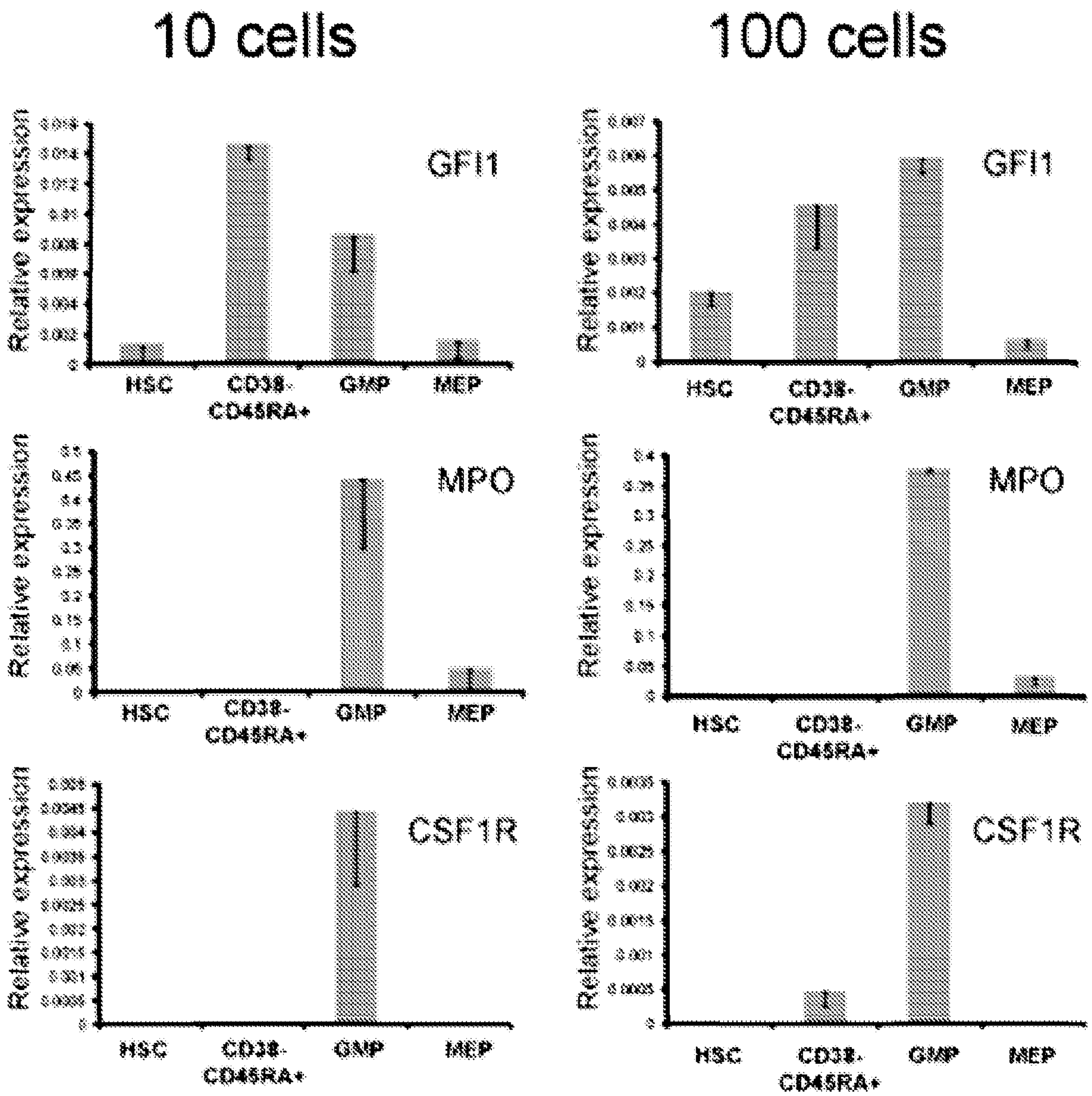

*Fig. 11 (continued)*

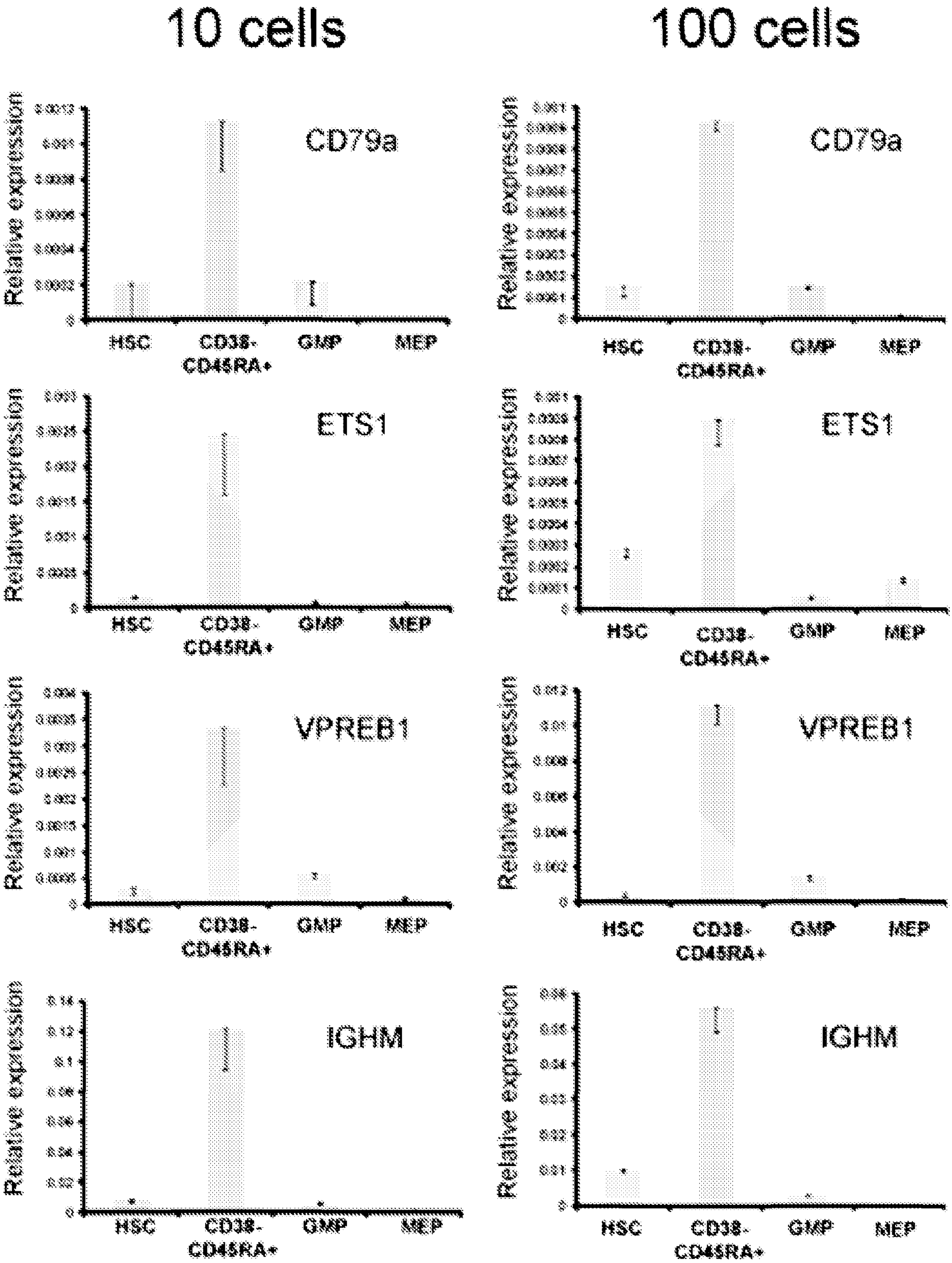

*Fig. 11 (continued)*

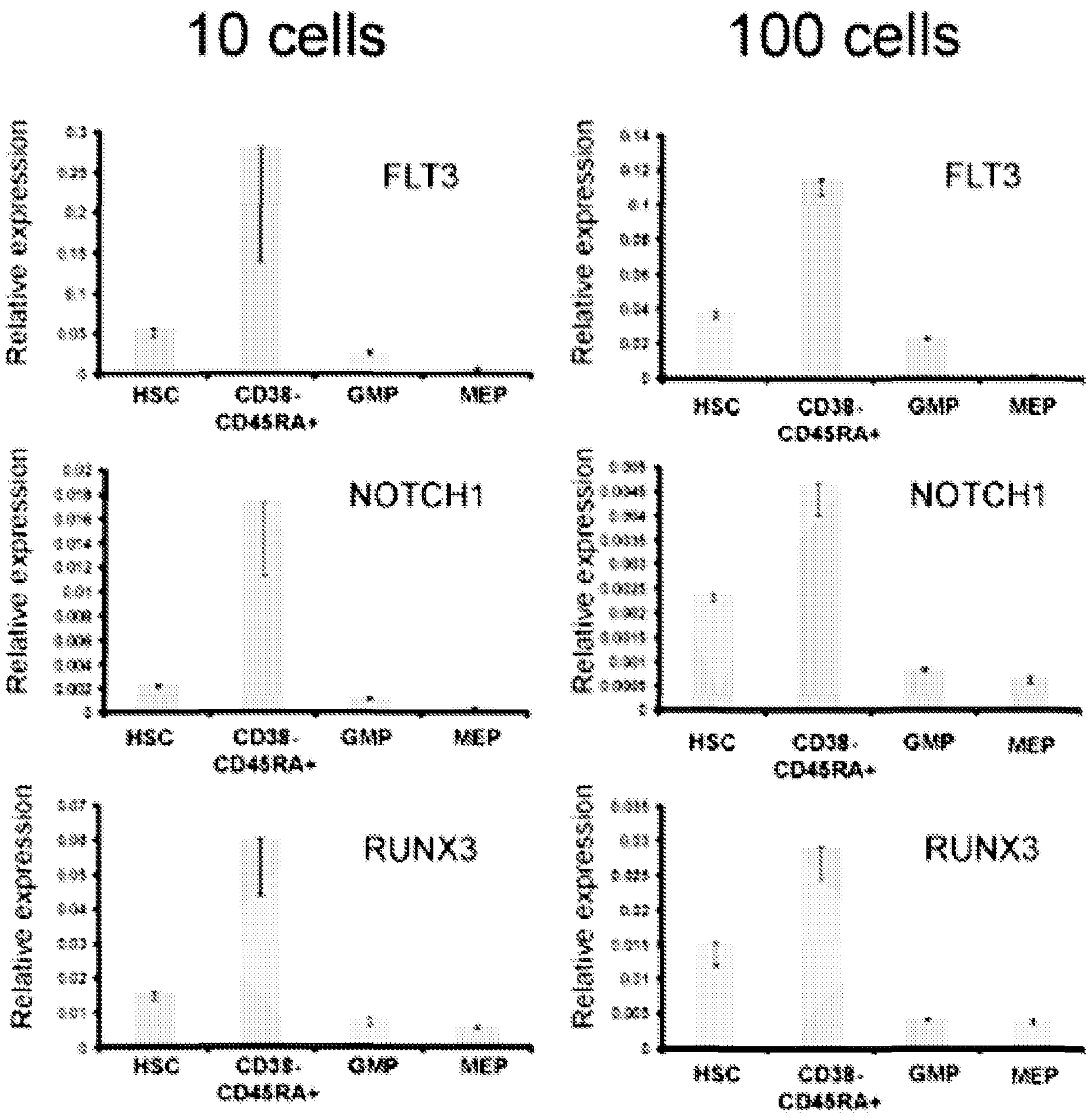

*Fig. 11 (continued)*

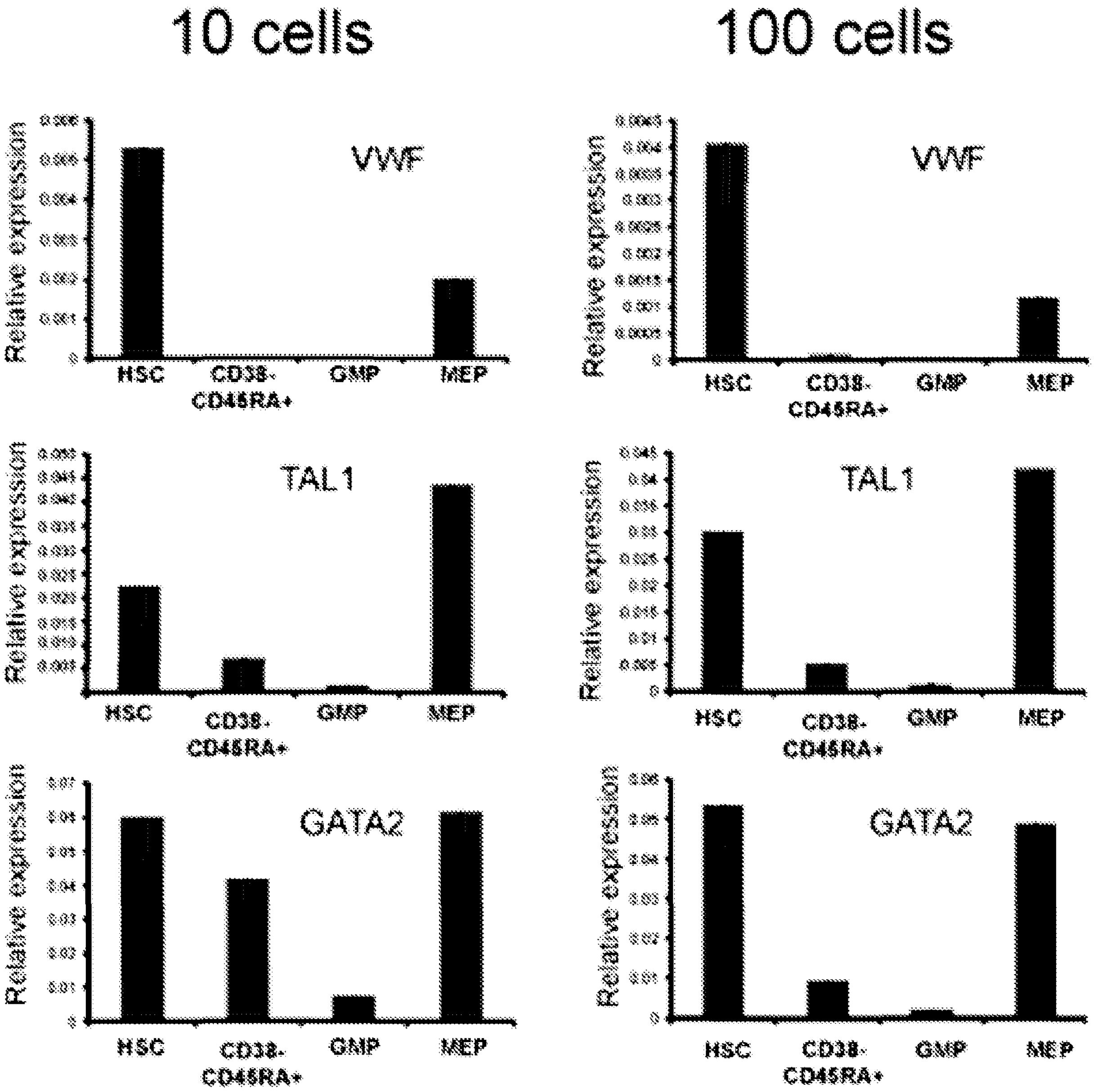

*Fig. 11 (continued)*

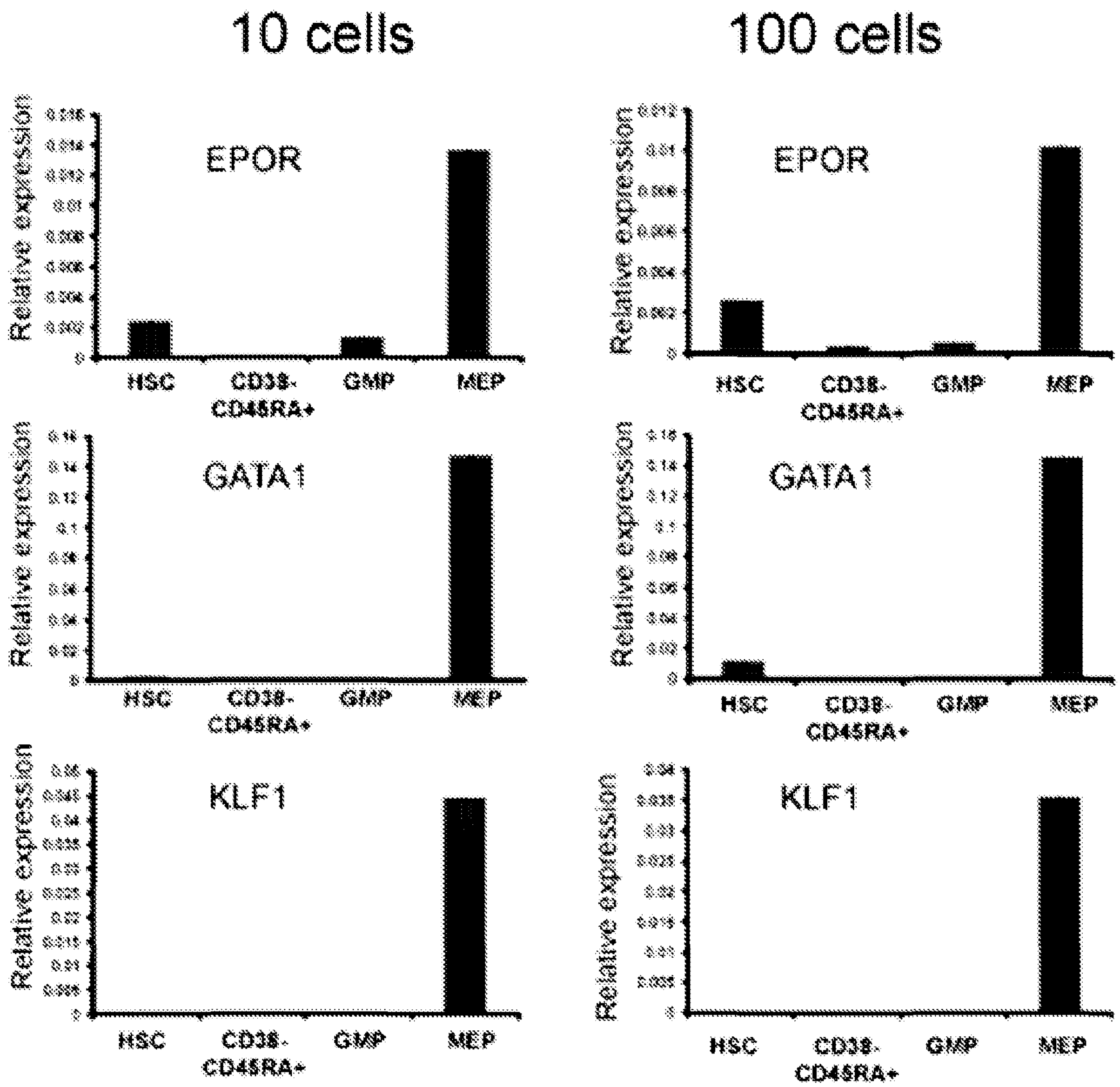

*Fig. 12*

| | Age | Diagnosis | cytogenetics | FAB | Blast (%) | Flt3 | NPM 1 | KIT | % CD38- | % CD38+ | Figure 3B* | % HSC | % MPP | % CD38-CD45RA+ | CD45RA+-like /MPP-like | Figure 3C^ | % CMP | % GMP | % MEP | GMP-like /CMP-like | Figure 3D& |
|---|---|---|---|---|---|---|---|---|---|---|---|---|---|---|---|---|---|---|---|---|---|
| OX208 | 75 | AML | 47,XY,+14[3] /46,XY[7] | M0 | 55% | ND | WT | WT | 51.68 | 38.99 | v | 0.21 | 12.2 | 38.36 | 45RA+ | v | 4.53 | 32.8 | 0.04 | GMP | iv |
| OX268 | 37 | AML | 47, XY+8[20] | M5 | 90% | ND | WT | WT | 48.58 | 44.94 | v | 0 | 0.02 | 48.83 | 45RA+ | vi | 0.01 | 42.6 | 0.01 | GMP | vi |
| SW037 | 68 | Refractory AML | 7,+mar [3] / 47,XY,+8 [17] | M1 | 82% | WT | WT | WT | 49.89 | 42.59 | v | 0.41 | 0.39 | 48.87 | 45RA+ | vi | 0.31 | 37 | 0.05 | GMP | vi |
| BOU014 | 85 | Secondary AML | ND | Secondary AML | 65% | WT | WT | WT | 9.38 | 89.95 | v | 0 | 0.03 | 9.33 | 45RA+ | vi | 2.46 | 82 | 0.02 | GMP | vi |
| LEE015 | 73 | Secondary AML | 46, XY, add (5)(q23), del (7)(q?21)[10]. | Secondary AML | NA | WT | WT | WT | 2.92 | 96.75 | vi | 0 | 0.09 | 2.82 | 45RA+ | v | 0.5 | 79.4 | 0.01 | GMP | vi |
| BIR093 | 20 | AML | 46,XY[20] | M4 | 40% | WT | WT | ND | 3.93 | 92.47 | vi | 0.01 | 0.05 | 3.82 | 45RA+ | v | 2.46 | 89.4 | 0.12 | GMP | v |

Within the CD34+compartment AML samples have variable numbers of cells that express CD38. Samples with an "MPP-like phenotype" fall into two groups: <10% CD38+ cells (Figure 3B ii) and >10%CD38+ cells (Figure 3B iii). Samples with an "45RA+like" expanded phenotype fall into three groups: <10% CD38+ (Figure 3B vi); <90% but >10% CD38+ (Figure 3B v) >90% CD38+ (Figure 3 B vi).

^Within the CD34+CD38- compartment, AML samples have variable numbers of cells that express CD90 and CD45RA. Samples with an "MPP-like phenotype" fall into three groups: >5% CD45RA+ and >2%CD90+ cells (Figure 3C i); <5% CD45RA+ cells and <2% CD90+ (Figure 3C ii); <2% CD45RA+ and <2%CD90+ (Figure 3C iii). Samples with an "CD45RA+-like phenotype" fall into three groups: >5% CD45RA- and >5%CD90+ cells (Figure 3C iv); >5% CD45RA- and <5% CD90+ cells (Figure 3C v); <5% CD45RA+ <5%CD90+ (Figure 3C vi).

*Fig. 13*

| Sample | Karyotype | FISH Probe | Interphase FISH Result | Comments |
|---|---|---|---|---|
| | | | | |
| OX208 CD38-CD45RA+ | 47,XY,+14[3]/46,XY[7] | Vysis LSI IGH dual colour breakapart (14q32.3) | 43/46 interphase cells had three copies of IGH; 3/46 cells had two copies of IGH | Consistent with a population of cells with trisomy 14 |
| OX208 GMP | | | 48/50 interphase cells had three copies of IGH; 2/50 cells had two copies of IGH | Consistent with a population of cells with trisomy 14 |
| | | | | |
| OX268 CD38-CD45RA+ | 47,XY,+8 | Vysis CEP8/CEP12 (8 centromere and 12 centromere) | 35/38 interphase cells had three copies of CEP8 and two copies CEP12; 3/38 cells had two copies of CEP8 and CEP12 | Consistent with a population of cells with trisomy 8 |
| OX268 GMP | | | 45/50 interphase cells had three copies of CEP8 and two copies of CEP12; 5/50 cells had two copies of CEP8 and CEP12 | Consistent with a population of cells with trisomy 8 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20070111238 A1
- WO 2010102244 A1
- GB 1021623 A


### Non-patent literature cited in the description


- **JORDAN et al.** *Leukemia,* 2000, vol. 14 (10), 1777-1784
- **KABAT, E.A. et al.** Sequences of Proteins of Immunological Interest. U.S. Department of Health and Human Services, NIH, 1991
- **CHOTHIA, C. et al.** *J. Mol. Biol.,* 1987, vol. 196, 901-917
- **WARD et al.** *Nature,* 1989, vol. 341, 544-546
- **BIRD et al.** *Science IAI-ATi-AIβ,* 1988
- **HUSTON et al.** *Proc. Natl. Acad. ScL USA,* 1988, vol. 85, 5879-5883
- **GC HOWARD.** Making and Using Antibodies: A Practical Handbook. CRC Books, 2006
- **JULIE D. THOMPSON et al.** CLUSTAL W: Improving the Sensitivity of Progressive Multiple Sequence Alignment Through Sequence Weighting, Position-Specific Gap Penalties and Weight Matrix Choice. *Nucleic Acids Research,* 1994, vol. 22 (22), 4673-4680
- **OSAMU GOTOH.** Significant Improvement in Accuracy of Multiple Protein. Sequence Alignments by Iterative Refinement as Assessed by Reference to Structural Alignments. *J. Mol. Biol.,* 1996, vol. 264 (4), 823-838
- **ERIC DEPIEREUX ; ERNEST FEYTMANS.** Match-Box: A Fundamentally New Algorithm for the Simultaneous Alignment of Several Protein Sequences. *CABIOS,* 1992, vol. 8 (5), 501-509
- **C. E. LAWRENCE et al.** Detecting Subtle Sequence Signals: A Gibbs Sampling Strategy for Multiple Alignment. *Science,* 1993, vol. 262 (5131), 208-214
- **IVO VAN WALLE et al.** Align-M - A New Algorithm for Multiple Alignment of Highly Divergent Sequences. *Bioinformatics,* 2004, vol. 20 (9), 1428-1435
- **ALTSCHUL et al.** *Bull. Math. Bio.,* 1986, vol. 48, 603-16
- **HENIKOFF ; HENIKOFF.** *Proc. Natl. Acad. Sci. USA,* 1992, vol. 89, 10915-19